# EUROPÄISCHE PATENTSCHRIFT

(11) **EP 2 010 514 B1**
(45) Veröffentlichungstag und Bekanntmachung des Hinweises auf die Patenterteilung: **29.12.2010**
(21) Anmeldenummer: 07727983.4
(22) Anmeldetag: 11.04.2007
(51) Int. Cl.: C07D 401/04, C07D 401/14, C07D 405/14, C07D 409/14, A01N 43/707

(54) **3-(PYRIDIN-2-YL)-[1,2,4]-TRIAZINE ALS FUNGIZIDE**
3-(PYRIDIN-2-YL)-[1,2,4]-TRIAZINES FOR USE AS FUNGICIDES
3-(PYRIDINE-2-YL)-[1,2,4]-TRIAZINES UTILISÉES COMME FONGICIDES

(30) Priorität: 12.04.2006 EP 06007743
(43) Veröffentlichungstag der Anmeldung: 07.01.2009
(73) Patentinhaber: BASF SE, 67056 Ludwigshafen (DE)
(72) Erfinder: GRAMMENOS, Wassilios, 67071 Ludwigshafen (DE); GROTE, Thomas, 67157 Wachenheim (DE); DIETZ, Jochen, 68167 Mannheim (DE); LOHMANN, Jan Klaas, 67063 Ludwigshafen (DE); RENNER, Jens, 67098 Bad Dürkheim (DE); MÜLLER, Bernd, 67227 Frankenthal (DE); ULMSCHNEIDER, Sarah, 67098 Bad Dürkheim (DE)
(86) Internationale Anmeldenummer: PCT/EP2007/053515
(87) Internationale Veröffentlichungsnummer: WO 2007/116078

(56) Entgegenhaltungen:
- EP-A1- 0 407 888
- EP-A2- 0 259 139
- US-A- 4 033 752
- SCHILT, ALFRED A. ET AL: "New chromogens of the ferroin type. VII. 3-Substituted-1,2,4- triazines, 3,5-disubstituted-1,2,4-triazolines, and triazoles, and 2,4- and 2,6-bis(triazinyl) and triazolinyl substituted pyridines" TALANTA , 21(8), 831-6 CODEN: TLNTA2; ISSN: 0039-9140, 1974, XP002443684
- SCHILT, ALFRED A. ET AL: "New chromogens of the ferroin type. VIII. Some di- and trisubstituted 1,2,4-triazines, 3-substituted-9H-indeno[1,2-e]-1,2,4- triazin-9-ones, and di- and trisubstituted 1,2,4-triazolines" TALANTA , 24(11), 685-7 CODEN: TLNTA2; ISSN: 0039-9140, 1977, XP002443685
- DREW, M. G. B. ET AL: "Unusual complexes formed by the early lanthanides with 2,6-bis(5,6-dialkyl-1,2,4-triazin-3-yl)-py ridines" INORGANIC CHEMISTRY COMMUNICATIONS , 4(9), 462-466 CODEN: ICCOFP; ISSN: 1387-7003, 2001, XP002443686
- COLETTE, SONIA ET AL: "Use of Electrospray Mass Spectrometry (ESI-MS) for the Study of Europium(III) Complexation with Bis(dialkyltriazinyl)pyridines and Its Implications in the Design of New Extracting Agents" INORGANIC CHEMISTRY , 41(26), 7031-7041 CODEN: INOCAJ; ISSN: 0020-1669, 2002, XP002443687
- DREW M G B ET AL: "QSAR studies of multidentate nitrogen ligands used in lanthanide and actinide extraction processes" JOURNAL OF ALLOYS AND COMPOUNDS, ELSEVIER SEQUOIA, LAUSANNE, CH, Bd. 374, Nr. 1-2, 14. Juli 2004 (2004-07-14), Seiten 408-415, XP004514974 ISSN: 0925-8388
- HUDSON, MICHAEL J. ET AL: "The coordination chemistry of 1,2,4-triazinyl bipyridines with lanthanide(III) elements - implications for the partitioning of americium(III)" DALTON TRANSACTIONS , (9), 1675-1685 CODEN: DTARAF; ISSN: 1477-9226, 2003, XP002443688
- DATABASE CAPLUS [Online] CHEMICAL ABSTRACTS SERVICE, COLUMBUS, OHIO, US; ALPHONSE, F.-A. ET AL: "Polyheterocyclic molecules for selective complexation of metallic cations" XP002443692 gefunden im STN Database accession no. 2006:895172 & CHOCS , 32, 22-29 CODEN: CHCSE5; ISSN: 1157-741X, 2006,
- DATABASE CAPLUS [Online] CHEMICAL ABSTRACTS SERVICE, COLUMBUS, OHIO, US; WEI, YUEZHOU ET AL: "Preparation of novel silica-based R-BTP extraction-resins and their application to trivalent actinides and lanthanides separation" XP002443693 gefunden im STN Database accession no. 2003:109406 & JOURNAL OF NUCLEAR SCIENCE AND TECHNOLOGY , (SUPPL. 3), 761-764 CODEN: JNSTAX; ISSN: 0022-3131, 2002,

## Beschreibung

Die vorliegende Erfindung betrifft 3-(Pyridin-2-yl)-[1,2,4]-triazine und ihre Verwendung zur Bekämpfung von Schadpilzen sowie Pflanzenschutzmittel, die derartige Verbindungen als wirksamen Bestandteil enthalten.

Die EP-A 234 104 beschreibt 2-(Pyridin-2-y)-pyrimidine, die in der 6-Position des Pyridinrestes eine Alkylgruppe aufweisen und die in der 3,4-Position des Pyrimidinrings einen anellierten gesättigten 5- oder 6-Ring aufweisen können. Die Verbindungen sind zur Bekämpfung von pflanzenpathogenen Pilzen (Schadpilzen) geeignet.

Aus der US 4,873,248 sind 2-(Pyridin-2-yl)-pyrimidine mit fungizider Wirkung bekannt, die in der 4-Position des Pyrimidinrings einen gegebenenfalls substituierten Phenylring tragen.

Die EP-A 259 139 beschreibt 2-(Pyridin-2-yl)-pyrimidine, die in der 6-Position des Pyridinrestes eine gegebenenfalls substituierte Phenylgruppe aufweisen und die in der 3,4-Position des Pyrimidinrings einen anellierten gesättigten 5- oder 6-Ring aufweisen können. Die Verbindungen sind ebenfalls zur Bekämpfung von pflanzenpathogenen Pilzen (Schadpifzen) geeignet.

Die WO 2006/010570 beschreibt fungizid wirksame 2-(6-Phenylpyridin-2-yl)-pyrimidinVerbindungen der folgenden Formel B: 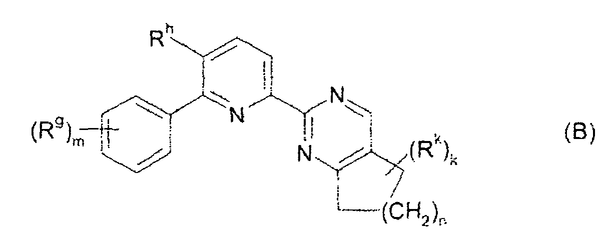 worin: k für 0, 1, 2 oder 3, m für 0, 1, 2, 3, 4 oder 5 und n für 1, 2, 3, 4 oder 5 stehen, die Substituenten R⁹ unter anderem Halogen, OH, CN, NO₂, C₁-C₄-Alkyl, C₁-C₄-Halogenalkyl, C₁-C₄-Alkoxy, C₁-C₄-Halogenalkoxy, C₂-C₄-Alkenyl, C₂-C₄-Alkinyl, C₃-C₈-Cycloalkyl, C₁-C₄-Alkoxy-C₁-C₄-alkyl, Amino, Phenoxy, etc. bedeuten, R^{h}C₁-C₄-Halogenalkyl, C₁-C₄-Alkoxy, C₁-C₄-Halogenalkoxy, Hydroxy, Halogen. CN oder NO₂ bedeutet und R^{k} für C₁-C₄-Alkyl steht.

In Alfred A. Schilt et al., Talanta, 21(8), 1974, 831-836 (D1), werden 39 neue Ferroinverbindungen beschrieben, z.B. 2,6-Bis-(5,6-dimethyl-1,2,4-triazin-3-yl)pyridin. Deren Chelatisierungs- und chromogene Eigenschaften in Reaktionen mit Eisen(II), Kupfer(I) und Cobalt(II) wurden photospektrometrisch untersucht.

In Alfred A. Schilt et al., Talanta, 24(11), 1977, 685-687 (D2), werden 36 neue Ferroinverbindungen beschrieben, welche sich zu Dedektion und zur Spurenanalytik von Eisen(II), Kupfer(I) und Cobalt(II) eignen, z.B. 3-[6-(2,2' -bipyridyl)]-5,6-dimethyl-1,2,4-triazin.

In M. G .B. Drew et al., Inorganic Chemistry Communications 4(9), 2001, 462-466 (D3), werden 2,6-Bis(5,6-dialkyl-1,2,4-triazin-3-yl)-pyridine beschrieben, welches durch Zugabe von Lanthanoid(III)nitrat in dimeren Komplexen kristallisieren.

In Sonia Colette et al., Inorganic Chemistry 41(26), 2002, 7031-7041 (D4), werden 2,6-Bis(5,6-dialkyl-1,2,4-triazin-3-yl)-pyridine beschrieben, welche als dreizähniger Ligand für die Komplexierung von Europium(III) massenspektrometisch untersucht wurden.

In M. G. B. Drew et al., Journal of Alloys and Compounds, 374(1-2), 2004, 408-415 (D5), werden (Pyridyl)-1,3,5-triazine und (Triazin-3-yl)pyridine beschrieben, welche durch QSAR-Studien für ihren Einsatz zur Optimierung der Abtrennung von Aktinoiden aus Europium(III) untersucht wurden.

In Michael J. Hudson et al., Dalton Transaction (9), 2003, 1675-1685 (D6), werden Bis(1,2,4-triazin-3-yl)pyridine zur Abtrennung von Americum(III) von Europium(III) beschrieben.

In F.-A. Alphonse et al., Database CAPLUS [Online] Chemical Abstracts Service, werden unter der Accession-Nr. 2006:895172 (D7) 2,6-Bis-(5,6-dimethoxy-1,2,4-triazin-3-yl)pyridin und 2,6-Bis-(5,6-diethoxy-1,2,4-triazin-3-yl)pyridin zur selektiven Komplexierung von Metallkationen beschrieben.

In Yuezhou Wei et al., Database CAPLUS [Online] Chemical Abstracts Service, werden unter der Accession-Nr. 2003:109406 (D8) 2,6-Bis-(5,6-dialkyl-1,2,4-triazin-3-yl)pyridine zur Darstellung von Extraktionsharzen für trivalente Aktinoide und Lanthanoide beschrieben.

Die EP-A1-0407888 beschreibt Pyrimido[5,4-e]-as-triazin-5,7(6H,8H)-dione, die herbizide Wirkung aufweisen.

Die US-A-4,033,752 beschreibt Pyridyltriazinone, welche am Cα-Atom alkyliert oder alkoxyliert sein können. Diese Verbindungen weisen herbizide und fungizide Eigenschaften auf.

Die aus dem Stand der Technik bekannten 2-(Pyridin-2-yl)-pyrimidine sind hinsichtlich ihrer fungiziden Wirkung teilweise nicht zufriedenstellend oder besitzen unerwünschte Eigenschaften, wie eine geringe Nutzpflanzenverträglichkeit.

Der vorliegenden Erfindung liegt daher die Aufgabe zugrunde, neue Verbindungen mit besserer fungizider Wirksamkeit und/oder einer besseren Nutzpflanzenverträglichkeit bereitzustellen.

Diese Aufgabe wird überraschenderweise gelöst durch 3-(Pyridin-2-yl)-[1,2,4]-triazinVerbindungen der allgemeinen Formel I 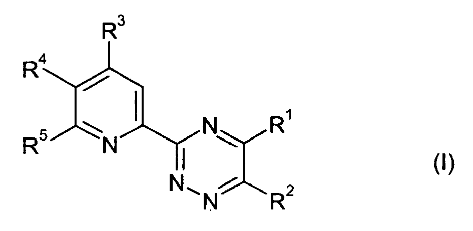 worin:
- R¹, R²: unabhängig voneinander für OH, Halogen, NO₂, NH₂, C₁-C₈-Alkyl, C₁-C₈-Alkoxy, C₁-C₈-Halogenalkyl, C₁-C₈-Halogenalkoxy, C₁-C₈-Alkylamino oder Di(C₁-C₈-alkyl)amino stehen, oder gemeinsam mit den C-Atomen, an die sie gebunden sind, einen gesättigten 5-, 6- oder 7-gliedrigen Carbocyclus oder Heterocyclus bilden können, der neben den Kohlenstoffringgliedern ein oder zwei, unter Sauerstoff und Schwefel ausge- wählte Heteroatome als Ringglieder aufweist, wobei der Carbocyclus und der He- terocyclus unsubstituiert sind oder 1, 2, 3 oder 4 C₁-C₄-Alkylgruppen als Substi- tuenten aufweisen;
- R³: für Wasserstoff, C₁-C₄-Alkyl, C₁-C₄-Alkoxy, C₁-C₄-Halogenalkyl, C₁-C₄-Halogenalkoxy, C₃-C₆-Cycloalkyl, C₃-C₆-Cycloalkylmethyl oder Halogen steht;
- R⁴: für Wasserstoff, C₁-C₄-Alkyl, C₁-C₄-Alkoxy, C₁-C₄-Halogenalkyl, C₁-C₄-Halogenalkoxy oder Halogen steht;
- R⁵: für C₁-C₈-Alkyl, C₁-C₈-Halogenalkyl, C₁-C₈-Alkoxy, C₁-C₈-Halogenalkoxy, C₃-C₈-Cycloalkyl, C₃-C₈-Cycloalkyloxy, 5- oder 6-gliedriges Heteroaryl, Phenyl, Phenoxy, Benzyl, Benzyloxy, 5- oder 6-gliedriges Heteroarylmethyl oder 5- oder 6-gliedriges Heteroaryloxy, wobei die zuvor genannten cyclischen Reste unsub- stituiert sind oder 1, 2, 3, 4 oder 5 Reste R^{a} aufweisen können, wobei
- R^{a}: ausgewählt ist unter OH, SH, Halogen, NO₂, NH₂, CN, COOH, C₁-C₈-Alkyl. C₁-C₈-Alkoxy, C₁-C₈-Halogenalkyl, C₁-C₈-Halogenalkoxy, C₁-C₈-Alkylamino, Di(C₁-C₈-alkyl)amino, C₁-C₈-Alkylthio, C₁-C₈-Halogenalkylthio, C₁-C₈-Alkylsulfinyl, C₁-C₈-Halogenalkylsulfinyl, C₁-C₈-Alkylsulfonyl, C₁-C₈-Halogenalkylsulfonyl, C₃-C₈-Cycloalkyl, Phenyl, Phenoxy und Resten der Formel C(=Z)R^{aa}, worin Z für O, S, N(C₁-C₈-Alkyl), N(C₁-C₈-Alkoxy), N(C₃-C₈-Alkenyloxy) oder N(C₃-C₈-Alkinyloxy) steht und R^{aa} Wasserstoff, C₁-C₈-Alkyl, C₁-C₆-Alkoxy, NH₂, C₁-C₈-Alkylamino oder Di(C₁-C₈-alkyl)amino bedeutet, oder zwei an benachbarte C-Atome gebundene Reste R^{a} gemeinsam mit den C-Atomen, an die sie gebun- den sind, auch einen gesättigten 5-, 6- oder 7-gliedrigen Carbocyclus, einen Benzolring oder einen 5-, 6- oder 7-gliedrigen Heterocyclus bilden können, der neben den Kohlenstoffringgliedern ein oder zwei, unter Sauerstoff und Schwefel ausgewählte Heteroatome als Ringglieder aufweist, wobei der Carbocyclus und der Heterocyclus unsubstituiert sind oder 1, 2, 3 oder 4 C₁-C₄-Alkylgruppen als Substituenten aufweisen;
und die landwirtschaftlich brauchbaren Salze von Verbindungen der Formel I.

Gegenstand der vorliegenden Erfindung sind somit die 3-(Pyridin-2-yl)-triazine der allgemeinen Formel I und deren landwirtschaftlich verträglichen Salzen, ausgenommen die folgenden, in den einleitend zitierten Schriften D1-D8 beschriebenen Verbindungen:
2,6-Bis-(5,6-dimethyl-1,2,4-triazin-3-yl)pyridin;
2,6-Bis-(5,6-diethyl-1,2,4-triazin-3-yl)pyridin;
2,6-Bis-(5,6-dipropyl-1,2,4-triazin-3-yl)pyridin;
2,6-Bis-(5,6-diisopropyl-1,2,4-triazin-3-yl)pyridin;
2,6-Bis-(5,6-dibutyl-1,2,4-triazin-3-yl)pyridin;
2,6-Bis-(5,6-diisobutyl-1,2,4-triazin-3-yl)pyridin;
2,6-Bis-(5,6-dipentyl-1,2,4-triazin-3-yl)pyridin;
2,6-Bis-(5,6-dihexyl-1,2,4-triazin-3-yl)pyridin;
2,6-Bis-(5,6-diheptyl-1,2,4-triazin-3-yl)pyridin;
3-[6-(2,2'-Bipyridyl)]-5,6-dimethyl-1,2,4-triazin;
3-[6-(2,2'-Bipyridyl)]-5,6-diethyl-1,2,4-triazin;
3-[6-(2,2'-Bipyridyl)]-5,6-dipropyl-1,2,4-triazin;
3-[6-(2,2'-Bipyridyl)]-5,6-dibutyl-1,2,4-triazin;
5,6-Diethyl-3-[6-(2-pyridyl)-4-methoxypyridin-2-yl]-1,2,4-triazin;
3-(6-Methylpyridin-2-yl)-5,6-dimethyl-1,2,4-triazin;
3-(6-Methylpyridin-2-yl)-5,6-diethyl-1,2,4-triazin;
2,6-Bis-(5,6-dimethoxy-1,2,4-triazin-3-yl)pyridin; und
2,6-Bis-(5,6-diethoxy-1,2,4-triazin-3-yl)pyridin.

Gegenstand der vorliegenden Erfindung ist weiterhin die Verwendung der 3-(Pyridin-2-yl)-triazine der allgemeinen Formel I und ihrer landwirtschaftlich verträglichen Salze zur Bekämpfung von pflanzenpathogenen Pilzen (= Schadpilzen) sowie ein Verfahren zur Bekämpfung von pflanzenpathogenen Pilzen, das dadurch gekennzeichnet ist, dass man die Pilze, oder die vor Pilzbefall zu schützenden Materialien, Pflanzen, den Boden oder Saatgüter mit einer wirksamen Menge einer Verbindung der allgemeinen Formel I und/oder mit einem landwirtschaftlich verträglichen Salz von I behandelt.

Gegenstand der vorliegenden Erfindung ist weiterhin ein Mittel zur Bekämpfung von Schadpilzen, enthaltend wenigstens eine 3-(Pyridin-2-yl)-triazin-Verbindung der allgemeinen Formel I und/oder ein landwirtschaftlich verträgliches Salz davon und wenigstens einen flüssigen oder festen Trägerstoff.

Die Verbindungen der Formel I und deren Tautomere können je nach Substitutionsmuster ein oder mehrere Chiralitätszentren aufweisen und liegen dann als reine Enantiomere oder reine Diastereomere oder als Enantiomeren- oder Diastereomerengemische vor. Gegenstand der Erfindung sind sowohl die reinen Enantiomere oder Diastereomere als auch deren Gemische.

Unter landwirtschaftlich brauchbaren Salzen kommen vor allem die Salze derjenigen Kationen oder die Säureadditionssalze derjenigen Säuren in Betracht, deren Kationen beziehungsweise Anionen die fungizide Wirkung der Verbindungen I nicht negativ beeinträchtigen. So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat. Sie können durch Reaktion von I mit einer Säure des entsprechenden Anions, vorzugsweise der Chlorwasserstoffsäure, Bromwasserstoffsäure, Schwefelsäure, Phosphorsäure oder Salpetersäure, gebildet werden.

Bei den in den vorstehenden Formeln angegebenen Definitionen der Variablen werden Sammelbegriffe verwendet, die allgemein repräsentativ für die jeweiligen Substituenten stehen. Die Bedeutung Cₙ-Cₘ gibt die jeweils mögliche Anzahl von Kohlenstoffatomen in dem jeweiligen Substituenten oder Substituententeil an:

Halogen: Fluor, Chlor, Brom und Iod;

Alkyl sowie alle Alkylteile in Alkoxy, Alkoxyalkyl, Alkylcarbonyl, Alkoxycarbonyl, Alkylthio, Alkylsulfonyl, Alkylsulfinyl, Alkylamino, Dialkylamino, Alkylaminocarbonyl, Dialkylaminocarbonyl: gesättigte, geradkettige oder verzweigte Kohlenwasserstoffreste mit 1 bis 8 (C₁-C₈-Alkyl), häufig 1 bis 6 (C₁-C₆-Alkyl) und insbesondere 1 bis 4 Kohlenstoffatomen (C₁-C₄-Alkyl) wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, Heptyl, 1-Methylhexyl, Octyl, 1-Methylheptyl und 2-Ethylhexyl;

Halo(gen)alkyl sowie alle Halogenalkylteile in Halogenalkoxy und Halogenalkylthio: geradkettige oder verzweigte Alkylgruppen mit 1 bis 8 und insbesondere 1 bis 4 Kohlenstoffatomen (wie vorstehend genannt), wobei in diesen Gruppen teilweise oder vollständig die Wasserstoffatome durch Halogenatome wie vorstehend genannt und insbesondere durch Fluor oder Chlor ersetzt sein können, insbesondere C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 1,1,1-Trifluorprop-2-yl;

Alkenyl: einfach ungesättigte, geradkettige oder verzweigte Kohlenwasserstoffreste mit 2 bis 8 oder 3 bis 8 Kohlenstoffatomen und einer Doppelbindung in einer beliebigen Position, z. B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl;

Alkinyl: geradkettige oder verzweigte Kohlenwasserstoffgruppen mit 2 bis 8 oder 3 bis 8 Kohlenstoffatomen und einer Dreifachbindung in einer beliebigen Position, z. B. Ethinyl, 1-Propinyl, 2-Propinyl, 1-Butinyl, 2-Butinyl, 3-Butinyl, 1-Methyl-2-propinyl;

Cycloalkyl: monocyclische, gesättigte Kohlenwasserstoffgruppen mit 3 bis 8, vorzugsweise bis 6 Kohlenstoffringgliedern, wie Cyclopropyl, Cyclobutyl, Cyclopentyl und Cyclohexyl;

Cycloalkylmethyl: für einen Cycloalkylrest, wie vorstehend genannt, der über eine Methylengruppe (CH₂) gebunden ist.

Alkylamino sowie die Alkylaminoteile in Alkylaminocarbonyl: für einen über eine NH-Gruppe gebundene Alkylgruppe, worin Alkyl für einen der zuvor genannten Alkylreste mit 1 bis 8 C-Atomen steht, wie Methylamino, Ethylamino, n-Propylamino, Isopropylamino, n-Butylamino und dergleichen;

Dialkylamino sowie die Dialkylaminoteile in Dialkylaminocarbonyl: für einen Rest der Formel N(Alkyl)₂, worin Alkyl für einen der zuvor genannten Alkylreste mit 1 bis 8 C-Atomen steht, z. B. für Dimethylamino, Diethylamino, Methylethylamino, N-Methyl-N-propylamino und dergleichen;

Alkoxy sowie die Alkoxyteile in Alkoxycarbonyl: für eine über ein Sauerstoff gebundene Alkylgruppe mit 1 bis 8, insbesondere 1 bis 6 und speziell 1 bis 4 C-Atomen, z. B. Methoxy, Ethoxy, n-Propoxy, 1-Methylethoxy, Butoxy, 1-Methylpropoxy, 2-Methylpropoxy oder 1,1-Dimethylethoxy;

Alkxoxycarbonyl: für einen über eine Carbonylgruppe gebundenen Alkoxyrest, wie vorstehend genannt;

Alkylthio: für einen über eine Schwefelatom gebundenen Alkylgruppe wie vorstehend genannt;

Alkylsulfinyl: für einen über eine S(=O)-Gruppe gebundenen Alkylgruppe wie vorstehend genannt;

Alkylsulfonyl: für einen über eine S(=O)₂-Gruppe gebundenen Alkylgruppe wie vorstehend genannt;

Halogenalkoxy: für einen Alkoxyrest mit 1 bis 8, insbesondere 1 bis 6 und speziell 1 bis 4 C-Atomen wie vorstehend genannt, der partiell oder vollständig durch Fluor, Chlor, Brom und/oder Iod, vorzugsweise durch Fluor substituiert ist, also z. B. OCH₂F, OCHF₂, OCF₃, OCH₂CI, OCHCI₂, OCCI₃, Chlorfluormethoxy, Dichlorfluormethoxy, Chlordifluormethoxy, 2-Fluorethoxy, 2-Chlorethoxy, 2-Bromethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy;

Alkylen: für eine lineare gesättigte Kohlenwasserstoffkette mit 2 bis 6 und insbesondere 2 bis 4 C-Atomen wie Ethan-1,2-diyl, Propan-1,3-diyl, Butan-1,4-diyl, Pentan-1,5-diyl oder Hexan-1,6-diyl.

Gesättigter 5-, 6- oder 7-gliedrieger Heterocyclus, der ein oder zwei unter Sauerstoff und Schwefel ausgewählte Heteroatome als Ringglieder aufweist: ein Ring, der aus Kohlenstoffatomen und 1 oder 2 unter Schwefel und Sauerstoff ausgewählten Heteroatomen aufgebaut ist, wobei die Gesamtzahl der Ringatome (Ringglieder) 5, 6 oder 7 beträgt, beispielsweise: Oxolan, Oxepan, Oxan (Tetrahydorpyran), 1,3-Dioxolan, 1,3-Dioxan, 1,4-Dioxan, Thiolan, Thian, Thiepan, 1,3-Dithiolan, 1,3-Dithian und 1,4-Dithian;

5- oder 6-gliedriges Heteroaryl: ein 5- oder 6-gliedriger aromatischer Ring, der neben Kohlenstoff 1, 2, 3 oder 4 Heteroatome als Ringglieder aufweist, wobei die Heteroatome typischerweise unter Sauerstoff, Stickstoff und Schwefel ausgewählt sind, insbesondere:
- 5-gliedriges Heteroaryl, das 1, 2, 3 oder 4 Stickstoffatome als Ringglieder aufweist, wie 1-, 2- oder 3-Pyrrolyl, 1-, 3- oder 4-Pyrazolyl, 1-, 2- oder 4-Imidazolyl,
   1,2,3-[1H]-Triazol-1-yl, 1,2,3-[2H]-Triazol-2-yl, 1,2,3-[1H]-Triazol-4-yl,
   1,2,3-[1H]-Triazol-5-yl, 1,2,3-[2H]-Triazol-4-yl, 1,2,4-[1H]-Triazol-1-yl,
   1,2,4-[1H]-Triazol-3-yl, 1,2,4-[1H]-Triazol-5-yl, 1,2,4-[4H]-Triazol-4-yl,
   1,2,4-[4H]-Triazol-3-yl, [1H]-Tetrazol-1-yl, [1H]-Tetrazol-5-yl, [2H]-Tetrazol-2-yl und [2H]-Tetrazol-5-yl;
- 5-gliedriges Heteroaryl, das 1 unter Sauerstoff und Schwefel ausgewähltes Heteroatome und gegebenenfalls 1, 2 oder 3 Stickstoffatome als Ringglieder aufweist, beispielsweise 2-Furyl, 3-Furyl, 2-Thienyl, 3-Thienyl, 3- oder 4-Isoxazolyl, 3- oder 4- Isothiazolyl, 2-, 4- oder 5-Oxazolyl, 2-, 4 oder 5-Thiazolyl, 1,2,4-Thiadiazol-3-yl, 1,2,4-Thiadiazol-5-yl, 1,3,4-Thiadiazol-2-yl, 1,2,4-Oxadiazol-3-yl, 1,2,4-Oxadiazol-5-yl und 1,3,4-Oxadiazol-2-yl;
- 6-gliedriges Heteroaryl, das 1, 2, 3 oder 4 Stickstoffatome als Ringglieder aufweist, wie 2-Pyridinyl, 3-Pyridinyl, 4-Pyridinyl, 2-Pyrimidinyl, 4-Pyrimidinyl, 5-Pyrimidinyl, 2-Pyrazinyl, 3-Pyridazinyl, 4-Pyridazinyl, 1,2,4-Triazin-3-yl, 1,2,4-Triazin-5-yl, 1,2,4-Triazin-6-yl und 1,3,5-Triazinyl.

Im Hinblick auf die Verwendung als Fungizide sind solche Verbindungen der Formel I bevorzugt, worin die Variablen R¹, R², R³, R⁴ und R⁵ unabhängig voneinander und insbesondere in Kombination die folgenden Bedeutungen aufweisen:

Gemäß einer ersten Ausführungsform der Erfindung bedeuten R¹ und R² unabhängig voneinander monovalente Reste. R¹ und R² können dabei gleich oder verschieden sein. Sie sind dann vorzugsweise ausgewählt unter Fluor, Chlor, C₁-C₄-Alkyl, speziell Methyl, Ethyl oder n-Propyl, weiterhin Methoxy, Ethoxy, CF₃, CHF₂, OCF₃ und OCHF₂.

Gemäß einer zweiten Ausführungsform der Erfindung bilden R¹ und R² gemeinsam mit den C-Atomen, an die sie gebunden sind, einen gesättigten 5-, 6- oder 7-gliedrigen Carbocyclus oder Heterocyclus, der wie vorstehend definiert ist, und ein oder mehrere C₁-C₄-Alkygruppen als Substituenten tragen kann. In dieser Ausführungsform stehen R¹ und R² gemeinsam mit den C-Atomen des Triazinrings, an die sie gebunden sind, vorzugsweise für einen der folgenden Ringe: 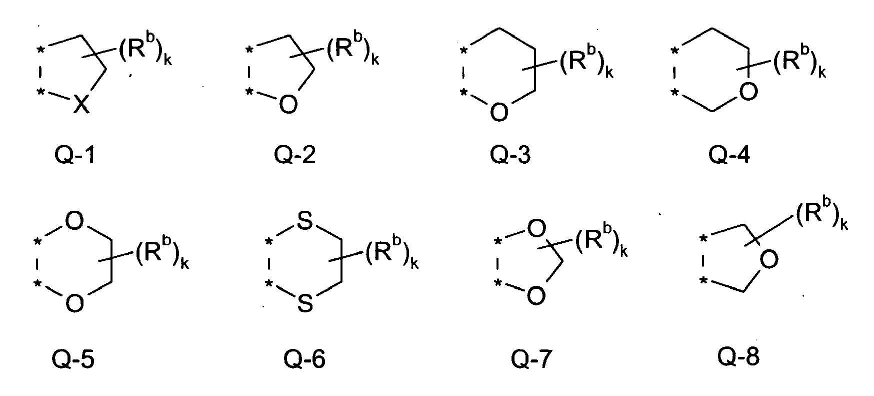 worin
- *: die Atome des Triazinrings bezeichnet;
- k: 0, 1, 2, 3 oder 4 bedeutet;
- R^{b}: für C₁-C₄-Alkyl, insbesondere für Methyl steht; und
- X: für (CH₂)ₙ steht mit n = 1, 2 oder 3.

Die Reste R^{b} können an beliebigen Kohlenstoffatomen dieser Ringe angeordnet sein und beispielsweise, wenn k ≠ 0 ist, können 1, 2, 3 oder 4 der Wasserstoffatome in (CH₂)ₙ durch R^{b} ersetzt sein. Die Orientierung der Reste Q -2, Q-3 und Q-4 bezügliche des Triazinrings ist beliebig. Unter den Resten Q-1 bis Q-8 ist insbesondere der Rest Q-1 bevorzugt und speziell Reste Q-1 mit n = 2 oder 3. Die Variable k steht insbesondere für 0, 1 oder 2.

R³ steht vorzugsweise für Wasserstoff, Fluor, Chlor, C₁-C₄-Alkyl, speziell Methyl, Ethyl, Isopropyl oder tert.-Butyl, Methoxy, Ethoxy, CF₃, CHF₂, OCF₃ oder OCHF₂, insbesondere für Wasserstoff oder Methyl. Besonders bevorzugt sind weiterhin Verbindungen der Formel I, worin R³ für Chlor steht. Besonders bevorzugt sind weiterhin Verbindungen der Formel I, worin R³ für CF₃ steht. Besonders bevorzugt sind weiterhin Verbindungen der Formel I, worin R³ für Methoxy oder Ethoxy steht.

R⁴ steht vorzugsweise für Wasserstoff, Fluor, Chlor, C₁-C₄-Alkyl, speziell Methyl oder Ethyl, Methoxy, Ethoxy, CF₃, CHF₂, OCF₃ oder OCHF₂. Insbesondere steht R⁴ für Wasserstoff, Fluor, Chlor oder Methyl.

In bevorzugten Verbindungen der Formel I steht R⁵ für Phenyl, Phenoxy oder Benzyl, wobei der Phenylring in den drei vorgenannten Resten unsubstituiert ist oder 1, 2, 3, 4 oder 5 Reste R^{a}, insbesondere 1, 2 oder 3 Reste Rₐ aufweist.

Bevorzugte Reste R^{a} sind ausgewählt ist unter Halogen, C₁-C₄-Alkyl, C₁-C₂-Halogenalkyl, C₁-C₄-Alkoxy, C₁-C₂-Halogenalkoxy, C₁-C₄-Alkylthio, C₁-C₄-Alkylcarbonyl, C₁-C₄-Alkoxycarbonyl, und Resten der Formel C(=N-O-C₁-C₈-Alkyl)R^{aa}, worin R^{aa} für Wasserstoff oder C₁-C₄-Alkyl steht. Insbesondere bevorzugt sind die Reste R^{a} ausgewählt unter Halogen, speziell Chlor oder Fluor, Methyl, Methoxy, Trifluormethyl, Difluormethyl, Trifluormethoxy, Difluormethoxy und Methylthio.

Gemäß einer bevorzugten Ausführungsform der Erfindung steht R⁵ in Formel I für Phenyl, Phenoxy oder Benzyl, worin der Phenylring 1, 2, 3, 4 oder 5 und insbesondere 1, 2 oder 3 Reste R^{a} aufweist, wobei die Reste R^{a} vorzugsweise unter den als bevorzugt genannten Resten R^{a} und insbesondere unter den als besonders bevorzugt genannten Resten R^{a} ausgewählt sind. In dieser Ausführungsform steht der Phenylrest in Phenyl, Phenoxy oder Benzyl für einen Rest der Formel P: 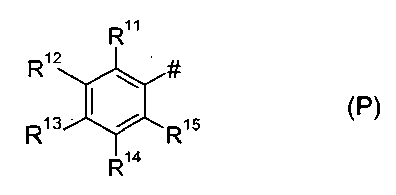 worin # die Verknüpfungsstelle mit dem Pyridinring ist und R¹¹, R¹², R¹³, R¹⁴ und R¹⁵ für Wasserstoff stehen oder wenigstens einer dieser Reste, z.B. 1, 2, 3, 4 oder 5 dieser Reste eine der für R^{a} angegebenen, insbesondere eine der als bevorzugt oder besonders bevorzugt angegebenen Bedeutungen aufweisen. In einer bevorzugten Ausführungsform ist wenigstens einer und speziell 1, 2 oder 3 der Reste R¹¹, R¹², R¹³, R¹⁴ oder R¹⁵ von Wasserstoff verschieden. Insbesondere bedeuten:
- R¹¹: Wasserstoff, Fluor, Chlor, CH₃, OCH₃, OCHF₂, OCF₃ oder CF₃;
- R¹², R¹⁴: unabhängig voneinander Wasserstoff, Chlor, Fluor, CH₃, OCH₃, OCHF₂, OCF₃ oder CF₃, wobei einer der Reste R¹² und R¹⁴ auch für NO₂, C(O)CH₃ oder COOCH₃ stehen kann; insbesondere stehen R¹² und R¹⁴ für Wasser- stoff, Fluor, Methyl oder Trifluormethyl;
- R¹³: Wasserstoff, Fluor, Chlor, Cyano, OH, CHO, NO₂, NH₂, Methylamino, Di- methylamino, Diethylamino, C₁-C₄-Alkyl, speziell CH₃, C₂H₅, CH(CH₃)₂, C₃-C₈-Cycloalkyl, speziell Cyclopropyl, Cyclopentyl oder Cyclohexyl, C₁-C₄-Alkoxy, speziell OCH₃, C₁-C₄-Alkylthio, speziell Methylthio oder Ethylthio, C₁-C₄-Haloalkyl, speziell CF₃, C₁-C₄-Haloalkoxy, speziell OCHF₂ oder OCF₃, oder CO(A²), worin A² für C₁-C₄-Alkyl, speziell Methyl oder für C₁-C₄-Alkoxy, speziell OCH₃, steht, oder eine Gruppe C(R^{13a})=NOR^{13b}, wor- in R^{13a} für Wasserstoff oder Methyl steht und R^{13b} für C₁-C₄-Alkyl, Propargyl oder Allyl steht, bedeutet oder R¹² und R¹³ gemeinsam eine Gruppe O-CH₂-O bilden; und
- R¹⁵: Wasserstoff, Fluor, Chlor, oder C₁-C₄-Alkyl, speziell CH₃, insbesondere Wasserstoff oder Fluor.

Sofern mehr als einer der Reste R¹¹, R¹², R¹³, R¹⁴ oder R¹⁵ von Wasserstoff verschieden ist, dann ist vorteilhafterweise nur einer der von Wasserstoff verschiedenen Reste von Halogen oder Methyl verschieden. Speziell wenn einer der Reste R¹¹, R¹², R¹³, R¹⁴ oder R¹⁵ von Wasserstoff, Halogen oder Methyl verschieden ist, dann sind die verbleibenden Reste R¹¹, R¹², R¹³, R¹⁴, R¹⁵ unter Halogen und Wasserstoff ausgewählt.

Beispiele für Reste P sind die im folgenden genannten Reste: Phenyl, 2-Fluorphenyl, 3-Fluorphenyl, 4-Fluorphenyl, 2-Chlorphenyl, 3-Chlorphenyl, 4-Chlorphenyl, 3-Bromphenyl, 4-Bromphenyl, 2-Trifluormethylphenyl, 3-Trifluormethylphenyl, 4-Trifluormethylphenyl, 2-(Methylthio)phenyl, 3-(Methylthio)phenyl, 4-(Methylthio)phenyl, 2-Methoxyphenyl, 3-Methoxyphenyl, 4-Methoxyphenyl, 4-Nitrophenyl, 4-Cyanophenyl, 4-tert.-Butylphenyl, 4-Isopropylphenyl, 3-Ethoxyphenyl, 4-Ethoxyphenyl, 4-n-Propoxyphenyl, 4-Isopropoxyphenyl, 3-Isopropoxyphenyl, 4-n-Butoxyphenyl, 4-tert.-Butoxyphenyl, 4-Acetylphenyl, 4-Methoxycarbonylphenyl, 4-Ethoxycarbonylphenyl, 4-tert.-Butoxycarbonylphenyl, 4-(Methoxyiminomethyl)phenyl, 4-(1-(Methoxyimino)ethyl)phenyl, 2,3-Difluorphenyl, 2,4-Difluorphenyl, 2,5-Difluorphenyl, 3,4-Difluorphenyl, 3,5-Difluorphenyl, 2,6-Difluorphenyl, 2,4,6-Trifluorphenyl, 2,4,5-Trifluorphenyl, 2,3,4-Trifluorphenyl, 2,3,5-Trifluorphenyl, 3,4,5-Trifluorphenyl, 2,3-Dichlorphenyl, 2,5-Dichlorphenyl, 3,5-Dichlorphenyl, 2,6-Dichlorphenyl, 2,3-Dimethylphenyl, 2,4-Dimethylphenyl, 2,5-Dimethylphenyl, 2,4,5-Trimethylphenyl, 2,3-Dimethoxyphenyl, 2,4-Dimethoxyphenyl, 3,4-Dimethoxyphenyl, 2,4-Bis(trifluormethyl)phenyl, 3,5-Bis(trifluormethyl)phenyl, 2-Methyl-3-methoxyphenyl, 2-Methyl-4-methoxyphenyl, 2-Methyl-6-methoxyphenyl, 3-Chlor-4-fluorphenyl, 2-Chlor-4-fluorphenyl, 2-Chlor-6-fluorphenyl, 4-Chlor-2-fluorphenyl, 5-Chlor-2-fluorphenyl, 4-Fluor-3-methylphenyl, 2-Fluor-4-methylphenyl, 4-Fluor-2-methylphenyl, 2-Fluor-3-methoxyphenyl, 2-Fluor-4-methoxyphenyl, 2-Fluor-6-methoxyphenyl, 2-Fluor-4-trifluormethylphenyl, 4-Chlor-3-methylphenyl, 2-Chlor-4-methylphenyl, 2-Chlor-6-methylphenyl, 3-Chlor-2-methylphenyl, 5-Chlor-2-methylphenyl, 2-Chlor-4-methoxyphenyl, 2-Chlor-6-methoxyphenyl, 2-Chlor-4-trifluormethylphenyl, 3-Fluor-4-methylphenyl, 4-Fluor-3-methylphenyl, 3-Fluor-4-methoxyphenyl, 3-Fluor-4-ethoxyphenyl, 3-Fluor-4-trifluormethylphenyl, 3-Chlor-4-methylphenyl, 3-Chlor-4-methoxyphenyl, 3-Chlor-4-ethoxyphenyl, 3-Chlor-4-trifluormethylphenyl, 3-Methyl-4-methoxyphenyl, 4-Chlor-2,5-difluorphenyl, 4-tert.-Butyl-2-fluorphenyl, 2-Fluor-4-isopropylphenyl, 4-Ethoxy-2-fluorphenyl, 4-Acetyl-2-fluorphenyl, 2-Methylphenyl, 3-Methylphenyl, 4-Methylphenyl, 2-Ethylphenyl, 3-Ethylphenyl, 4-Ethylphenyl.

Besonders bevorzugt steht R⁵ in Formel I für Phenyl und speziell für einen Rest P und insbesondere für einen der hier als Beispiel angegebenen Reste P.

Gemäß einer weiteren bevorzugten Ausführungsform steht R⁵ für C₁-C₆-Alkyl oder C₁-C₆-Haloalkyl, insbesondere für C₃-C₆-Alkyl, speziell n-Propyl, Isopropyl, tert.-Butyl, 1 ,2-Dimethylpropyl oder 1,2,2-Trimethylpropyl, oder für Trifluormethyl.

Gemäß einer weiteren bevorzugten Ausführungsform steht R⁵ für 5-gliedriges Heteroaryl, das neben Kohlenstoff 1, 2, 3 oder 4 Stickstoffatome als Ringatome aufweist; oder für 5-gliedriges Heteroaryl, das neben Kohlenstoff 1 unter Sauerstoff und Schwefel ausgewähltes Heteroatom und gegebenenfalls 1, 2 oder 3 Stickstoffatome als Ringatome aufweist, oder für 6-gliedriges Hetaryl, das 1, 2, 3 oder 4 Stickstoffatome als Ringatome aufweist, wobei 5- und 6-gliedriges Hetaryl unsubstituiert sein kann oder die Wasserstoffatome im unsubstituierten Hetaryl teilweise oder vollständig durch Substituenten R^{a} der oben bezeichneten Art ersetzt sein können, so dass die Gesamtzahl aller Substituenten R^{a} an Hetaryl typischerweise 1, 2, 3 oder 4 beträgt. Substituenten an Stickstoff-Ringatomen sind insbesondere C-gebundene Reste R^{a} und speziell C₁-C₄-Alkyl.

In dieser Ausführungsform steht R⁵ vorzugsweise für gegebenenfalls substituiertes 2-Furyl, 3-Furyl, 2-Thienyl, 3-Thienyl, 2-Pyridyl, 3-Pyridyl, 4-Pyridyl, 2-Pyrimidinyl, 4-Pyrimidinyl oder 5-Pyrimidinyl, wobei die vorgenannten heterocyclischen Reste vorzugsweise unsubstituiert sind oder 1, 2 oder 3 Substituenten R^{a} aufweisen. Bezüglich der bevorzugten und besonders bevorzugten Reste gilt das zuvor Gesagte.

Beispiele für bevorzugte heteroaromatische Reste R⁵ sind
- gegebenenfalls substituiertes 2-Thienyl, wie unsubstituiertes 2-Thienyl, 5-Methylthiophen-2-yl, 4-Methylthiophen-2-yl, 5-Chlorthiophen-2-yl, 3-Cyanothiophen-2-yl, 5-Formylthiophen-2-yl, 5-Acetylthiophen-2-yl, 5-(Methoxyiminomethyl)thiophen-2-yl, 5-(1-(Methoxyimino)ethyl)thiophen-2-yl, 4-Bromthiophen-2-yl, 3,5-Dichlorthiophen-2-yl,
- gegebenenfalls substituiertes 3-Thienyl, wie unsubstituiertes 3-Thienyl, 2-Methylthiophen-3-yl, 2,5-Dichlorthiophen-3-yl, 2,4,5-Trichlor-thiophen-3-yl,
- gegebenenfalls substituiertes 2-Furyl, wie unsubstituiertes 2-Furyl, 5-Methylfuran-2-yl, 5-Chlorfuran-2-yl, 4-Methylfuran-2-yl, 3-Cyanofuran-2-yl, 5-Acetylfuran-2-yl,
- gegebenenfalls substituiertes 3-Furyl, wie unsubstituiertes 3-Furyl, 2-Methylfuran-3-yl, 2,5-Dimethylfuran-3-yl,
- gegebenenfalls substituiertes 2-Pyridyl, wie unsubstituiertes 2-Pyridyl, 3-Fluor-pyridin-2-yl, 3-Chlor-pyridin-2-yl, 3-Brompyridin-2-yl, 3-Trifluormethyl-pyridin-2-yl, 3-Methyl-pyridin-2-yl, 3-Ethyl-pyridin-2-yl, 3,5-Difluor-pyridin-2-yl, 3,5-Dichlor-pyridin-2-yl, 3,5-Dibrom-pyridin-2-yl, 3,5-Dimethyl-pyridin-2-yl, 3-Fluor-5-trifluormethyl-pyridin-2-yl, 3-Chlor-5-fluor-pyridin-2-yl, 3-Chlor-5-methyl-pyridin-2-yl, 3-Fluor-5-chlor-pyridin-2-yl, 3-Fluor-5-methyl-pyridin-2-yl, 3-Methyl-5-fluor-pyridin-2-yl, 3-Methyl-5-chlor-pyridin-2-yl, 5-Nitro-pyridin-2-yl, 5-Cyano-pyridin-2-yl, 5-Methoxycarbonyl-pyridin-2-yl, 5-Trifluormethyl-pyridin-2-yl, 5-Methyl-pyridin-2-yl, 4-Methyl-pyridin-2-yl, 6-Methyl-pyridin-2-yl,
- gegebenenfalls substituiertes 3-Pyridyl, wie unsubstituiertes 3-Pyridyl, 2-Chlor-pyridin-3-yl, 2-Brom-pyridin-3-yl, 2-Methyl-pyridin-3-yl, 2,4-Dichlor-pyridin-3-yl, 2,4-Dibrom-pyridin-3-yl, 2,4-Difluorpyridin-3-yl, 2-Fluor-4-chlorpyridin-3-yl, 2-Chlor-4-fluor-pyridin-3-yl, 2-Chlor-4-methyl-pyridin-3-yl, 2-Methyl-4-fluor-pyridin-3-yl, 2-Methyl-4-chlor-pyridin-3-yl, 2,4-Dimethyl-pyridin-3-yl, 2,4,6-Trichlorpyridin-3-yl, 2,4,6-Tribrompyridin-3-yl, 2,4,6-Trimethyl-pyridin-3-yl, 2,4-Dichlor-6-methylpyridin-3-yl, 6-Methoxypyridin-3-yl, 6-Chlorpyridin-3-yl,
- gegebenenfalls substituiertes 4-Pyridyl, wie unsubstituiertes 4-Pyridyl, 3-Chlorpyridin-4-yl, 3-Brom-pyridin-4-yl, 3-Methyl-pyridin-4-yl, 3,5-Dichlor-pyridin-4-yl, 3,5-Dibrom-pyridin-4-yl, 3,5-Dimethyl-pyridin-4-yl,
- gegebenenfalls substituiertes 4-Pyrimidinyl, wie unsubstituiertes 4-Pyrimidinyl, 5-Chlorpyrimidin-4-yl, 5-Fluorpyrimidin-4-yl, 5-Fluor-6-chlorpyrimidin-4-yl, 2-Methyl-6-trifluormethyl-pyrimidin-4-yl, 2,5-Dimethyl-6-trifluormethyl-pyrimidin-4-yl, 5-Methyl-6-trifluormethyl-pyrimidin-4-yl, 6-Trifluormethyl-pyrimidin-4-yl, 2-Methyl-5-fluor-pyrimidin-4-yl, 2-Methyl-5-chlor-pyrimidin-4-yl, 5-Chlor-6-methyl-pyrimdin-4-yl, 5-Chlor-6-ethyl-pyrimdin-4-yl, 5-Chlor-6-isopropyl-pyrimdin-4-yl, 5-Brom-6-methyl-pyrimidin-4-yl, 5-Fluor-6-methyl-pyrimidin-4-yl, 5-Fluor-6-fluormethyl-pyrimidin-4-yl, 2,6-Dimethyl-5-chlor-pyrimdin-4-yl, 5,6-Dimethyl-pyrimidin-4-yl, 2,5-Dimethyl-pyrimidin-4-yl, 2,5,6-Trimethyl-pyrimidin-4-yl, 5-Methyl-6-methoxy-pyrimidin-4-yl,
- gegebenenfalls substituiertes 5-Pyrimidinyl, wie unsubstituiertes 5-Pyrimidinyl, 4-Methyl-pyrimidin-5-yl, 4,6-Dimethyl-pyrimidin-5-yl, 2,4,6-Trimethylpyrimidin-5-yl, 4-Trifluormethyl-6-methyl-pyrimidin-5-yl,
- gegebenenfalls substituiertes 2-Pyrimidinyl, wie unsubstituiertes 2-Pyrimidinyl, 4,6-Dimethylpyrimidin-2-yl, 4,5,6-Trimethylpyrimidin-2-yl, 4,6-Ditrifluormethyl-pyrimidin-2-yl und 4,6-Dimethyl-5-chlor-pyrimidin-2-yl.

Insbesondere sind die folgenden Gruppen von Verbindungen der Formel I bevorzugt:

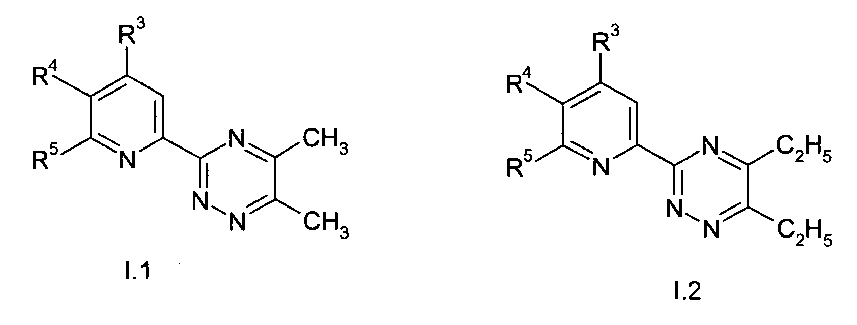

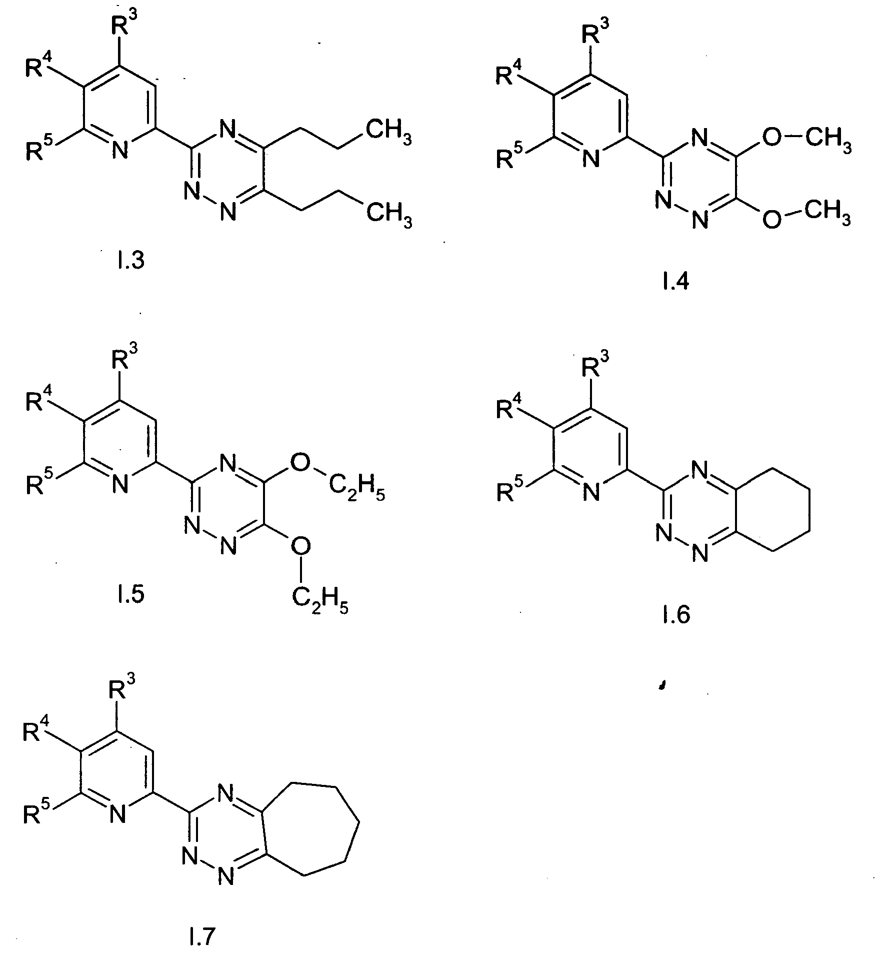

Insbesondere sind im Hinblick auf ihre Verwendung die in den folgenden Tabellen 1 bis 9 genannten Verbindungen der allgemeinen Formeln I.1, I.2, I.3, 1.4, I.5, 1.6 und 1.7 bevorzugt.

### Tabelle 1

Verbindungen der Formeln I.1, I.2, 1.3, I.4, I.5, I.6 und I.7, in denen R³ Wasserstoff bedeutet und die Kombination von R⁴ und R⁵ für eine Verbindung jeweils einer Zeile der Tabelle A entspricht.

### Tabelle 2

Verbindungen der Formeln I.1, I.2, I.3, I.4, I.5, 1.6 und I.7, in denen R³ Methyl bedeutet und die Kombination von R⁴ und R⁵ für eine Verbindung jeweils einer Zeile der Tabelle A entspricht.

### Tabelle 3

Verbindungen der Formeln I.1, I.2, I.3, I.4, I.5, I.6 und 1.7, in denen R³ Ethyl bedeutet und die Kombination von R⁴ und R⁵ für eine Verbindung jeweils einer Zeile der Tabelle A entspricht.

### Tabelle 4

Verbindungen der Formeln I.1, 1.2, I.3, I.4, I.5, I.6 und 1.7, in denen R³ Methoxy bedeutet und die Kombination von R⁴ und R⁵ für eine Verbindung jeweils einer Zeile der Tabelle A entspricht.

### Tabelle 5

Verbindungen der Formeln I.1, 1.2, I.3, I.4, I.5, I.6 und 1.7, in denen R³ Ethoxy bedeutet und die Kombination von R⁴ und R⁵ für eine Verbindung jeweils einer Zeile der Tabelle A entspricht.

### Tabelle 6

Verbindungen der Formeln I.1, I.2, I.3, I.4, I.5, 1.6 und 1.7 in denen R³ iso-Propyl bedeutet und die Kombination von R⁴ und R⁵ für eine Verbindung jeweils einer Zeile der Tabelle A entspricht.

### Tabelle 7

Verbindungen der Formeln I.1, I.2, I.3, I.4, 1.5, 1.6 und I.7, in denen R³ tert.-Butyl bedeutet und die Kombination von R⁴ und R⁵ für eine Verbindung jeweils einer Zeile der Tabelle A entspricht.

### Tabelle 8

Verbindungen der Formeln I.1, I.2, I.3, I.4, I.5, 1.6 und 1.7 in denen R³ Trifluormethyl bedeutet und die Kombination von R⁴ und R⁵ für eine Verbindung jeweils einer Zeile der Tabelle A entspricht.

### Tabelle 9

Verbindungen der Formeln I.1, 1.2, I.3, I.4, I.5, 1.6 und 1.7, in denen R³ Difluormethoxy bedeutet und die Kombination von R⁴ und R⁵ für eine Verbindung jeweils einer Zeile der Tabelle A entspricht.

**Tabelle A**

| Nr. | R⁴ | R⁵ |
|---|---|---|
| A-1. | H | Phenyl |
| A-2. | H | 2-Fluorphenyl |
| A-3. | H | 3-Fluorphenyl |
| A-4. | H | 4-Fluorphenyl |
| A-5. | H | 2-Chlorphenyl |
| A-6. | H | 3-Chlorphenyl |
| A-7. | H | 4-Chlorphenyl |
| A-8. | H | 3-Bromphenyl |
| A-9. | H | 4-Bromphenyl |
| A-10. | H | 2-Trifluormethylphenyl |
| A-11. | H | 3-Trifluormethylphenyl |
| A-12. | H | 4-Trifluormethylphenyl |
| A-13. | H | 2-(Methylthio)phenyl |
| A-14. | H | 3-(Methylthio)phenyl |
| A-15. | H | 4-(Methylthio)phenyl |
| A-16. | H | 2-Methoxyphenyl |
| A-17. | H | 3-Methoxyphenyl |
| A-18. | H | 4-Methoxyphenyl |
| A-19. | H | 4-Nitrophenyl |
| A-20. | H | 4-Cyanophenyl |
| A-21. | H | 4-tert.-Butylphenyl |
| A-22. | H | 4-Isopropylphenyl |
| A-23. | H | 3-Ethoxyphenyl |
| A-24. | H | 4-Ethoxyphenyl |
| A-25. | H | 4-n-Propoxyphenyl |
| A-26. | H | 3-Isopropoxyphenyl |
| A-27. | H | 4-Isopropoxyphenyl |
| A-28. | H | 4-n-Butoxyphenyl |
| A-29. | H | 4-tert.-Butoxyphenyl |
| A-30. | H | 4-(Methoxyiminomethyl)phenyl |
| A-31. | H | 4-(1-(Methoxyimino)ethyl)phenyl |
| A-32. | H | 2,3-Difluorphenyl |
| A-33. | H | 2,4-Difluorphenyl |
| A-34. | H | 2,5-Difluorphenyl |
| A-35. | H | 3,4-Difluorphenyl |
| A-36. | H | 3,5-Difluorphenyl |
| A-37. | H | 2,6-Difluorphenyl |
| A-38. | H | 2,4,6-Trifluorphenyl |
| A-39. | H | 2,4,5-Trifluorphenyl |
| A-40. | H | 2,3,4-Trifluorphenyl |
| A-41. | H | 2,3,5-Trifluorphenyl |
| A-42. | H | 3,4,5-Trifluorphenyl |
| A-43. | H | 2,3-Dichlorphenyl |
| A-44. | H | 2,5-Dichlorphenyl |
| A-45. | H | 3,5-Dichlorphenyl |
| A-46. | H | 2,6-Dichlorphenyl |
| A-47. | H | 2,3-Dimethylphenyl |
| A-48. | H | 2,4-Dimethylphenyl |
| A-49. | H | 2,5-Dimethylphenyl |
| A-50. | H | 2,4,5-Trimethylphenyl |
| A-51. | H | 2,3-Dimethoxyphenyl |
| A-52. | H | 2,4-Dimethoxyphenyl |
| A-53. | H | 3,4-Dimethoxyphenyl |
| A-54. | H | 2,4-Bis(trifluormethyl)phenyl |
| A-55. | H | 3,5-Bis(trifluormethyl)phenyl |
| A-56. | H | 2-Methyl-3-methoxyphenyl |
| A-57. | H | 2-Methyl-4-methoxyphenyl |
| A-58. | H | 2-Methyl-6-methoxyphenyl |
| A-59. | H | 3-Chlor-4-fluorphenyl |
| A-60. | H | 2-Chlor-4-fluorphenyl |
| A-61. | H | 2-Chlor-6-fluorphenyl |
| A-62. | H | 4-Chlor-2-fluorphenyl |
| A-63. | H | 5-Chlor-2-fluorphenyl |
| A-64. | H | 4-Fluor-3-methylphenyl |
| A-65. | H | 2-Fluor-4-methylphenyl |
| A-66. | H | 4-Fluor-2-methylphenyl |
| A-67. | H | 2-Fluor-3-methoxyphenyl |
| A-68. | H | 2-Fluor-4-methoxyphenyl |
| A-69. | H | 2-Fluor-6-methoxyphenyl |
| A-70. | H | 2-Fluor-4-trifluormethylphenyl |
| A-71. | H | 4-Chlor-3-methylphenyl |
| A-72. | H | 2-Chlor-4-methylphenyl |
| A-73. | H | 2-Chlor-6-methylphenyl |
| A-74. | H | 5-Chlor-2-methylphenyl |
| A-75. | H | 3-Chlor-2-methylphenyl |
| A-76. | H | 2-Chlor-4-methoxyphenyl |
| A-77. | H | 2-Chlor-6-methoxyphenyl |
| A-78. | H | 2-Chlor-4-trifluormethylphenyl |
| A-79. | H | 3-Fluor-4-methylphenyl |
| A-80. | H | 3-Fluor-4-isopropylphenyl |
| A-81. | H | 4-Fluor-3-methylphenyl |
| A-82. | H | 3-Fluor-4-methoxyphenyl |
| A-83. | H | 3-Fluor-4-ethoxyphenyl |
| A-84. | H | 3-Fluor-4-trifluormethylphenyl |
| A-85. | H | 3-Chlor-4-methylphenyl |
| A-86. | H | 3-Chlor-4-methoxyphenyl |
| A-87. | H | 3-Chlor-4-ethoxyphenyl |
| A-88. | H | 3-Chlor-4-trifluormethylphenyl |
| A-89. | H | 3-Methyl-4-methoxyphenyl |
| A-90. | H | 4-Chlor-2,5-difluorphenyl |
| A-91. | H | 4-tert.-Butyl-2-fluorphenyl |
| A-92. | H | 2-Fluor-4-isopropylphenyl |
| A-93. | H | 4-Ethoxy-2-fluorphenyl |
| A-94. | H | 4-Acetyl-2-fluorphenyl |
| A-95. | H | 2-Thienyl |
| A-96. | H | 5-Methylthiophen-2-yl |
| A-97. | H | 4-Methylthiophen-2-yl |
| A-98. | H | 5-Chlorthiophen-2-yl |
| A-99. | H | 3-Cyanothiophen-2-yl |
| A-100. | H | 5-Formylthiophen-2-yl |
| A-101. | H | 5-Acetylthiophen-2-yl |
| A-102. | H | 5-(Methoxyiminomethyl)thiophen-2-yl |
| A-103. | H | 5-(1-(Methoxyimino)ethyl)thiophen-2-yl |
| A-104. | H | 4-Bromthiophen-2-yl |
| A-105. | H | 3,5-Dichlorthiophen-2-yl |
| A-106. | H | 3-Thienyl |
| A-107. | H | 2-Methylthiophen-3-yl |
| A-108. | H | 2,5-Dichlorthiophen-3-yl |
| A-109. | H | 2,4,5-Trichlor-thiophen-3-yl |
| A-110. | H | 2-Furyl |
| A-111. | H | 5-Methylfuran-2-yl |
| A-112. | H | 5-Chlorfuran-2-yl |
| A-113. | H | 4-Methylfuran-2-yl |
| A-114. | H | 3-Cyanofuran-2-yl |
| A-115. | H | 5-Acetylfuran-2-yl |
| A-116. | H | 3-Furyl |
| A-117. | H | 2-Methylfuran-3-yl |
| A-118. | H | 2,5-Dimethylfuran-3-yl |
| A-119. | H | 2-Pyridyl |
| A-120. | H | 3-Fluor-pyridin-2-yl |
| A-121. | H | 3-Chlor-pyridin-2-yl |
| A-122. | H | 3-Brompyridin-2-yl |
| A-123. | H | 3-Trifluormethyl-pyridin-2-yl |
| A-124. | H | 3-Methyl-pyridin-2-yl |
| A-125. | H | 3-Ethyl-pyridin-2-yl |
| A-126. | H | 3,5-Difluor-pyridin-2-yl |
| A-127. | H | 3,5-Dichlor-pyridin-2-yl |
| A-128. | H | 3,5-Dibrom-pyridin-2-yl |
| A-129. | H | 3,5-Dimethyl-pyridin-2-yl |
| A-130. | H | 3-Fluor-5-trifluormethyl-pyridin-2-yl |
| A-131. | H | 3-Chlor-5-fluor-pyridin-2-yl |
| A-132. | H | 3-Chlor-5-methyl-pyridin-2-yl |
| A-133. | H | 3-Fluor-5-chlor-pyridin-2-yl |
| A-134. | H | 3-Fluor-5-methyl-pyridin-2-yl |
| A-135. | H | 3-Methyl-5-fluor-pyridin-2-yl |
| A-136. | H | 3-Methyl-5-chlor-pyridin-2-yl |
| A-137. | H | 5-Nitro-pyridin-2-yl |
| A-138. | H | 5-Cyano-pyridin-2-yl |
| A-139. | H | 5-Methoxycarbonyl-pyridin-2-yl |
| A-140. | H | 5-Trifluormethyl-pyridin-2-yl |
| A-141. | H | 5-Methyl-pyridin-2-yl |
| A-142. | H | 4-Methyl-pyridin-2-yl |
| A-143. | H | 6-Methyl-pyridin-2-yl |
| A-144. | H | 3-Pyridyl |
| A-145. | H | 2-Chlor-pyridin-3-yl |
| A-146. | H | 2-Brom-pyridin-3-yl |
| A-147. | H | 2-Methyl-pyridin-3-yl |
| A-148. | H | 2,4-Dichlor-pyridin-3-yl |
| A-149. | H | 2,4-Dibrom-pyridin-3-yl |
| A-150. | H | 2,4-Difluorpyridin-3-yl |
| A-151. | H | 2-Fluor-4-chlorpyridin-3-yl |
| A-152. | H | 2-Chlor-4-fluor-pyridin-3-yl |
| A-153. | H | 2-Chlor-4-methyl-pyridin-3-yl |
| A-154. | H | 2-Methyl-4-fluor-pyridin-3-yl |
| A-155. | H | 2-Methyl-4-chlor-pyridin-3-yl |
| A-156. | H | 2,4-Dimethyl-pyridin-3-yl |
| A-157. | H | 2,4,6-Trichlorpyridin-3-yl |
| A-158. | H | 2,4,6-Tribrompyridin-3-yl |
| A-159. | H | 2,4,6-Trimethyl-pyridin-3-yl |
| A-160. | H | 2,4-Dichlor-6-methylpyridin-3-yl |
| A-161. | H | 4-Pyridyl |
| A-162. | H | 3-Chlor-pyridin-4-yl |
| A-163. | H | 3-Brom-pyridin-4-yl |
| A-164. | H | 3-Methyl-pyridin-4-yl |
| A-165. | H | 3,5-Dichlor-pyridin-4-yl |
| A-166. | H | 3,5-Dibrom-pyridin-4-yl |
| A-167. | H | 3,5-Dimethyl-pyridin-4-yl |
| A-168. | H | 4-Pyrimidinyl |
| A-169. | H | 5-Chlorpyrimidin-4-yl |
| A-170. | H | 5-Fluorpyrimidin-4-yl |
| A-171. | H | 5-Fluor-6-chlorpyrimidin-4-yl |
| A-172. | H | 2-Methyl-6-trifluormethyl-pyrimidin-4-yl |
| A-173. | H | 2,5-Dimethyl-6-trifluormethyl-pyrimidin-4-yl |
| A-174. | H | 5-Methyl-6-trifluormethyl-pyrimidin-4-yl |
| A-175. | H | 6-Trifluormethyl-pyrimidin-4-yl |
| A-176. | H | 2-Methyl-5-fluor-pyrimidin-4-yl |
| A-177. | H | 2-Methyl-5-chlor-pyrimidin-4-yl |
| A-178. | H | 5-Chlor-6-methyl-pyrimdin-4-yl |
| A-179. | H | 5-Chlor-6-ethyl-pyrimdin-4-yl |
| A-180. | H | 5-Chlor-6-isopropyl-pyrimdin-4-yl |
| A-181. | H | 5-Brom-6-methyl-pyrimidin-4-yl |
| A-182. | H | 5-Fluor-6-methyl-pyrimidin-4-yl |
| A-183. | H | 5-Fluor-6-fluormethyl-pyrimidin-4-yl |
| A-184. | H | 2,6-Dimethyl-5-chlor-pyrimdin-4-yl |
| A-185. | H | 5,6-Dimethyl-pyrimidin-4-yl |
| A-186. | H | 2,5-Dimethyl-pyrimidin-4-yl |
| A-187. | H | 2,5,6-Trimethyl-pyrimidin-4-yl |
| A-188. | H | 5-Methyl-6-methoxy-pyrimidin-4-yl |
| A-189. | H | 5-Pyrimidinyl |
| A-190. | H | 4-Methyl-pyrimidin-5-yl |
| A-191. | H | 4,6-Dimethyl-pyrimidin-5-yl |
| A-192. | H | 2,4,6-Trimethylpyrimidin-5-yl |
| A-193. | H | 4-Trifluormethyl-6-methyl-pyrimidin-5-yl |
| A-194. | H | 2-Pyrimidinyl |
| A-195. | H | 4,6-Dimethylpyrimidin-2-yl |
| A-196. | H | 4,5,6-Trimethylpyrimidin-2-yl |
| A-197. | H | 4,6-Ditrifluormethyl-pyrimidin-2-yl |
| A-198. | H | 4,6-Dimethyl-5-chlor-pyrimidin-2-yl |
| A-199. | CH₃ | Phenyl |
| A-200. | CH₃ | 2-Fluorphenyl |
| A-201. | CH₃ | 3-Fluorphenyl |
| A-202. | CH₃ | 4-Fluorphenyl |
| A-203. | CH₃ | 2-Chlorphenyl |
| A-204. | CH₃ | 3-Chlorphenyl |
| A-205. | CH₃ | 4-Chlorphenyl |
| A-206. | CH₃ | 3-Bromphenyl |
| A-207. | CH₃ | 4-Bromphenyl |
| A-208. | CH₃ | 2-Trifluormethylphenyl |
| A-209. | CH₃ | 3-Trifluormethylphenyl |
| A-210. | CH₃ | 4-Trifluormethylphenyl |
| A-211. | CH₃ | 2-(Methylthio)phenyl |
| A-212. | CH₃ | 3-(Methylthio)phenyl |
| A-213. | CH₃ | 4-(Methylthio)phenyl |
| A-214. | CH₃ | 2-Methoxyphenyl |
| A-215. | CH₃ | 3-Methoxyphenyl |
| A-216. | CH₃ | 4-Methoxyphenyl |
| A-217. | CH₃ | 4-Nitrophenyl |
| A-218. | CH₃ | 4-Cyanophenyl |
| A-219. | CH₃ | 4-tert.-Butylphenyl |
| A-220. | CH₃ | 4-Isopropylphenyl |
| A-221. | CH₃ | 3-Ethoxyphenyl |
| A-222. | CH₃ | 4-Ethoxyphenyl |
| A-223. | CH₃ | 4-n-Propoxyphenyl |
| A-224. | CH₃ | 3-Isopropoxyphenyl |
| A-225. | CH₃ | 4-Isopropoxyphenyl |
| A-226. | CH₃ | 4-n-Butoxyphenyl |
| A-227. | CH₃ | 4-tert.-Butoxyphenyl |
| A-228. | CH₃ | 4-(Methoxyiminomethyl)phenyl |
| A-229. | CH₃ | 4-(1-(Methoxyimino)ethyl)phenyl |
| A-230. | CH₃ | 2,3-Difluorphenyl |
| A-231. | CH₃ | 2,4-Difluorphenyl |
| A-232. | CH₃ | 2,5-Difluorphenyl |
| A-233. | CH₃ | 3,4-Difluorphenyl |
| A-234. | CH₃ | 3,5-Difluorphenyl |
| A-235. | CH₃ | 2,6-Difluorphenyl |
| A-236. | CH₃ | 2,4,6-Trifluorphenyl |
| A-237. | CH₃ | 2,4,5-Trifluorphenyl |
| A-238. | CH₃ | 2,3,4-Trifluorphenyl |
| A-239. | CH₃ | 2,3,5-Trifluorphenyl |
| A-240. | CH₃ | 3,4,5-Trifluorphenyl |
| A-241. | CH₃ | 2,3-Dichlorphenyl |
| A-242. | CH₃ | 2,5-Dichlorphenyl |
| A-243. | CH₃ | 3,5-Dichlorphenyl |
| A-244. | CH₃ | 2,6-Dichlorphenyl |
| A-245. | CH₃ | 2,3-Dimethylphenyl |
| A-246. | CH₃ | 2,4-Dimethylphenyl |
| A-247. | CH₃ | 2,5-Dimethylphenyl |
| A-248. | CH₃ | 2,4,5-Trimethylphenyl |
| A-249. | CH₃ | 2,3-Dimethoxyphenyl |
| A-250. | CH₃ | 2,4-Dimethoxyphenyl |
| A-251. | CH₃ | 3,4-Dimethoxyphenyl |
| A-252. | CH₃ | 2,4-Bis(trifluormethyl)phenyl |
| A-253. | CH₃ | 3,5-Bis(trifluormethyl)phenyl |
| A-254. | CH₃ | 2-Methyl-3-methoxyphenyl |
| A-255. | CH₃ | 2-Methyl-4-methoxyphenyl |
| A-256. | CH₃ | 2-Methyl-6-methoxyphenyl |
| A-257. | CH₃ | 3-Chlor-4-fluorphenyl |
| A-258. | CH₃ | 2-Chlor-4-fluorphenyl |
| A-259. | CH₃ | 2-Chlor-6-fluorphenyl |
| A-260. | CH₃ | 4-Chlor-2-fluorphenyl |
| A-261. | CH₃ | 5-Chlor-2-fluorphenyl |
| A-262. | CH₃ | 4-Fluor-3-methylphenyl |
| A-263. | CH₃ | 2-Fluor-4-methylphenyl |
| A-264. | CH₃ | 4-Fluor-2-methylphenyl |
| A-265. | CH₃ | 2-Fluor-3-methoxyphenyl |
| A-266. | CH₃ | 2-Fluor-4-methoxyphenyl |
| A-267. | CH₃ | 2-Fluor-6-methoxyphenyl |
| A-268. | CH₃ | 2-Fluor-4-trifluormethylphenyl |
| A-269. | CH₃ | 4-Chlor-3-methylphenyl |
| A-270. | CH₃ | 2-Chlor-4-methylphenyl |
| A-271. | CH₃ | 2-Chlor-6-methylphenyl |
| A-272. | CH₃ | 5-Chlor-2-methylphenyl |
| A-273. | CH₃ | 3-Chlor-2-methylphenyl |
| A-274. | CH₃ | 2-Chlor-4-methoxyphenyl |
| A-275. | CH₃ | 2-Chlor-6-methoxyphenyl |
| A-276. | CH₃ | 2-Chlor-4-trifluormethylphenyl |
| A-277. | CH₃ | 3-Fluor-4-methylphenyl |
| A-278. | CH₃ | 3-Fluor-4-isopropylphenyl |
| A-279. | CH₃ | 4-Fluor-3-methylphenyl |
| A-280. | CH₃ | 3-Fluor-4-methoxyphenyl |
| A-281. | CH₃ | 3-Fluor-4-ethoxyphenyl |
| A-282. | CH₃ | 3-Fluor-4-trifluormethylphenyl |
| A-283. | CH₃ | 3-Chlor-4-methylphenyl |
| A-284. | CH₃ | 3-Chlor-4-methoxyphenyl |
| A-285. | CH₃ | 3-Chlor-4-ethoxyphenyl |
| A-286. | CH₃ | 3-Chlor-4-trifluormethylphenyl |
| A-287. | CH₃ | 3-Methyl-4-methoxyphenyl |
| A-288. | CH₃ | 4-Chlor-2,5-difluorphenyl |
| A-289. | CH₃ | 4-tert.-Butyl-2-fluorphenyl |
| A-290. | CH₃ | 2-Fluor-4-isopropylphenyl |
| A-291. | CH₃ | 4-Ethoxy-2-fluorphenyl |
| A-292. | CH₃ | 4-Acetyl-2-fluorphenyl |
| A-293. | CH₃ | 2-Thienyl |
| A-294. | CH₃ | 5-Methylthiophen-2-yl |
| A-295. | OCH₃ | 4-Methylthiophen-2-yl |
| A-296. | CH₃ | 5-Chlorthiophen-2-yl |
| A-297. | CH₃ | 3-Cyanothiophen-2-yl |
| A-298. | CH₃ | 5-Formylthiophen-2-yl |
| A-299. | CH₃ | 5-Acetylthiophen-2-yl |
| A-300. | CH₃ | 5-(Methoxyiminomethyl)thiophen-2-yl |
| A-301. | CH₃ | 5-(1-(Methoxyimino)ethyl)thiophen-2-yl |
| A-302. | CH₃ | 4-Bromthiophen-2-yl |
| A-303. | CH₃ | 3,5-Dichlorthiophen-2-yl |
| A-304. | CH₃ | 3-Thienyl |
| A-305. | CH₃ | 2-Methylthiophen-3-yl |
| A-306. | CH₃ | 2,5-Dichlorthiophen-3-yl |
| A-307. | CH₃ | 2,4,5-Trichlor-thiophen-3-yl |
| A-308. | CH₃ | 2-Furyl |
| A-309. | CH₃ | 5-Methylfuran-2-yl |
| A-310. | CH₃ | 5-Chlorfuran-2-yl |
| A-311. | CH₃ | 4-Methylfuran-2-yl |
| A-312. | CH₃ | 3-Cyanofuran-2-yl |
| A-313. | CH₃ | 5-Acetylfuran-2-yl |
| A-314. | CH₃ | 3-Furyl |
| A-315. | CH₃ | 2-Methylfuran-3-yl |
| A-316. | CH₃ | 2,5-Dimethylfuran-3-yl |
| A-317. | CH₃ | 2-Pyridyl |
| A-318. | CH₃ | 3-Fluor-pyridin-2-yl |
| A-319. | CH₃ | 3-Chlor-pyridin-2-yl |
| A-320. | CH₃ | 3-Brompyridin-2-yl |
| A-321. | CH₃ | 3-Trifluormethyl-pyridin-2-yl |
| A-322. | CH₃ | 3-Methyl-pyridin-2-yl |
| A-323. | CH₃ | 3-Ethyl-pyridin-2-yl |
| A-324. | CH₃ | 3,5-Difluor-pyridin-2-yl |
| A-325. | CH₃ | 3,5-Dichlor-pyridin-2-yl |
| A-326. | CH₃ | 3,5-Dibrom-pyridin-2-yl |
| A-327. | CH₃ | 3,5-Dimethyl-pyridin-2-yl |
| A-328. | CH₃ | 3-Fluor-5-trifluormethyl-pyridin-2-yl |
| A-329. | CH₃ | 3-Chlor-5-fluor-pyridin-2-yl |
| A-330. | CH₃ | 3-Chlor-5-methyl-pyridin-2-yl |
| A-331. | CH₃ | 3-Fluor-5-chlor-pyridin-2-yl |
| A-332. | CH₃ | 3-Fluor-5-methyl-pyridin-2-yl |
| A-333. | CH₃ | 3-Methyl-5-fluor-pyridin-2-yl |
| A-334. | CH₃ | 3-Methyl-5-chlor-pyridin-2-yl |
| A-335. | CH₃ | 5-Nitro-pyridin-2-yl |
| A-336. | CH₃ | 5-Cyano-pyridin-2-yl |
| A-337. | CH₃ | 5-Methoxycarbonyl-pyridin-2-yl |
| A-338. | CH₃ | 5-Trifluormethyl-pyridin-2-yl |
| A-339. | CH₃ | 5-Methyl-pyridin-2-yl |
| A-340. | CH₃ | 4-Methyl-pyridin-2-yl |
| A-341. | CH₃ | 6-Methyl-pyridin-2-yl |
| A-342. | CH₃ | 3-Pyridyl |
| A-343. | CH₃ | 2-Chlor-pyridin-3-yl |
| A-344. | CH₃ | 2-Brom-pyridin-3-yl |
| A-345. | CH₃ | 2-Methyl-pyridin-3-yl |
| A-346. | CH₃ | 2,4-Dichlor-pyridin-3-yl |
| A-347. | CH₃ | 2,4-Dibrom-pyridin-3-yl |
| A-348. | CH₃ | 2,4-Difluorpyridin-3-yl |
| A-349. | CH₃ | 2-Fluor-4-chlorpyridin-3-yl |
| A-350. | CH₃ | 2-Chlor-4-fluor-pyridin-3-yl |
| A-351. | CH₃ | 2-Chlor-4-methyl-pyridin-3-yl |
| A-352. | CH₃ | 2-Methyl-4-fluor-pyridin-3-yl |
| A-353. | CH₃ | 2-Methyl-4-chlor-pyridin-3-yl |
| A-354. | CH₃ | 2,4-Dimethyl-pyridin-3-yl |
| A-355. | CH₃ | 2,4,6-Trichlorpyridin-3-yl |
| A-356. | CH₃ | 2,4,6-Tribrompyridin-3-yl |
| A-357. | CH₃ | 2,4,6-Trimethyl-pyridin-3-yl |
| A-358. | CH₃ | 2,4-Dichlor-6-methylpyridin-3-yl |
| A-359. | CH₃ | 4-Pyridyl |
| A-360. | CH₃ | 3-Chlor-pyridin-4-yl |
| A-361. | CH₃ | 3-Brom-pyridin-4-yl |
| A-362. | CH₃ | 3-Methyl-pyridin-4-yl |
| A-363. | CH₃ | 3,5-Dichlor-pyridin-4-yl |
| A-364. | CH₃ | 3,5-Dibrom-pyridin-4-yl |
| A-365. | CH₃ | 3,5-Dimethyl-pyridin-4-yl |
| A-366. | CH₃ | 4-Pyrimidinyl |
| A-367. | CH₃ | 5-Chlorpyrimidin-4-yl |
| A-368. | CH₃ | 5-Fluorpyrimidin-4-yl |
| A-369. | CH₃ | 5-Fluor-6-chlorpyrimidin-4-yl |
| A-370. | CH₃ | 2-Methyl-6-trifluormethyl-pyrimidin-4-yl |
| A-371. | CH₃ | 2,5-Dimethyl-6-trifluormethyl-pyrimidin-4-yl |
| A-372. | CH₃ | 5-Methyl-6-trifluormethyl-pyrimidin-4-yl |
| A-373. | CH₃ | 6-Trifluormethyl-pyrimidin-4-yl |
| A-374. | CH₃ | 2-Methyl-5-fluor-pyrimidin-4-yl |
| A-375. | CH₃ | 2-Methyl-5-chlor-pyrimidin-4-yl |
| A-376. | CH₃ | 5-Chlor-6-methyl-pyrimdin-4-yl |
| A-377. | CH₃ | 5-Chlor-6-ethyl-pyrimdin-4-yl |
| A-378. | CH₃ | 5-Chlor-6-isopropyl-pyrimdin-4-yl |
| A-379. | CH₃ | 5-Brom-6-methyl-pyrimidin-4-yl |
| A-380. | CH₃ | 5-Fluor-6-methyl-pyrimidin-4-yl |
| A-381. | CH₃ | 5-Fluor-6-fluormethyl-pyrimidin-4-yl |
| A-382. | CH₃ | 2,6-Dimethyl-5-chlor-pyrimdin-4-yl |
| A-383. | CH₃ | 5,6-Dimethyl-pyrimidin-4-yl |
| A-384. | CH₃ | 2,5-Dimethyl-pyrimidin-4-yl |
| A-385. | CH₃ | 2,5,6-Trimethyl-pyrimidin-4-yl |
| A-386. | CH₃ | 5-Methyl-6-methoxy-pyrimidin-4-yl |
| A-387. | CH₃ | 5-Pyrimidinyl |
| A-388. | CH₃ | 4-Methyl-pyrimidin-5-yl |
| A-389. | CH₃ | 4,6-Dimethyl-pyrimidin-5-yl |
| A-390. | CH₃ | 2,4,6-Trimethylpyrimidin-5-yl |
| A-391. | CH₃ | 4-Trifluormethyl-6-methyl-pyrimidin-5-yl |
| A-392. | CH₃ | 2-Pyrimidinyl |
| A-393. | CH₃ | 4,6-Dimethylpyrimidin-2-yl |
| A-394. | CH₃ | 4,5,6-Trimethylpyrimidin-2-yl |
| A-395. | CH₃ | 4,6-Ditrifluormethyl-pyrimidin-2-yl |
| A-396. | CH₃ | 4,6-Dimethyl-5-chlor-pyrimidin-2-yl |
| A-397. | CF₃ | Phenyl |
| A-398. | CF₃ | 2-Fluorphenyl |
| A-399. | CF₃ | 3-Fluorphenyl |
| A-400. | CF₃ | 4-Fluorphenyl |
| A-401. | CF₃ | 2-Chlorphenyl |
| A-402. | CF₃ | 3-Chlorphenyl |
| A-403. | CF₃ | 4-Chlorphenyl |
| A-404. | CF₃ | 3-Bromphenyl |
| A-405. | CF₃ | 4-Bromphenyl |
| A-406. | CF₃ | 2-Trifluormethylphenyl |
| A-407. | CF₃ | 3-Trifluormethylphenyl |
| A-408. | CF₃ | 4-Trifluormethylphenyl |
| A-409. | CF₃ | 2-(Methylthio)phenyl |
| A-410. | CF₃ | 3-(Methylthio)phenyl |
| A-411. | CF₃ | 4-(Methylthio)phenyl |
| A-412. | CF₃ | 2-Methoxyphenyl |
| A-413. | CF₃ | 3-Methoxyphenyl |
| A-414. | CF₃ | 4-Methoxyphenyl |
| A-415. | CF₃ | 4-Nitrophenyl |
| A-416. | CF₃ | 4-Cyanophenyl |
| A-417. | CF₃ | 4-tert.-Butylphenyl |
| A-418. | CF₃ | 4-Isopropylphenyl |
| A-419. | CF₃ | 3-Ethoxyphenyl |
| A-420. | CF₃ | 4-Ethoxyphenyl |
| A-421. | CF₃ | 4-n-Propoxyphenyl |
| A-422. | CF₃ | 3-Isopropoxyphenyl |
| A-423. | CF₃ | 4-Isopropoxyphenyl |
| A-424. | CF₃ | 4-n-Butoxyphenyl |
| A-425. | CF₃ | 4-tert.-Butoxyphenyl |
| A-426. | CF₃ | 4-(Methoxyiminomethyl)phenyl |
| A-427. | CF₃ | 4-(1-(Methoxyimino)ethyl)phenyl |
| A-428. | CF₃ | 2,3-Difluorphenyl |
| A-429. | CF₃ | 2,4-Difluorphenyl |
| A-430. | CF₃ | 2,5-Difluorphenyl |
| A-431. | CF₃ | 3,4-Difluorphenyl |
| A-432. | CF₃ | 3,5-Difluorphenyl |
| A-433. | CF₃ | 2,6-Difluorphenyl |
| A-434. | CF₃ | 2,4,6-Trifluorphenyl |
| A-435. | CF₃ | 2,4,5-Trifluorphenyl |
| A-436. | CF₃ | 2,3,4-Trifluorphenyl |
| A-437. | CF₃ | 2,3,5-Trifluorphenyl |
| A-438. | CF₃ | 3,4,5-Trifluorphenyl |
| A-439. | CF₃ | 2,3-Dichlorphenyl |
| A-440. | CF₃ | 2,5-Dichlorphenyl |
| A-441. | CF₃ | 3,5-Dichlorphenyl |
| A-442. | CF₃ | 2,6-Dichlorphenyl |
| A-443. | CF₃ | 2,3-Dimethylphenyl |
| A-444. | CF₃ | 2,4-Dimethylphenyl |
| A-445. | CF₃ | 2,5-Dimethylphenyl |
| A-446. | CF₃ | 2,4,5-Trimethylphenyl |
| A-447. | CF₃ | 2,3-Dimethoxyphenyl |
| A-448. | CF₃ | 2,4-Dimethoxyphenyl |
| A-449. | CF₃ | 3,4-Dimethoxyphenyl |
| A-450. | CF₃ | 2,4-Bis(trifluormethyl)phenyl |
| A-451. | CF₃ | 3,5-Bis(trifluormethyl)phenyl |
| A-452. | CF₃ | 2-Methyl-3-methoxyphenyl |
| A-453. | CF₃ | 2-Methyl-4-methoxyphenyl |
| A-454. | CF₃ | 2-Methyl-6-methoxyphenyl |
| A-455. | CF₃ | 3-Chlor-4-fluorphenyl |
| A-456. | CF₃ | 2-Chlor-4-fluorphenyl |
| A-457. | CF₃ | 2-Chlor-6-fluorphenyl |
| A-458. | CF₃ | 4-Chlor-2-fluorphenyl |
| A-459. | CF₃ | 5-Chlor-2-fluorphenyl |
| A-460. | CF₃ | 4-Fluor-3-methylphenyl |
| A-461. | CF₃ | 2-Fluor-4-methylphenyl |
| A-462. | CF₃ | 4-Fluor-2-methylphenyl |
| A-463. | CF₃ | 2-Fluor-3-methoxyphenyl |
| A-464. | CF₃ | 2-Fluor-4-methoxyphenyl |
| A-465. | CF₃ | 2-Fluor-6-methoxyphenyl |
| A-466. | CF₃ | 2-Fluor-4-trifluormethylphenyl |
| A-467. | CF₃ | 4-Chlor-3-methylphenyl |
| A-468. | CF₃ | 2-Chlor-4-methylphenyl |
| A-469. | CF₃ | 2-Chlor-6-methylphenyl |
| A-470. | CF₃ | 5-Chlor-2-methylphenyl |
| A-471. | CF₃ | 3-Chlor-2-methylphenyl |
| A-472. | CF₃ | 2-Chlor-4-methoxyphenyl |
| A-473. | CF₃ | 2-Chlor-6-methoxyphenyl |
| A-474. | CF₃ | 2-Chlor-4-trifluormethylphenyl |
| A-475. | CF₃ | 3-Fluor-4-methylphenyl |
| A-476. | CF₃ | 3-Fluor-4-isopropylphenyl |
| A-477. | CF₃ | 4-Fluor-3-methylphenyl |
| A-478. | CF₃ | 3-Fluor-4-methoxyphenyl |
| A-479. | CF₃ | 3-Fluor-4-ethoxyphenyl |
| A-480. | CF₃ | 3-Fluor-4-trifluormethylphenyl |
| A-481. | CF₃ | 3-Chlor-4-methylphenyl |
| A-482. | CF₃ | 3-Chlor-4-methoxyphenyl |
| A-483. | CF₃ | 3-Chlor-4-ethoxyphenyl |
| A-484. | CF₃ | 3-Chlor-4-trifluormethylphenyl |
| A-485. | CF₃ | 3-Methyl-4-methoxyphenyl |
| A-486. | CF₃ | 4-Chlor-2,5-difluorphenyl |
| A-487. | CF₃ | 4-tert.-Butyl-2-fluorphenyl |
| A-488. | CF₃ | 2-Fluor-4-isopropylphenyl |
| A-489. | CF₃ | 4-Ethoxy-2-fluorphenyl |
| A-490. | CF₃ | 4-Acetyl-2-fluorphenyl |
| A-491. | CF₃ | 2-Thienyl |
| A-492. | CF₃ | 5-Methylthiophen-2-yl |
| A-493. | CF₃ | 4-Methylthiophen-2-yl |
| A-494. | CF₃ | 5-Chlorthiophen-2-yl |
| A-495. | CF₃ | 3-Cyanothiophen-2-yl |
| A-496. | CF₃ | 5-Formylthiophen-2-yl |
| A-497. | CF₃ | 5-Acetylthiophen-2-yl |
| A-498. | CF₃ | 5-(Methoxyiminomethyl)thiophen-2-yl |
| A-499. | CF₃ | 5-(1-(Methoxyimino)ethyl)thiophen-2-yl |
| A-500. | CF₃ | 4-Bromthiophen-2-yl |
| A-501. | CF₃ | 3,5-Dichlorthiophen-2-yl |
| A-502. | CF₃ | 3-Thienyl |
| A-503. | CF₃ | 2-Methylthiophen-3-yl |
| A-504. | CF₃ | 2,5-Dichlorthiophen-3-yl |
| A-505. | CF₃ | 2,4,5-Trichlor-thiophen-3-yl |
| A-506. | CF₃ | 2-Furyl |
| A-507. | CF₃ | 5-Methylfuran-2-yl |
| A-508. | CF₃ | 5-Chlorfuran-2-yl |
| A-509. | CF₃ | 4-Methylfuran-2-yl |
| A-510. | CF₃ | 3-Cyanofuran-2-yl |
| A-511. | CF₃ | 5-Acetylfuran-2-yl |
| A-512. | CF₃ | 3-Furyl |
| A-513. | CF₃ | 2-Methylfuran-3-yl |
| A-514. | CF₃ | 2,5-Dimethylfuran-3-yl |
| A-515. | CF₃ | 2-Pyridyl |
| A-516. | CF₃ | 3-Fluor-pyridin-2-yl |
| A-517. | CF₃ | 3-Chlor-pyridin-2-yl |
| A-518. | CF₃ | 3-Brompyridin-2-yl |
| A-519. | CF₃ | 3-Trifluormethyl-pyridin-2-yl |
| A-520. | CF₃ | 3-Methyl-pyridin-2-yl |
| A-521. | CF₃ | 3-Ethyl-pyridin-2-yl |
| A-522. | CF₃ | 3,5-Difluor-pyridin-2-yl |
| A-523. | CF₃ | 3,5-Dichlor-pyridin-2-yl |
| A-524. | CF₃ | 3,5-Dibrom-pyridin-2-yl |
| A-525. | CF₃ | 3,5-Dimethyl-pyridin-2-yl |
| A-526. | CF₃ | 3-Fluor-5-trifluormethyl-pyridin-2-yl |
| A-527. | CF₃ | 3-Chlor-5-fluor-pyridin-2-yl |
| A-528. | CF₃ | 3-Chlor-5-methyl-pyridin-2-yl |
| A-529. | CF₃ | 3-Fluor-5-chlor-pyridin-2-yl |
| A-530. | CF₃ | 3-Fluor-5-methyl-pyridin-2-yl |
| A-531. | CF₃ | 3-Methyl-5-fluor-pyridin-2-yl |
| A-532. | CF₃ | 3-Methyl-5-chlor-pyridin-2-yl |
| A-533. | CF₃ | 5-Nitro-pyridin-2-yl |
| A-534. | CF₃ | 5-Cyano-pyridin-2-yl |
| A-535. | CF₃ | 5-Methoxycarbonyl-pyridin-2-yl |
| A-536. | CF₃ | 5-Trifluormethyl-pyridin-2-yl |
| A-537. | CF₃ | 5-Methyl-pyridin-2-yl |
| A-538. | CF₃ | 4-Methyl-pyridin-2-yl |
| A-539. | CF₃ | 6-Methyl-pyridin-2-yl |
| A-540. | CF₃ | 3-Pyridyl |
| A-541. | CF₃ | 2-Chlor-pyridin-3-yl |
| A-542. | CF₃ | 2-Brom-pyridin-3-yl |
| A-543. | CF₃ | 2-Methyl-pyridin-3-yl |
| A-544. | CF₃ | 2,4-Dichlor-pyridin-3-yl |
| A-545. | CF₃ | 2,4-Dibrom-pyridin-3-yl |
| A-546. | CF₃ | 2,4-Difluorpyridin-3-yl |
| A-547. | CF₃ | 2-Fluor-4-chlorpyridin-3-yl |
| A-548. | CF₃ | 2-Chlor-4-fluor-pyridin-3-yl |
| A-549. | CF₃ | 2-Chlor-4-methyl-pyridin-3-yl |
| A-550. | CF₃ | 2-Methyl-4-fluor-pyridin-3-yl |
| A-551. | CF₃ | 2-Methyl-4-chior-pyridin-3-yl |
| A-552. | CF₃ | 2,4-Dimethyl-pyridin-3-yl |
| A-553. | CF₃ | 2,4,6-Trichlorpyridin-3-yl |
| A-554. | CF₃ | 2,4,6-Tribrompyridin-3-yl |
| A-555. | CF₃ | 2,4,6-Trimethyl-pyridin-3-yl |
| A-556. | CF₃ | 2,4-Dichlor-6-methylpyridin-3-yl |
| A-557. | CF₃ | 4-Pyridyl |
| A-558. | CF₃ | 3-Chlor-pyridin-4-yl |
| A-559. | CF₃ | 3-Brom-pyridin-4-yl |
| A-560. | CF₃ | 3-Methyl-pyridin-4-yl |
| A-561. | CF₃ | 3,5-Dichlor-pyridin-4-yl |
| A-562. | CF₃ | 3,5-Dibrom-pyridin-4-yl |
| A-563. | CF₃ | 3,5-Dimethyl-pyridin-4-yl |
| A-564. | CF₃ | 4-Pyrimidinyl |
| A-565. | CF₃ | 5-Chlorpyrimidin-4-yl |
| A-566. | CF₃ | 5-Fluorpyrimidin-4-yl |
| A-567. | CF₃ | 5-Fluor-6-chlorpyrimidin-4-yl |
| A-568. | CF₃ | 2-Methyl-6-trifluormethyl-pyrimidin-4-yl |
| A-569. | CF₃ | 2,5-Dimethyl-6-trifluormethyl-pyrimidin-4-yl |
| A-570. | CF₃ | 5-Methyl-6-trifluormethyl-pyrimidin-4-yl |
| A-571. | CF₃ | 6-Trifluormethyl-pyrimidin-4-yl |
| A-572. | CF₃ | 2-Methyl-5-fluor-pyrimidin-4-yl |
| A-573. | CF₃ | 2-Methyl-5-chlor-pyrimidin-4-yl |
| A-574. | CF₃ | 5-Chlor-6-methyl-pyrimdin-4-yl |
| A-575. | CF₃ | 5-Chlor-6-ethyl-pyrimdin-4-yl |
| A-576. | CF₃ | 5-Chlor-6-isopropyl-pyrimdin-4-yl |
| A-577. | CF₃ | 5-Brom-6-methyl-pyrimidin-4-yl |
| A-578. | CF₃ | 5-Fluor-6-methyl-pyrimidin-4-yl |
| A-579. | CF₃ | 5-Fluor-6-fluormethyl-pyrimidin-4-yl |
| A-580. | CF₃ | 2,6-Dimethyl-5-chlor-pyrimdin-4-yl |
| A-581. | CF₃ | 5,6-Dimethyl-pyrimidin-4-yl |
| A-582. | CF₃ | 2,5-Dimethyl-pyrimidin-4-yl |
| A-583. | CF₃ | 2,5,6-Trimethyl-pyrimidin-4-yl |
| A-584. | CF₃ | 5-Methyl-6-methoxy-pyrimidin-4-yl |
| A-585. | CF₃ | 5-Pyrimidinyl |
| A-586. | CF₃ | 4-Methyl-pyrimidin-5-yl |
| A-587. | CF₃ | 4,6-Dimethyl-pyrimidin-5-yl |
| A-588. | CF₃ | 2,4,6-Trimethylpyrimidin-5-yl |
| A-589. | CF₃ | 4-Trifluormethyl-6-methyl-pyrimidin-5-yl |
| A-590. | CF₃ | 2-Pyrimidinyl |
| A-591. | CF₃ | 4,6-Dimethylpyrimidin-2-yl |
| A-592. | CF₃ | 4,5,6-Trimethylpyrimidin-2-yl |
| A-593. | CF₃ | 4,6-Ditrifluormethyl-pyrimidin-2-yl |
| A-594. | CF₃ | 4,6-Dimethyl-5-chlor-pyrimidin-2-yl |
| A-595. | OCH₃ | Phenyl |
| A-596. | OCH₃ | 2-Fluorphenyl |
| A-597. | OCH₃ | 3-Fluorphenyl |
| A-598. | OCH₃ | 4-Fluorphenyl |
| A-599. | OCH₃ | 2-Chlorphenyl |
| A-600. | OCH₃ | 3-Chlorphenyl |
| A-601. | OCH₃ | 4-Chlorphenyl |
| A-602. | OCH₃ | 3-Bromphenyl |
| A-603. | OCH₃ | 4-Bromphenyl |
| A-604. | OCH₃ | 2-Trifluormethylphenyl |
| A-605. | OCH₃ | 3-Trifluormethylphenyl |
| A-606. | OCH₃ | 4-Trifluormethylphenyl |
| A-607. | OCH₃ | 2-(Methylthio)phenyl |
| A-608. | OCH₃ | 3-(Methylthio)phenyl |
| A-609. | OCH₃ | 4-(Methylthio)phenyl |
| A-610. | OCH₃ | 2-Methoxyphenyl |
| A-611. | OCH₃ | 3-Methoxyphenyl |
| A-612. | OCH₃ | 4-Methoxyphenyl |
| A-613. | OCH₃ | 4-Nitrophenyl |
| A-614. | OCH₃ | 4-Cyanophenyl |
| A-615. | OCH₃ | 4-tert.-Butylphenyl |
| A-616. | OCH₃ | 4-Isopropylphenyl |
| A-617. | OCH₃ | 3-Ethoxyphenyl |
| A-618. | OCH₃ | 4-Ethoxyphenyl |
| A-619. | OCH₃ | 4-n-Propoxyphenyl |
| A-620. | OCH₃ | 3-Isopropoxyphenyl |
| A-621. | OCH₃ | 4-Isopropoxyphenyl |
| A-622. | OCH₃ | 4-n-Butoxyphenyl |
| A-623. | OCH₃ | 4-tert.-Butoxyphenyl |
| A-624. | OCH₃ | 4-(Methoxyiminomethyl)phenyl |
| A-625. | OCH₃ | 4-(1-(Methoxyimino)ethyl)phenyl |
| A-626. | OCH₃ | 2,3-Difluorphenyl |
| A-627. | OCH₃ | 2,4-Difluorphenyl |
| A-628. | OCH₃ | 2,5-Difluorphenyl |
| A-629. | OCH₃ | 3,4-Difluorphenyl |
| A-630. | OCH₃ | 3,5-Difluorphenyl |
| A-631. | OCH₃ | 2,6-Difluorphenyl |
| A-632. | OCH₃ | 2,4,6-Trifluorphenyl |
| A-633. | OCH₃ | 2,4,5-Trifluorphenyl |
| A-634. | OCH₃ | 2,3,4-Trifluorphenyl |
| A-635. | OCH₃ | 2,3,5-Trifluorphenyl |
| A-636. | OCH₃ | 3,4,5-Trifluorphenyl |
| A-637. | OCH₃ | 2,3-Dichlorphenyl |
| A-638. | OCH₃ | 2,5-Dichlorphenyl |
| A-639. | OCH₃ | 3,5-Dichlorphenyl |
| A-640. | OCH₃ | 2,6-Dichlorphenyl |
| A-641. | OCH₃ | 2,3-Dimethylphenyl |
| A-642. | OCH₃ | 2,4-Dimethylphenyl |
| A-643. | OCH₃ | 2,5-Dimethylphenyl |
| A-644. | OCH₃ | 2,4,5-Trimethylphenyl |
| A-645. | OCH₃ | 2,3-Dimethoxyphenyl |
| A-646. | OCH₃ | 2,4-Dimethoxyphenyl |
| A-647. | OCH₃ | 3,4-Dimethoxyphenyl |
| A-648. | OCH₃ | 2,4-Bis(trifluormethyl)phenyl |
| A-649. | OCH₃ | 3,5-Bis(trifluormethyl)phenyl |
| A-650. | OCH₃ | 2-Methyl-3-methoxyphenyl |
| A-651. | OCH₃ | 2-Methyl-4-methoxyphenyl |
| A-652. | OCH₃ | 2-Methyl-6-methoxyphenyl |
| A-653. | OCH₃ | 3-Chlor-4-fluorphenyl |
| A-654. | OCH₃ | 2-Chlor-4-fluorphenyl |
| A-655. | OCH₃ | 2-Chlor-6-fluorphenyl |
| A-656. | OCH₃ | 4-Chlor-2-fluorphenyl |
| A-657. | OCH₃ | 5-Chlor-2-fluorphenyl |
| A-658. | OCH₃ | 4-Fluor-3-methylphenyl |
| A-659. | OCH₃ | 2-Fluor-4-methylphenyl |
| A-660. | OCH₃ | 4-Fluor-2-methylphenyl |
| A-661. | OCH₃ | 2-Fluor-3-methoxyphenyl |
| A-662. | OCH₃ | 2-Fluor-4-methoxyphenyl |
| A-663. | OCH₃ | 2-Fluor-6-methoxyphenyl |
| A-664. | OCH₃ | 2-Fluor-4-trifluormethylphenyl |
| A-665. | OCH₃ | 4-Chlor-3-methylphenyl |
| A-666. | OCH₃ | 2-Chlor-4-methylphenyl |
| A-667. | OCH₃ | 2-Chlor-6-methylphenyl |
| A-668. | OCH₃ | 5-Chlor-2-methylphenyl |
| A-669. | OCH₃ | 3-Chlor-2-methylphenyl |
| A-670. | OCH₃ | 2-Chlor-4-methoxyphenyl |
| A-671. | OCH₃ | 2-Chlor-6-methoxyphenyl |
| A-672. | OCH₃ | 2-Chlor-4-trifluormethylphenyl |
| A-673. | OCH₃ | 3-Fluor-4-methylphenyl |
| A-674. | OCH₃ | 3-Fluor-4-isopropylphenyl |
| A-675. | OCH₃ | 4-Fluor-3-methylphenyl |
| A-676. | OCH₃ | 3-Fluor-4-methoxyphenyl |
| A-677. | OCH₃ | 3-Fluor-4-ethoxyphenyl |
| A-678. | OCH₃ | 3-Fluor-4-trifluormethylphenyl |
| A-679. | OCH₃ | 3-Chlor-4-methylphenyl |
| A-680. | OCH₃ | 3-Chlor-4-methoxyphenyl |
| A-681. | OCH₃ | 3-Chlor-4-ethoxyphenyl |
| A-682. | OCH₃ | 3-Chlor-4-trifluormethylphenyl |
| A-683. | OCH₃ | 3-Methyl-4-methoxyphenyl |
| A-684. | OCH₃ | 4-Chlor-2,5-difluorphenyl |
| A-685. | OCH₃ | 4-tert.-Butyl-2-fluorphenyl |
| A-686. | OCH₃ | 2-Fluor-4-isopropylphenyl |
| A-687. | OCH₃ | 4-Ethoxy-2-fluorphenyl |
| A-688. | OCH₃ | 4-Acetyl-2-fluorphenyl |
| A-689. | OCH₃ | 2-Thienyl |
| A-690. | OCH₃ | 5-Methylthiophen-2-yl |
| A-691. | OCH₃ | 4-Methylthiophen-2-yl |
| A-692. | OCH₃ | 5-Chlorthiophen-2-yl |
| A-693. | OCH₃ | 3-Cyanothiophen-2-yl |
| A-694. | OCH₃ | 5-Formylthiophen-2-yl |
| A-695. | OCH₃ | 5-Acetylthiophen-2-yl |
| A-696. | OCH₃ | 5-(Methoxyiminomethyl)thiophen-2-yl |
| A-697. | OCH₃ | 5-(1-(Methoxyimino)ethyl)thiophen-2-yl |
| A-698. | OCH₃ | 4-Bromthiophen-2-yl |
| A-699. | OCH₃ | 3,5-Dichlorthiophen-2-yl |
| A-700. | OCH₃ | 3-Thienyl |
| A-701. | OCH₃ | 2-Methylthiophen-3-yl |
| A-702. | OCH₃ | 2,5-Dichlorthiophen-3-yl |
| A-703. | OCH₃ | 2,4,5-Trichlor-thiophen-3-yl |
| A-704. | OCH₃ | 2-Furyl |
| A-705. | OCH₃ | 5-Methylfuran-2-yl |
| A-706. | OCH₃ | 5-Chlorfuran-2-yl |
| A-707. | OCH₃ | 4-Methylfuran-2-yl |
| A-708. | OCH₃ | 3-Cyanofuran-2-yl |
| A-709. | OCH₃ | 5-Acetylfuran-2-yl |
| A-710. | OCH₃ | 3-Furyl |
| A-711. | OCH₃ | 2-Methylfuran-3-yl |
| A-712. | OCH₃ | 2,5-Dimethylfuran-3-yl |
| A-713. | OCH₃ | 2-Pyridyl |
| A-714. | OCH₃ | 3-Fluor-pyridin-2-yl |
| A-715. | OCH₃ | 3-Chlor-pyridin-2-yl |
| A-716. | OCH₃ | 3-Brompyridin-2-yl |
| A-717. | OCH₃ | 3-Trifluormethyl-pyridin-2-yl |
| A-718. | OCH₃ | 3-Methyl-pyridin-2-yl |
| A-719. | OCH₃ | 3-Ethyl-pyridin-2-yl |
| A-720. | OCH₃ | 3,5-Difluor-pyridin-2-yl |
| A-721. | OCH₃ | 3,5-Dichlor-pyridin-2-yl |
| A-722. | OCH₃ | 3,5-Dibrom-pyridin-2-yl |
| A-723. | OCH₃ | 3,5-Dimethyl-pyridin-2-yl |
| A-724. | OCH₃ | 3-Fluor-5-trifluormethyl-pyridin-2-yl |
| A-725. | OCH₃ | 3-Chlor-5-fluor-pyridin-2-yl |
| A-726. | OCH₃ | 3-Chlor-5-methyl-pyridin-2-yl |
| A-727. | OCH₃ | 3-Fluor-5-chlor-pyridin-2-yl |
| A-728. | OCH₃ | 3-Fluor-5-methyl-pyridin-2-yl |
| A-729. | OCH₃ | 3-Methyl-5-fluor-pyridin-2-yl |
| A-730. | OCH₃ | 3-Methyl-5-chlor-pyridin-2-yl |
| A-731. | OCH₃ | 5-Nitro-pyridin-2-yl |
| A-732. | OCH₃ | 5-Cyano-pyridin-2-yl |
| A-733. | OCH₃ | 5-Methoxycarbonyl-pyridin-2-yl |
| A-734. | OCH₃ | 5-Trifluormethyl-pyridin-2-yl |
| A-735. | OCH₃ | 5-Methyl-pyridin-2-yl |
| A-736. | OCH₃ | 4-Methyl-pyridin-2-yl |
| A-737. | OCH₃ | 6-Methyl-pyridin-2-yl |
| A-738. | OCH₃ | 3-Pyridyl |
| A-739. | OCH₃ | 2-Chlor-pyridin-3-yl |
| A-740. | OCH₃ | 2-Brom-pyridin-3-yl |
| A-741. | OCH₃ | 2-Methyl-pyridin-3-yl |
| A-742. | OCH₃ | 2,4-Dichlor-pyridin-3-yl |
| A-743. | OCH₃ | 2,4-Dibrom-pyridin-3-yl |
| A-744. | OCH₃ | 2,4-Difluorpyridin-3-yl |
| A-745. | OCH₃ | 2-Fluor-4-chlorpyridin-3-yl |
| A-746. | OCH₃ | 2-Chlor-4-fluor-pyridin-3-yl |
| A-747. | OCH₃ | 2-Chlor-4-methyl-pyridin-3-yl |
| A-748. | OCH₃ | 2-Methyl-4-fluor-pyridin-3-yl |
| A-749. | OCH₃ | 2-Methyl-4-chlor-pyridin-3-yl |
| A-750. | OCH₃ | 2,4-Dimethyl-pyridin-3-yl |
| A-751. | OCH₃ | 2,4,6-Trichlorpyridin-3-yl |
| A-752. | OCH₃ | 2,4,6-Tribrompyridin-3-yl |
| A-753. | OCH₃ | 2,4,6-Trimethyl-pyridin-3-yl |
| A-754. | OCH₃ | 2,4-Dichlor-6-methylpyridin-3-yl |
| A-755. | OCH₃ | 4-Pyridyl |
| A-756. | OCH₃ | 3-Chlor-pyridin-4-yl |
| A-757. | OCH₃ | 3-Brom-pyridin-4-yl |
| A-758. | OCH₃ | 3-Methyl-pyridin-4-yl |
| A-759. | OCH₃ | 3,5-Dichlor-pyridin-4-yl |
| A-760. | OCH₃ | 3,5-Dibrom-pyridin-4-yl |
| A-761. | OCH₃ | 3,5-Dimethyl-pyridin-4-yl |
| A-762. | OCH₃ | 4-Pyrimidinyl |
| A-763. | OCH₃ | 5-Chlorpyrimidin-4-yl |
| A-764. | OCH₃ | 5-Fluorpyrimidin-4-yl |
| A-765. | OCH₃ | 5-Fluor-6-chlorpyrimidin-4-yl |
| A-766. | OCH₃ | 2-Methyl-6-trifluormethyl-pyrimidin-4-yl |
| A-767. | OCH₃ | 2,5-Dimethyl-6-trifluormethyl-pyrimidin-4-yl |
| A-768. | OCH₃ | 5-Methyl-6-trifluormethyl-pyrimidin-4-yl |
| A-769. | OCH₃ | 6-Trifluormethyl-pyrimidin-4-yl |
| A-770. | OCH₃ | 2-Methyl-5-fluor-pyrimidin-4-yl |
| A-771. | OCH₃ | 2-Methyl-5-chlor-pyrimidin-4-yl |
| A-772. | OCH₃ | 5-Chlor-6-methyl-pyrimdin-4-yl |
| A-773. | OCH₃ | 5-Chlor-6-ethyl-pyrimdin-4-yl |
| A-774. | OCH₃ | 5-Chlor-6-isopropyl-pyrimdin-4-yl |
| A-775. | OCH₃ | 5-Brom-6-methyl-pyrimidin-4-yl |
| A-776. | OCH₃ | 5-Fluor-6-methyl-pyrimidin-4-yl |
| A₋777. | OCH₃ | 5-Fluor-6-fluormethyl-pyrimidin-4-yl |
| A-778. | OCH₃ | 2,6-Dimethyl-5-chlor-pyrimdin-4-yl |
| A-779. | OCH₃ | 5,6-Dimethyl-pyrimidin-4-yl |
| A-780. | OCH₃ | 2,5-Dimethyl-pyrimidin-4-yl |
| A-781. | OCH₃ | 2,5,6-Trimethyl-pyrimidin-4-yl |
| A-782. | OCH₃ | 5-Methyl-6-methoxy-pyrimidin-4-yl |
| A-783. | OCH₃ | 5-Pyrimidinyl |
| A-784. | OCH₃ | 4-Methyl-pyrimidin-5-yl |
| A-785. | OCH₃ | 4,6-Dimethyl-pyrimidin-5-yl |
| A-786. | OCH₃ | 2,4,6-Trimethylpyrimidin-5-yl |
| A-787. | OCH₃ | 4-Trifluormethyl-6-methyl-pyrimidin-5-yl |
| A-788. | OCH₃ | 2-Pyrimidinyl |
| A-789. | OCH₃ | 4,6-Dimethylpyrimidin-2-yl |
| A-790. | OCH₃ | 4,5,6-Trimethylpyrimidin-2-yl |
| A-791. | OCH₃ | 4,6-Ditrifluormethyl-pyrimidin-2-yl |
| A-792. | OCH₃ | 4,6-Dimethyl-5-chlor-pyrimidin-2-yl |
| A-793. | Cl | Phenyl |
| A-794. | Cl | 2-Fluorphenyl |
| A-795. | Cl | 3-Fluorphenyl |
| A-796. | Cl | 4-Fluorphenyl |
| A-797. | Cl | 2-Chlorphenyl |
| A-798. | Cl | 3-Chlorphenyl |
| A-799. | Cl | 4-Chlorphenyl |
| A-800. | Cl | 3-Bromphenyl |
| A-801. | Cl | 4-Bromphenyl |
| A-802. | Cl | 2-Trifluormethylphenyl |
| A-803. | Cl | 3-Trifluormethylphenyl |
| A-804. | Cl | 4-Trifluormethylphenyl |
| A-805. | Cl | 2-(Methylthio)phenyl |
| A-806. | Cl | 3-(Methylthio)phenyl |
| A-807. | Cl | 4-(Methylthio)phenyl |
| A-808. | Cl | 2-Methoxyphenyl |
| A-809. | Cl | 3-Methoxyphenyl |
| A-810. | Cl | 4-Methoxyphenyl |
| A-811. | Cl | 4-Nitrophenyl |
| A-812. | Cl | 4-Cyanophenyl |
| A-813. | Cl | 4-tert.-Butylphenyl |
| A-814. | Cl | 4-Isopropylphenyl |
| A-815. | Cl | 3-Ethoxyphenyl |
| A-816. | Cl | 4-Ethoxyphenyl |
| A-817. | Cl | 4-n-Propoxyphenyl |
| A-818. | Cl | 3-Isopropoxyphenyl |
| A-819. | Cl | 4-Isopropoxyphenyl |
| A-820. | Cl | 4-n-Butoxyphenyl |
| A-821. | Cl | 4-tert.-Butoxyphenyl |
| A-822. | Cl | 4-(Methoxyiminomethyl)phenyl |
| A-823. | Cl | 4-(1-(Methoxyimino)ethyl)phenyl |
| A-824. | Cl | 2,3-Difluorphenyl |
| A-825. | Cl | 2,4-Difluorphenyl |
| A-826. | Cl | 2,5-Difluorphenyl |
| A-827. | Cl | 3,4-Difluorphenyl |
| A-828. | Cl | 3,5-Difluorphenyl |
| A-829. | Cl | 2,6-Difluorphenyl |
| A-830. | Cl | 2,4,6-Trifluorphenyl |
| A-831. | Cl | 2,4,5-Trifluorphenyl |
| A-832. | Cl | 2,3,4-Trifluorphenyl |
| A-833. | Cl | 2,3,5-Trifluorphenyl |
| A-834. | Cl | 3,4,5-Trifluorphenyl |
| A-835. | Cl | 2,3-Dichlorphenyl |
| A-836. | Cl | 2,5-Dichlorphenyl |
| A-837. | Cl | 3,5-Dichlorphenyl |
| A-838. | Cl | 2,6-Dichlorphenyl |
| A-839. | Cl | 2,3-Dimethylphenyl |
| A-840. | Cl | 2,4-Dimethylphenyl |
| A-841. | Cl | 2,5-Dimethylphenyl |
| A-842. | Cl | 2,4,5-Trimethylphenyl |
| A-843. | Cl | 2,3-Dimethoxyphenyl |
| A-844. | Cl | 2,4-Dimethoxyphenyl |
| A-845. | Cl | 3,4-Dimethoxyphenyl |
| A-846. | Cl | 2,4-Bis(trifluormethyl)phenyl |
| A-847. | Cl | 3,5-Bis(trifluormethyl)phenyl |
| A-848. | Cl | 2-Methyl-3-methoxyphenyl |
| A-849. | Cl | 2-Methyl-4-methoxyphenyl |
| A-850. | Cl | 2-Methyl-6-methoxyphenyl |
| A-851. | Cl | 3-Chlor-4-fluorphenyl |
| A-852. | Cl | 2-Chlor-4-fluorphenyl |
| A-853. | Cl | 2-Chlor-6-fluorphenyl |
| A-854. | Cl | 4-Chlor-2-fluorphenyl |
| A-855. | Cl | 5-Chlor-2-fluorphenyl |
| A-856. | Cl | 4-Fluor-3-methylphenyl |
| A-857. | Cl | 2-Fluor-4-methylphenyl |
| A-858. | Cl | 4-Fluor-2-methylphenyl |
| A-859. | Cl | 2-Fluor-3-methoxyphenyl |
| A-860. | Cl | 2-Fluor-4-methoxyphenyl |
| A-861. | Cl | 2-Fluor-6-methoxyphenyl |
| A-862. | Cl | 2-Fluor-4-trifluormethylphenyl |
| A-863. | Cl | 4-Chlor-3-methylphenyl |
| A-864. | Cl | 2-Chlor-4-methylphenyl |
| A-865. | Cl | 2-Chlor-6-methylphenyl |
| A-866. | Cl | 5-Chlor-2-methylphenyl |
| A-867. | Cl | 3-Chlor-2-methylphenyl |
| A-868. | Cl | 2-Chlor-4-methoxyphenyl |
| A-869. | Cl | 2-Chlor-6-methoxyphenyl |
| A-870. | Cl | 2-Chlor-4-trifluormethylphenyl |
| A-871. | Cl | 3-Fluor-4-methylphenyl |
| A-872. | Cl | 3-Fluor-4-isopropylphenyl |
| A-873. | Cl | 4-Fluor-3-methylphenyl |
| A-874. | Cl | 3-Fluor-4-methoxyphenyl |
| A-875. | Cl | 3-Fluor-4-ethoxyphenyl |
| A-876. | Cl | 3-Fluor-4-trifluormethylphenyl |
| A-877. | Cl | 3-Chlor-4-methylphenyl |
| A-878. | Cl | 3-Chlor-4-methoxyphenyl |
| A-879. | Cl | 3-Chlor-4-ethoxyphenyl |
| A-880. | Cl | 3-Chlor-4-trifluormethylphenyl |
| A-881. | Cl | 3-Methyl-4-methoxyphenyl |
| A-882. | Cl | 4-Chlor-2,5-difluorphenyl |
| A-883. | Cl | 4-tert.-Butyl-2-fluorphenyl |
| A-884. | Cl | 2-Fluor-4-isopropylphenyl |
| A-885. | Cl | 4-Ethoxy-2-fluorphenyl |
| A-886. | Cl | 4-Acetyl-2-fluorphenyl |
| A-887. | Cl | 2-Thienyl |
| A-888. | Cl | 5-Methylthiophen-2-yl |
| A-889. | Cl | 4-Methylthiophen-2-yl |
| A-890. | Cl | 5-Chlorthiophen-2-yl |
| A-891. | Cl | 3-Cyanothiophen-2-yl |
| A-892. | Cl | 5-Formylthiophen-2-yl |
| A-893. | Cl | 5-Acetylthiophen-2-yl |
| A-894. | Cl | 5-(Methoxyiminomethyl)thiophen-2-yl |
| A-895. | Cl | 5-(1-(Methoxyimino)ethyl)thiophen-2-yl |
| A-896. | Cl | 4-Bromthiophen-2-yl |
| A-897. | Cl | 3,5-Dichlorthiophen-2-yl |
| A-898. | Cl | 3-Thienyl |
| A-899. | Cl | 2-Methylthiophen-3-yl |
| A-900. | Cl | 2,5-Dichlorthiophen-3-yl |
| A-901. | Cl | 2,4,5-Trichlor-thiophen-3-yl |
| A-902. | Cl | 2-Furyl |
| A-903. | Cl | 5-Methylfuran-2-yl |
| A-904. | Cl | 5-Chlorfuran-2-yl |
| A-905. | Cl | 4-Methylfuran-2-yl |
| A-906. | Cl | 3-Cyanofuran-2-yl |
| A-907. | Cl | 5-Acetylfuran-2-yl |
| A-908. | Cl | 3-Furyl |
| A-909. | Cl | 2-Methylfuran-3-yl |
| A-910. | Cl | 2,5-Dimethylfuran-3-yl |
| A-911. | Cl | 2-Pyridyl |
| A-912. | Cl | 3-Fluor-pyridin-2-yl |
| A-913. | Cl | 3-Chlor-pyridin-2-yl |
| A-914. | Cl | 3-Brompyridin-2-yl |
| A-915. | Cl | 3-Trifluormethyl-pyridin-2-yl |
| A-916. | Cl | 3-Methyl-pyridin-2-yl |
| A-917. | Cl | 3-Ethyl-pyridin-2-yl |
| A-918. | Cl | 3,5-Difluor-pyridin-2-yl |
| A-919. | Cl | 3,5-Dichlor-pyridin-2-yl |
| A-920. | Cl | 3, 5-Dibrom-pyrid in-2-yl |
| A-921. | Cl | 3,5-Dimethyl-pyridin-2-yl |
| A-922. | Cl | 3-Fluor-5-trifluormethyl-pyridin-2-yl |
| A-923. | Cl | 3-Chlor-5-fluor-pyridin-2-yl |
| A-924. | Cl | 3-Chlor-5-methyl-pyridin-2-yl |
| A-925. | Cl | 3-Fluor-5-chlor-pyridin-2-yl |
| A-926. | Cl | 3-Fluor-5-methyl-pyridin-2-yl |
| A-927. | Cl | 3-Methyl-5-fluor-pyridin-2-yl |
| A-928. | Cl | 3-Methyl-5-chlor-pyridin-2-yl |
| A-929. | Cl | 5-Nitro-pyridin-2-yl |
| A-930. | Cl | 5-Cyano-pyridin-2-yl |
| A-931. | Cl | 5-Methoxycarbonyl-pyridin-2-yl |
| A-932. | Cl | 5-Trifluormethyl-pyridin-2-yl |
| A-933. | Cl | 5-Methyl-pyridin-2-yl |
| A-934. | Cl | 4-Methyl-pyridin-2-yl |
| A-935. | Cl | 6-Methyl-pyridin-2-yl |
| A-936. | Cl | 3-Pyridyl |
| A-937. | Cl | 2-Chlor-pyridin-3-yl |
| A-938. | Cl | 2-Brom-pyridin-3-yl |
| A-939. | Cl | 2-Methyl-pyridin-3-yl |
| A-940. | Cl | 2,4-Dichlor-pyridin-3-yl |
| A-941. | Cl | 2,4-Dibrom-pyridin-3-yl |
| A-942. | Cl | 2,4-Difluorpyridin-3-yl |
| A-943. | Cl | 2-Fluor-4-chlorpyridin-3-yl |
| A-944. | Cl | 2-Chlor-4-fluor-pyridin-3-yl |
| A-945. | Cl | 2-Chlor-4-methyl-pyridin-3-yl |
| A-946. | Cl | 2-Methyl-4-fluor-pyridin-3-yl |
| A-947. | Cl | 2-Methyl-4-chlor-pyridin-3-yl |
| A-948. | Cl | 2,4-Dimethyl-pyridin-3-yl |
| A-949. | Cl | 2,4,6-Trichlorpyridin-3-yl |
| A-950. | Cl | 2,4,6-Tribrompyridin-3-yl |
| A-951. | Cl | 2,4,6-Trimethyl-pyridin-3-yl |
| A-952. | Cl | 2,4-Dichlor-6-methylpyridin-3-yl |
| A-953. | Cl | 4-Pyridyl |
| A-954. | Cl | 3-Chlor-pyridin-4-yl |
| A-955. | Cl | 3-Brom-pyridin-4-yl |
| A-956. | Cl | 3-Methyl-pyridin-4-yl |
| A-957. | Cl | 3,5-Dichlor-pyridin-4-yl |
| A-958. | Cl | 3,5-Dibrom-pyridin-4-yl |
| A-959. | Cl | 3,5-Dimethyl-pyridin-4-yl |
| A-960. | Cl | 4-Pyrimidinyl |
| A-961. | Cl | 5-Chlorpyrimidin-4-yl |
| A-962. | Cl | 5-Fluorpyrimidin-4-yl |
| A-963. | Cl | 5-Fluor-6-chlorpyrimidin-4-yl |
| A-964. | Cl | 2-Methyl-6-trifluormethyl-pyrimidin-4-yl |
| A-965. | Cl | 2,5-Dimethyl-6-trifluormethyl-pyrimidin-4-yl |
| A-966. | Cl | 5-Methyl-6-trifluormethyl-pyrimidin-4-yl |
| A-967. | Cl | 6-Trifluormethyl-pyrimidin-4-yl |
| A-968. | Cl | 2-Methyl-5-fluor-pyrimidin-4-yl |
| A-969. | Cl | 2-Methyl-5-chlor-pyrimidin-4-yl |
| A-970. | Cl | 5-Chlor-6-methyl-pyrimdin-4-yl |
| A-971. | Cl | 5-Chlor-6-ethyl-pyrimdin-4-yl |
| A-972. | Cl | 5-Chlor-6-isopropyl-pyrimdin-4-yl |
| A-973. | Cl | 5-Brom-6-methyl-pyrimidin-4-yl |
| A-974. | Cl | 5-Fluor-6-methyl-pyrimidin-4-yl |
| A-975. | Cl | 5-Fluor-6-fluormethyl-pyrimidin-4-yl |
| A-976. | Cl | 2,6-Dimethyl-5-chlor-pyrimdin-4-yl |
| A-977. | Cl | 5,6-Dimethyl-pyrimidin-4-yl |
| A-978. | Cl | 2,5-Dimethyl-pyrimidin-4-yl |
| A-979. | Cl | 2,5,6-Trimethyl-pyrimidin-4-yl |
| A-980. | Cl | 5-Methyl-6-methoxy-pyrimidin-4-yl |
| A-981. | Cl | 5-Pyrimidinyl |
| A-982. | Cl | 4-Methyl-pyrimidin-5-yl |
| A-983. | Cl | 4,6-Dimethyl-pyrimidin-5-yl |
| A-984. | Cl | 2,4,6-Trimethylpyrimidin-5-yl |
| A-985. | Cl | 4-Trifluormethyl-6-methyl-pyrimidin-5-yl |
| A-986. | Cl | 2-Pyrimidinyl |
| A-987. | Cl | 4,6-Dimethylpyrimidin-2-yl |
| A-988. | Cl | 4,5,6-Trimethylpyrimidin-2-yl |
| A-989. | Cl | 4,6-Ditrifluormethyl-pyrimidin-2-yl |
| A-990. | Cl | 4,6-Dimethyl-5-chlor-pyrimidin-2-yl |

Die erfindungsgemäßen Verbindungen der allgemeinen Formel I können in Analogie zu dem eingangs zitierten Stand der Technik durch Standardmethoden der organischen Synthese hergestellt werden.

Verbindungen der Formel I, worin R⁵ für gegebenenfalls substituiertes Phenyl oder Heteroaryl steht, können beispielsweise nach dem in Schema 1 dargestellten Verfahren hergestellt werden:

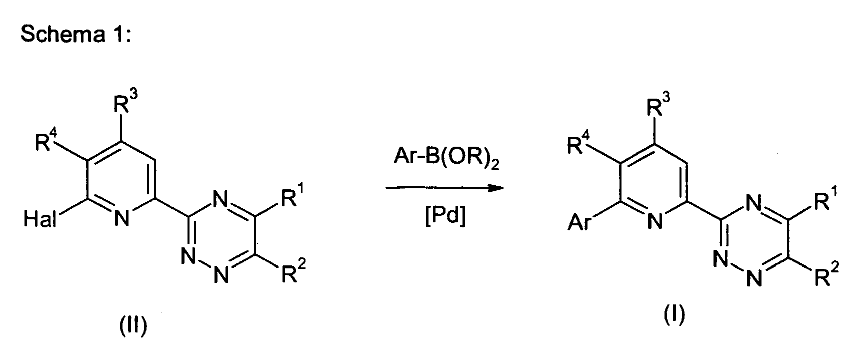

In Schema 1 haben R¹, R², R³ und R⁴ die zuvor genannten Bedeutungen. Ar steht für gegebenenfalls substituiertes Phenyl oder für gegebenenfalls substituiertes 5- oder 6-gliedriges Hetaryl. R steht für H oder C₁-C₄-Alkyl oder bildet mit weiteren Molekülen Ar-B(OR)₂ ein Phenylboronsäure-Anhydrid. Hal steht für Chlor, Brom oder Iod.

Gemäß Schema 1 wird das 2-(6-Halopyridin-2-yl)triazin der Formel II mit einem (Het)arylboronsäurederivat der allgemeinen Formel Ar-B(OR)₂ unter den Bedingungen einer Suzuki-Kupplung, d.h. in Gegenwart eines Palladiumkatalysators unter an sich bekannten Reaktionsbedingungen umgesetzt, wie sie z.B. aus Acc. Chem. Res. 15, S. 178-184 (1982), Chem. Rev. 95, S. 2457-2483 (1995), und der darin zitierten Literatur, sowie aus J. Org. Chem. 68, S. 9412 (2003) bekannt sind. Geeignete Katalysatoren sind insbesondere Tetrakis(triphenylphosphin)palladium(0), Bis(triphenylphosphin)palladium(II)-chlorid, Bis(acetonitril)palladium(II)-chlorid, der [1,1'-Bis-(diphenylphosphino)ferrocen]palladium(II)-chlorid-Dichlormethan-Komplex, Bis-[1,2-bis(diphenylphosphin)ethan]palladium(0) und [1,4-Bis(diphenylphosphin)butan]palladium(II)-chlorid. Die Menge an Katalysator beträgt üblicherweise 0,1 bis 10 mol-%, bezogen auf die Verbindung II. Das Molverhältnis von Verbindung II zu dem (Het)arylboronsäurederivat liegt typischerweise im Bereich von 1:2 bis 2:1.

In zu Schema 1 analoger Weise können Verbindungen der Formel I, worin R⁵ für Alkyl, Cycloalkyl, gegebenenfalls substituiertes Benzyl oder Heteroarylmethyl steht, auf dem Wege einer Übergangsmetall-katalysierten Kupplungsreaktion durch Umsetzung von Verbindung II mit einer metallorganischen Verbindung Met-R⁵, worin R⁵ die zuvor genannte Bedeutung hat und Met für einen Rest MgX, SnR₃ oder ZnX (X = Chlor, Brom oder Iod, R = Alkyl) steht, beispielsweise im Sinne einer Stille-Kupplung oder Kumada-Kupplung, hergestellt werden.

In zu Schema 1 analoger Weise können Verbindungen der Formel I, worin R⁵ für Alkoxy, Haloalkoxy, Cycloalkoxy, gegebenenfalls substituiertes Phenoxy oder Heteroaryloxy steht, durch Umsetzung von Verbindung II mit dem entsprechenden Alkoholat OR⁵, worin R⁵ die zuvor genannte Bedeutung hat, im Sinne einer nucleophilen Substitution hergestellt werden.

Die 3-(6-Halogenpyridin-2-yl)-triazine der Formel II können ihrerseits auf den in den folgenden Schemata dargestellten Methoden aus den entsprechenden Amidinverbindungen der Formel III hergestellt werden.

Die Herstellung von Verbindungen II, worin R¹, R² unabhängig voneinander für Alkyl, Halogenalkyl oder gemeinsam mit den C-Atomen, an die sie gebunden sind, einen gesättigten Carbocyclus oder einen Heterocyclus gemäß obiger Definition bilden, gelingt beispielsweise gemäß der in Schema 2 dargestellten Synthese.

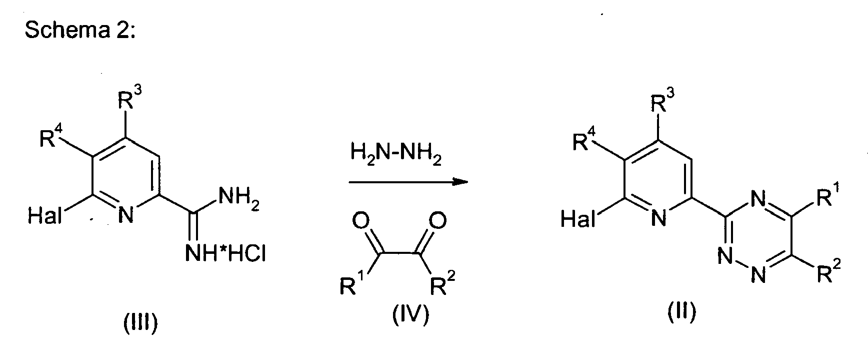

In Schema 2 haben Hal, R³ und R⁴ die zuvor genannten Bedeutungen. R¹ und R² stehen unabhängig voneinander für C₁-C₈-Alkyl, C₁-C₈-Halogenalkyl oder bilden gemeinsam mit den C-Atomen, an die sie gebunden sind, einen gesättigten Carbocyclus oder einen Heterocyclus, beispielsweise einen der zuvor genannten Cyclen Q-1, Q-4 oder Q-8. Gemäß Schema 2 wird die Amidinverbindung der Formel III sukzessive oder als Eintopfreaktion mit Hydrazin und der Verbindung IV umgesetzt, wobei man die Verbindung der Formel II erhält. In der Regel erfolgt zunächst die Umsetzung mit dem Hydrazin, das typischerweise als Hydrazin-Hydrat eingesetzt wird. Anschließend setzt man die Verbindung IV zu. Gegebenenfalls erhöht man zur Vervollständigung der Umsetzung die Temperatur.

Die Verbindungen der Formel IV sind bekannt oder können nach Standardverfahren hergestellt werden, beispielsweise nach EP 267 378, Tetrahedron Letters, 2003, S. 2307, Tetrahedron Letters, 1992, S. 8131, Tetrahedron Letters, 1987, S. 551, J. Molec. Catalysis 208 (1-2), 2004, S. 135-145. Die Herstellung von 3,4-Dioxaoxolan gelingt beispielsweise durch Oxidation des 3,4-Bishydroxyoxolans mit N-Bromsuccinimid und Tetrachlormethan in Pyridin (siehe Tetrahedron Letters, 44 (2003) S. 4909 oder mit o-Iodosobenzoesäure in Dimethylsulfoxid (siehe Tetrahedron Letters, 35 (1994) S. 8019). Die Amidinverbindungen III sind ebenfalls bekannt oder können beispielsweise analog der in US 2003/0087940 A1 und Bioorg. Med. Chem. Lett. 1571-1574 (2003) beschriebenen Methoden hergestellt werden.

In zu Schema II analoger Weise können die Verbindungen der Formel II, worin R¹ und R² gemeinsam mit den C-Atomen, an die sie gebunden sind, für einen Rest Q-2 oder Q-3 stehen, auf der in Schema 3 und 4 gezeigten Syntheseroute hergestellt werden:

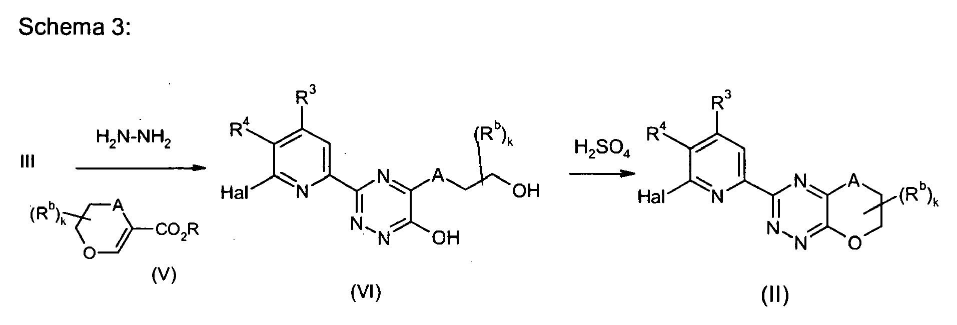

In Schema 3 haben Hal, k, R^{b}, R³ und R⁴ die zuvor genannten Bedeutungen. A steht für CH₂ oder eine chemische Bindung. R steht für C₁-C₄-Alkyl, insbesondere Methyl oder Ethyl. Gemäß Schema 3 werden die Amidinverbindung III sukzessive mit Hydrazin und dem Ester der Formel V umgesetzt. Bezüglich der Reaktionsbedingungen gilt das für Schema 2 Gesagte in analoger Weise. Die auf diese Weise erhaltene Bishydroxyverbindung der Formel VI wird anschließend einer cyclisierenden Dehydratisierung unterworfen, beispielsweise durch Behandlung mit Schwefelsäure. Die Ester der Formel V sind bekannt oder können in Analogie zu literaturbekannten Verfahren hergestellt werden (siehe J. Heterocycl. Chem., 32 (1995) S. 735 und Liebigs Ann. Chem. 1974, S. 468-476).

Die Verbindungen der allgemeinen Formel III können ihrerseits aus den entsprechenden 2-Cyanopyridinverbindungen der allgemeinen Formel VII hergestellt werden (siehe Schema 4). Hierzu wird die 2-Cyanopyridinverbindung VII nach der in US 4,873,248 beschriebenen Methode durch sukzessive Behandlung mit Alkalimetallalkoholat wie Natriummethanolat oder -ethanolat und anschließende Umsetzung mit Ammoniumchlorid in die Verbindung III überführt. Anstelle der Hydrochloride können in den in den Schemata 1 bis 3 gezeigten Folgeschritten auch die Hydrobromide, Acetate, Sulfate oder Formiate eingesetzt werden. Die Cyanopyridine der Formel VII sind bekannt, z. B. aus US 2003/087940, WO 2004/026305, WO 01/057046 und Bioorg. Med. Chem. Lett. S. 1571-1574 (2003) oder können nach bekannten Herstellungsverfahren hergestellt werden.

Gemäß einer zweiten Syntheseroute (siehe Schema 4) können die erfindungsgemäßen Verbindungen, worin R⁵ für einen Rest Ar, wie in Schema 1 definiert, steht, ausgehend von den Cyanopyridinen VII hergestellt werden. Hierzu führt man zunächst eine Kupplung der Verbindung VII mit der (Het)arylboronsäureverbindung Ar-B(OR)₂, wie für Schema 1 beschrieben, durch und wandelt das dabei erhaltene 6-(Het)aryl-2-cyanopyridin unter den für Verbindungen VII beschriebenen Reaktionsbedingungen in die Amidinverbindung IX um. Verbindung IX kann dann unter den für die Schemata 2 und 3 genannten Bedingungen in die entsprechende Triazinverbindung überführt werden.

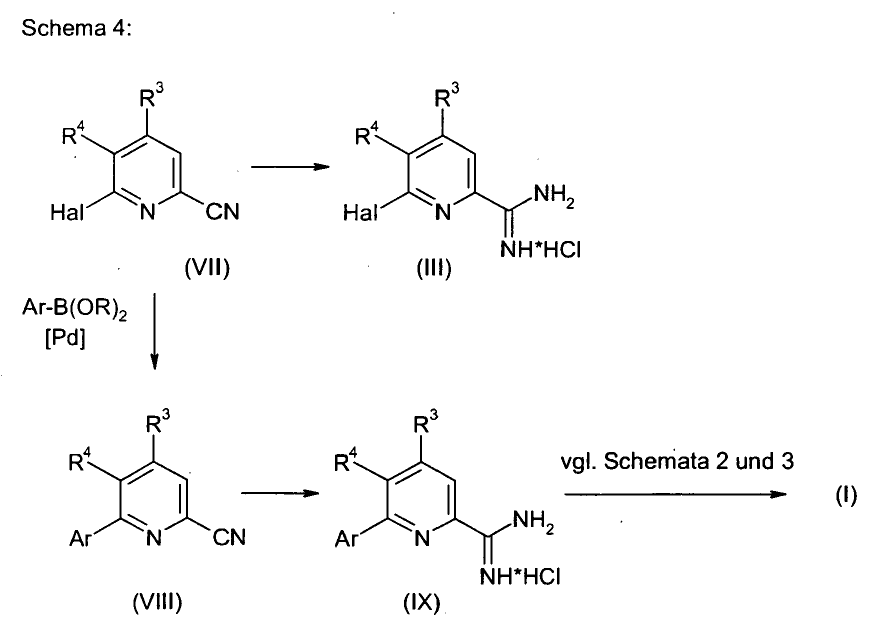

Im Übrigen sind Verbindungen der Formel VIIIa bekannt, worin R⁵ eine der zuvor genannten Bedeutungen, insbesondere eine von Ar verschiedene Bedeutung aufweist. Die Verbindungen VIIIa können nach zu den Schemata 2 bis 4 analogen Methoden in die entsprechenden erfindungsgemäßen Verbindungen I umgewandelt werden: 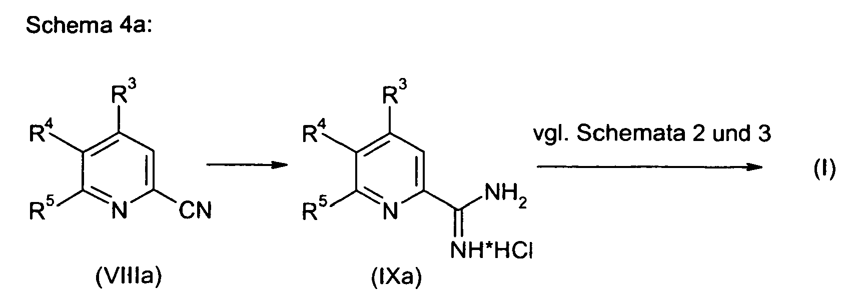 Verbindungen der allgemeinen Formel VII können, soweit sie nicht bekannt sind, insbesondere nach dem in Schema 5 dargestellten Verfahren hergestellt werden.

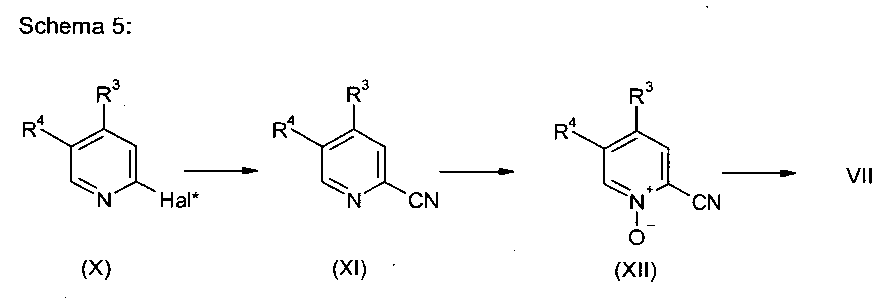

In Schema 5 haben R³ und R⁴ die zuvor genannten Bedeutungen. Hal* steht für Chlor, Brom oder Iod.

Die Umwandlung des 2-Halogenpyridins X in das 2-Cyanopyridin XI gelingt nach Standardmethoden der organischen Chemie durch Umsetzung von X mit Cyanidionen, z. B. mit Natrium- oder Kaliumcyanid (siehe EP-A 97460, Herstellungsbeispiel 1), Kupfer(I)-cyanid (siehe EP-A 34917, Herstellungsbeispiel 3) oder Trimethylsilylcyanid. Anschließend wird die so erhaltene Verbindung XI durch Behandlung mit einer Persäure nach an sich bekannten Methoden in das Pyridin-N-oxid XII umgewandelt. Die Umwandlung von XI in XII kann in Analogie zu bekannten Verfahren erfolgen, beispielsweise durch Behandlung von XI mit Wasserstoffperoxid in einer organischen Säure wie Ameisensäure, Essigsäure, Chloressigsäure oder Trifluoressigsäure (siehe z. B. J. Org. Chem. 55, S. 738-741 (1990) und Organic Synthesis, Collect. Vol. IV, S. 655-656 (1963)) oder durch Umsetzung von XI mit einer organischen Persäure wie meta-Chlorperbenzoesäure in einem inerten Lösungsmittel, z. B. einem halogenierten Kohlenwasserstoff wie Dichlormethan oder Dichlorethan (siehe z. B. Synthetic Commun. 22(18), S. 2645, (1992); J. Med. Chem. 2146 (1998)). Die Umwandlung von XI in XII gelingt auch in Analogie zu der von K. B. Sharpless (J. Org. Chem. 63(5), S. 7740 (1998)) beschriebenen Methode durch Umsetzung von XI mit Wasserstoffperoxid in einem halogenierten Kohlenwasserstoff wie Dichlormethan oder Dichlorethan in Gegenwart katalytischer Mengen (z. B. 5 Gew.-%) Rhenium(VII)-Verbindungen wie Methyltrioxorhenium (H₃CReO₃).

Anschließend wird XII mit einem Halogenierungsmittel wie POCl₃ oder POBr₃ umgesetzt, wobei man die entsprechende Verbindung VII erhält. Bei der Umsetzung von XII zu VII setzt man das Halogenierungsmittel in der Regel im Überschuss, bezogen auf die Stöchiometrie der Reaktion ein. Die Umsetzung kann in einem inerten organischen Lösungsmittel durchgeführt werden und erfolgt häufig in Abwesenheit eines Lösungsmittels, wobei dann in der Regel das Halogenierungsmittel als Lösungsmittel fungiert. Die Reaktionstemperatur liegt üblicherweise im Bereich von 20 °C bis zur Siedetemperatur des Halogenierungsmittels. Gegebenenfalls ist es von Vorteil, zunächst mit einem Chlorierungsmittel wie POCl₃ ein Chloratom in die 2-Position des Pyridin-N-Oxids XII einzuführen und anschließend einen Halogenaustausch, z.B. durch Behandlung mit HBr oder einem Iodierungsmittel, vorzunehmen, wobei man eine Verbindung der Formel VII mit Hal = Br oder I erhält.

Weiterhin kann man die erfindungsgemäßen Verbindungen I gemäß der in Schema 6 dargestellten Synthese herstellen:

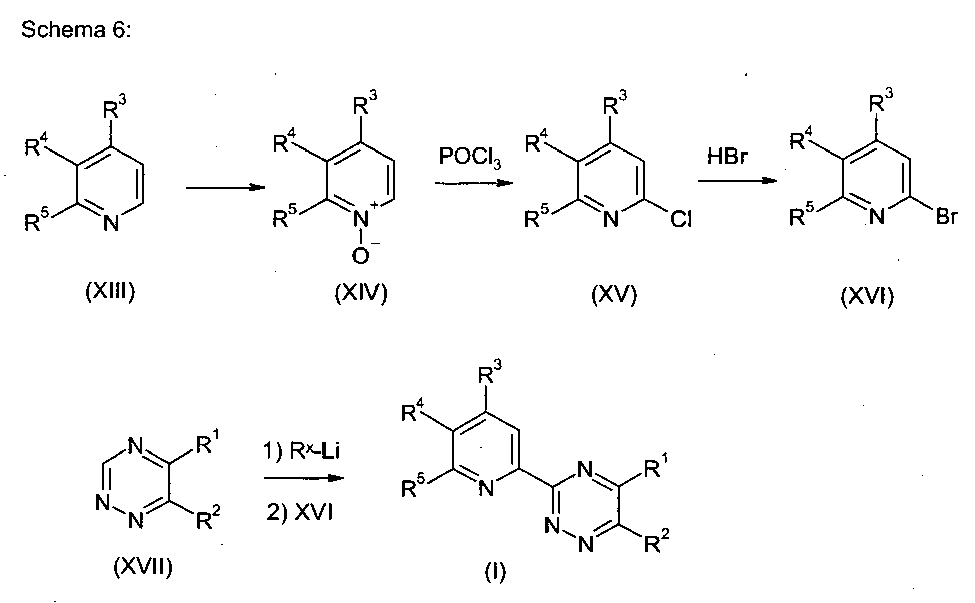

In Schema 6 haben R¹, R², R³, R⁴ und R⁵ die zuvor genannten Bedeutungen. R^{x} steht beispielsweise für C₁-C₆-Alkyl, speziell n-Butyl, oder für Phenyl.

Gemäß Schema 6 wird in einem ersten Schritt die Pyridinverbindung XIII nach Standardmethoden der organischen Chemie in das entsprechende N-Oxid XIV überführt. Die in Schema 5 für die Umwandlung der Verbindung XI in die Verbindung XII genannten Methoden können entsprechend angewendet werden. Das N-Oxid XIV wird anschließend durch Umsetzung mit einem Chlorierungsmittel wie POCl₃ in das entsprechende 2-Chlorpyridin XV umgewandelt. Die in Schema 5 für die Umwandlung der Verbindung XII in die Verbindung VII genannten Methoden können entsprechend angewendet werden. Anschließend führt man einen Chlor-Brom-Austausch nach Standardmethoden der Organischen Chemie, beispielsweise durch Behandlung des Chlorpyridins XV mit HBr in Analogie zu der in US 5,271,217 und Can. J. Chem. 75 (2) (1997) S. 169 genannten Methode durch.

Das auf diese Weise erhaltene Brompyridin wird anschließend mit der Triazinverbindung XVII gekuppelt. Hierzu wird die Triazinverbindung der Formel XVII durch Behandlung mit einer Lithiumbase, insbesondere einer C₁-C₆-Alkyllithiumverbindung wie n-Butyllithium oder Phenyllithium in die entsprechende Lithium-Triazinverbindung überführt, welche anschließend mit der Brompyridinverbindung XVI zur Reaktion gebracht wird. Die Umsetzung von XVI mit der lithiierten Verbindung XVII kann in Analogie zu der in Tetrahedron Lett. 41(10) (2000) S. 1653 beschriebenen Methode durchgeführt werden.

Die Triazinverbindungen XVII sind bekannt oder können in Analogie zu bekannten Methoden zur Herstellung von Triazinverbindungen hergestellt werden. Triazinverbindungen XVII, in denen R¹ und R² für über Sauerstoff gebundene Reste stehen können ausgehend von 5,6-Dialkoxy-1,2,4-triazinen wie 5,6-Dimethoxy-1,2,4-triazin (siehe Chem. Ber. 109 (1976), S.1113) hergestellt werden:

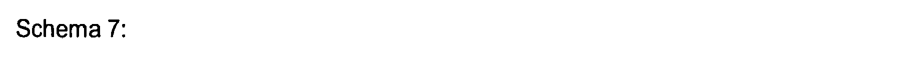

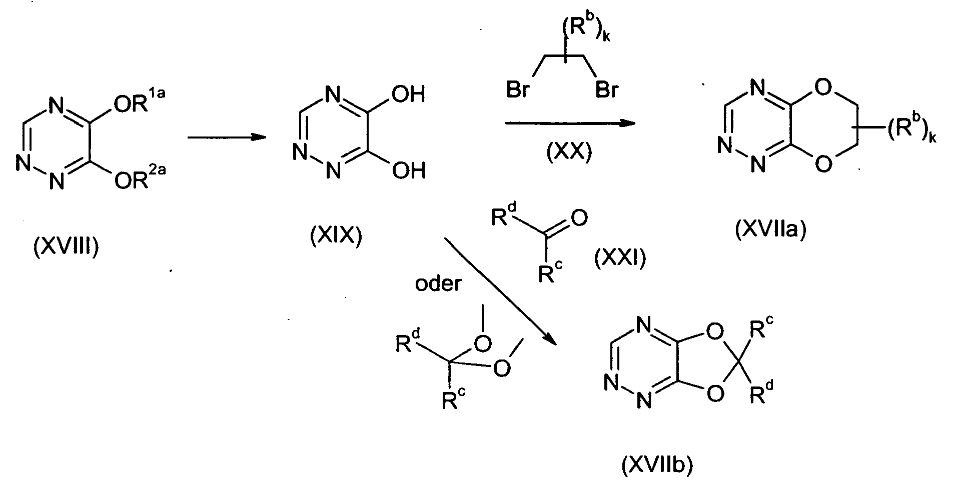

In Schema 7 haben R^{b} und k die zuvor genannten Bedeutungen. R^{1a} und R^{1b} stehen für C₁-C₆-Alkyl, insbesondere Methyl. R^{c} und R^{d} stehen unabhängig voneinander für Wasserstoff oder C₁-C₄-Alkyl. Die Umwandlung von XVIII in das 5,6-Bishydroxy-1,2,4-triazin gelingt in Analogie zu der in Chem. Ber. 109 (1976), S.1113 beschriebenen Methode. Das auf diese Weise erhaltene Bishydroxytriazin XIX kann anschließend mit einem 1,2-Dibromalkan XX, vorzugsweise in Gegenwart einer Base wie Alkalimetallhydroxid oder Alkalialkoholat in Analogie zu der in Heterocycl. Chem. 27 (1990) S. 151 beschriebenen Methode umgesetzt werden, wobei man das kondensierte Triazin XVIIa erhält. Außerdem kann man das Bishydroxytriazin XIX in Analogie zu der in Chem. Berichte 124(3) (1991) S. 481, J. Chem. Socl., Perkin Trans 1, 1998, S. 3561; Synthesis (1986), S. 122; beschriebenen Methode mit einem Keton bzw. Aldehyd XXI umsetzen, wobei man das kondensierte Triazin XVIIb erhält.

Die nach den in den Schemata 1 bis 7 dargestellten Methoden erhaltenen Reaktionsgemische werden in üblicher Weise aufgearbeitet, z. B. durch Mischen mit Wasser, Trennung der Phasen und gegebenenfalls chromatographische Reinigung der Rohprodukte. Die Zwischen- und Endprodukte fallen z. T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voran stehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z. B. unter Licht-, Säure- oder Baseneinwirkung) ineinander umwandeln können. Entsprechende Umwandlungen können auch nach der Anwendung, beispielsweise bei der Behandlung von Pflanzen, in der behandelten Pflanze oder im zu bekämpfenden Schadpilz erfolgen.

Die Verbindungen der Formel I eignen sich als Fungizide. Sie zeichnen sich aus durch eine hervorragende Wirksamkeit gegen ein breites Spektrum von pflanzenpathogenen Pilzen aus der Klasse der Ascomyceten, Deuteromyceten, Oomyceten und Basidiomyceten, insbesondere aus der Klasse der Oomyceten. Sie sind zum Teil systemisch wirksam und können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbissen, sowie an den Samen dieser Pflanzen.

Speziell eignen sie sich zur Bekämpfung folgender Pflanzenkrankheiten:
- Alternaria Arten an Gemüse, Raps, Zuckerrüben, Obst, Reis, Sojabohnen sowie an Kartoffeln (z. B. A. solani oder A. alternata) und Tomaten (z. B. A. solani oder A. alternata) und Alternaria ssp. (Ährenschwärze) an Weizen,
- Aphanomyces Arten an Zuckerrüben und Gemüse,
- Ascochyta Arten an Getreide and Gemüse z. B. Ascochyta tritici (Blattdürre) an Weizen,
- Bipolaris- und Drechslera Arten an Mais, Getreide, Reis und Rasen (z. B. D. maydis an Mais, D. teres an Gerste, D. tritici-repentis an Weizen),
- Blumeria graminis (Echter Mehltau) an Getreide (z.B. Weizen oder Gerste),
- Botrytis cinerea (Grauschimmel) an Erdbeeren, Gemüse, Blumen, Weizen und Weinreben,
- Bremia lactucae an Salat,
- Cercospora Arten an Mais, Sojabohnen, Reis und Zuckerrüben und z. B. Cercospora sojina (Blattflecken) oder Cercospora kikuchii (Blattflecken) an Sojabohnen, .
- Cladosporium herbarum (Ährenschwärze) an Weizen,
- Cochliobolus Arten an Mais, Getreide, Reis (z. B. Cochliobolus sativus an Getreide, Cochliobolus miyabeanus an Reis),
- Colletotricum Arten an Sojabohnen, Baumwolle und anderen Pflanzen (z. B. C. acutatum an verschiedenen Pflanzen) und z. B. Colletotrichum truncatum (Antracnose) an Sojabohnen),
- Corynespora cassiicola (Blattflecken) an Sojabohnen,
- Dematophora necatrix (Wurzel-/Stengelfäule) an Sojabohnen,
- Diaporthe phaseolorum (Stengelkrankheit) an Sojabohnen,
- Drechslera Arten, Pyrenophora Arten an Mais, Getreide, Reis und Rasen, an Gerste (z. B. D. teres) und an Weizen (z. B. D. tritici-repentis),
- Esca an Weinrebe, verursacht durch Phaeoacremonium chlamydosporium, Ph. Aleophilum, und Fomitipora punctata (syn. Phellinus punctatus),
- Elsinoe ampelina an Weinrebe,
- Epicoccum spp. (Ährenschwärze) an Weizen,
- Exserohilum Arten an Mais,
- Erysiphe cichoracearumund Sphaerotheca fuliginea an Gurkengewächsen,
- Fusarium und Verticillium Arten (z. B. V. dahliae) an verschiedenen Pflanzen: z. B. F. graminearum oder F. culmorum (Wurzelfäule) an Getreide (z. B. Weizen oder Gerste) oder z. B. F. oxysporum an Tomaten und Fusarium solani (Stengelkrankheit) an Sojabohnen,
- Gaeumanomyces graminis an Getreide (z. B. Weizen oder Gerste),
- Gibberella Arten an Getreide und Reis (z. B. Gibberella fujikuroi an Reis),
- Glomerella cingulata an Weinrebe und anderen Pflanzen,
- Grainstaining complex an Reis,
- Guignardia budwelli an Weinrebe,
- Helminthosporium Arten (z. B. H. graminicola) an Mais und Reis,
- Isariopsis clavispora an Weinrebe,
- Macrophomina phaseolina (Wurzel-/Stengelfäule) an Sojabohnen,
- Michrodochium nivale an Getreide (z. B. Weizen oder Gerste),
- Microsphaera diffusa (Echter Mehltau) an Sojabohnen,
- Mycosphaerella Arten an Getreide, Bananen und Erdnüssen (M. graminicola an Weizen, M. fijiesis an Banane),
- Peronospora Arten an Kohl (z. B. P. brassicae), Zwiebelgewächsen (z. B. P. destructor) und z. B. Peronospora manshurica (Falscher Mehltau) an Sojabohnen,
- Phakopsara pachyrhizi und Phakopsara meibomiae an Sojabohnen,
- Phialophora gregata (Stengelkrankheit) an Sojabohnen,
- Phomopsis Arten an Sojabohnen, Sonnenblumen und Weinreben (P. viticola an Weinreben, P. helianthii an Sonnenblumen),
- Phytophthora Arten an verschiedenen Pflanzen z. B. P. capsici an Paprika, Phytopthora megasperma (Blatt-/Stengelfäule) an Sojabohnen, Phytophthora infestans an Kartoffeln und Tomaten,
- Plasmopara viticola an Weinreben,
- Podosphaera leucotricha an Apfel,
- Pseudocercosporella herpotrichoides an Getreide,
- Pseudoperonospora Arten an Hopfen und Gurkengewächsen (z. B. P. cubenis an Gurke oder P. humili an Hopfen)
- Pseudopezicula tracheiphilai an Weinrebe,
- Puccinia Arten an verschiedenen Pflanzen z. B. P. triticina, P. striformins, P. hordei oder P. graminis an Getreide (z.B. Weizen oder Gerste) oder an Spargel (z. B. P. asparagi),
- Pyrenophora Arten an Getreide,
- Pyricularia oryzae, Corticium sasakii, Sarocladium oryzae, S. attenuatum, Entyloma oryzae an Reis, Pyricularia grisea an Rasen und Getreide,
- Pythium spp. an Rasen, Reis, Mais, Baumwolle, Raps, Sonnenblumen, Zuckerrüben, Gemüse und anderen Pflanzen (z. B. P. ultiumum oder P. aphanidermatum),
- Ramularia collo-cygni (Ramularia/Sonnenbrand-Komplex/Physiological leaf spots) an Gerste,
- Rhizoctonia-Arten (z. B. R. solani) an Baumwolle, Reis, Kartoffeln, Rasen, Mais, Raps, Zuckerrüben, Gemüse und anderen Pflanzen, z. B. Rhizoctonia solani (Wurzel-/Stengelfäule) an Sojabohnen oder Rhizoctonia cerealis (Spitzer Augenfleck) an Weizen oder Gerste,
- Rhynchosporium secalis an Gerste (Blattflecken), Roggen und Triticale,
- Sclerotinia Arten an Raps, Sonnenblumen und anderen Pflanzen, z. B. Sclerotinia sclerotiorum (Stengelkrankheit) oder Sclerotinia rolfsii (Stengelkrankheit) an Sojabohnen,
- Septoria glycines (Blattflecken) an Sojabohnen,
- Septoria tritici und Stagonospora nodorum an Weizen,
- Erysiphe (syn. Uncinulanecator) an Weinrebe,
- Setospaeria Arten an Mais und Rasen,
- Sphacelotheca reilinia an Mais,
- Stagonospora nodorum (Ährenseptoria) an Weizen,
- Thievaliopsis Arten an Sojabohnen und Baumwolle,
- Tilletia Arten an Getreide,
- Typhula incamata (Schneefäule) an Weizen oder Gerste,
- Ustilago Arten an Getreide, Mais und Zuckerrübe und
- Venturia Arten (Schorf) an Apfel und Birne (z. B. V. inaequalis an Apfel).

Die Verbindungen der Formel I eignen sich außerdem zur Bekämpfung von Schadpilzen im Materialschutz (z. B. Holz, Papier, Dispersionen für den Anstrich, Fasern bzw. Gewebe) und im Vorratsschutz. Im Holzschutz finden insbesondere folgende Schadpilze Beachtung:
Ascomyceten wie Ophiostoma spp., Ceratocystis spp., Aureobasidium pullulans, Sclerophoma spp., Chaetomium spp., Humicola spp., Petriella spp., Trichurus spp.; Basidiomyceten wie Coniophora spp., Coriolus spp., Gloeophyllum spp., Lentinus spp., Pleurotus spp., Poria spp., Serpula spp. und Tyromyces spp., Deuteromyceten wie Aspergillus spp., Cladosporium spp., Penicillium spp., Trichoderma spp., Alternaria spp., Paecilomyces spp. und Zygomyceten wie Mucor spp., darüber hinaus im Materialschutz folgende Hefepilze: Candida spp. und Saccharomyces cerevisae.

Die Verbindungen der Formel I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im Allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 1 bis 1000 g/100 kg, vorzugsweise 5 bis 100 g/100 kg Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen der Formel I können in verschiedenen Kristallmodifikationen vorliegen, die sich in der biologischen Wirksamkeit unterscheiden können. Sie sind ebenfalls Gegenstand der vorliegenden Erfindung.

Die Verbindungen der Formel I können in die üblichen Formulierungen überführt werden, z. B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z. B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im Wesentlichen in Betracht:
- Wasser, aromatische Lösungsmittel (z. B. Solvesso Produkte, Xylol), Paraffine (z. B. Erdölfraktionen), Alkohole (z. B. Methanol, Butanol, Pentanol, Benzylalkohol), Ketone (z. B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristearylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z. B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogehgranulate, können durch Bindung der Wirkstoffe an feste Trägerstoffe hergestellt werden. Feste Trägerstoffe sind z. B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten im Allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:

### 1. Produkte zur Verdünnung in Wasser

A Wasserlösliche Konzentrate (SL, LS)
   10 Gew.-Teile der Wirkstoffe werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.
B Dispergierbare Konzentrate (DC)
   20 Gew.-Teile der Wirkstoffe werden in 70 Gew.-Teilen Cyclohexanon unter Zusatz von 10 Gew.-Teilen eines Dispergiermittels z. B. Polyvinylpyrrolidon gelöst. Bei Verdünnung in Wasser ergibt sich eine Dispersion. Der Wirkstoffgehalt beträgt 20 Gew.-%.
C Emulgierbare Konzentrate (EC)
   15 Gew.-Teile der Wirkstoffe werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.
D Emulsionen (EW, EO, ES)
   25 Gew.-Teile der Wirkstoffe werden in 35 Gew.-Teile Xylol unter Zusatz von Ca-Dodecylbenzolsulfonat und Ricinusölethoxylat (jeweils 5 Gew.-Teile) gelöst. Diese Mischung wird mittels einer Emulgiermaschine (z. B. Ultraturax) in 30 Gew.-Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.
E Suspensionen (SC, OD, FS)
   20 Gew.-Teile der Wirkstoffe werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-%.
F Wasserdispergierbare und wasserlösliche Granulate (WG, SG)
   50 Gew.-Teile der Wirkstoffe werden unter Zusatz von 50 Gew.-Teilen Dispergier- und Netzmitteln fein gemahlen und mittels technischer Geräte (z. B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.
G Wasserdispergierbare und wasserlösliche Pulver (WP, SP, SS, WS)
   75 Gew.-Teile der Wirkstoffe werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.
H Gelformulierungen
   In einer Kugelmühle werden 20 Gew.-Teile der Wirkstoffe, 10 Gew.-Teile Dispergiermittel, 1 Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

### 2. Produkte für die Direktapplikation

I Stäube (DP, DS)
   5 Gew.-Teile der Wirkstoffe werden fein gemahlen und mit 95 Gew.-Teilen feinteiligem Kaolin innig vermischt. Man erhält dadurch ein Stäubemittel mit 5 Gew.-% Wirkstoffgehalt.
J Granulate (GR, FG, GG, MG)
   0,5 Gew.-Teile der Wirkstoffe werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit
   0,5 Gew.-% Wirkstoffgehalt.
K ULV- Lösungen (UL)
   10 Gew.-Teile der Wirkstoffe werden in 90 Gew.-Teilen eines organischen Lösungsmittel z. B. Xylol gelöst. Dadurch erhält man ein Produkt für die Direktapplikation mit 10 Gew.-% Wirkstoffgehalt.

Für die Saatgutbehandlung werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gelformulierungen (GF) verwendet. Diese Formulierungen können auf das Saatgut unverdünnt oder, bevorzugt, verdünnt angewendet werden. Die Anwendung kann vor der Aussaat erfolgen.

Die Wirkstoffe können als solche, in Form ihrer Formulierungen oder den daraus bereiteten Anwendungsformen, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im Allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 100 bis 100 : 1, bevorzugt 1 : 10 bis 10 : 1 zugemischt werden.

Als Adjuvants in diesem Sinne kommen insbesondere in Frage: organisch modifizierte Polysiloxane, z. B. Break Thru S 240^{®}; Alkoholalkoxylate, z. B. Atplus 245^{®}, Atplus MBA 1303^{®}, Plurafac LF 300^{®} und Lutensol ON 30^{®}; EO-PO-Blockpolymerisate, z. B. Pluronic RPE 2035^{®} und Genapol B^{®}; Alkoholethoxylate, z. B. Lutensol XP 80^{®}; und Natriumdioctylsulfosuccinat, z. B. Leophen RA^{®}.

Die erfindungsgemäßen Mittel können in der Anwendungsform als Fungizide auch zusammen mit anderen Wirkstoffen vorliegen, der z. B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:

### Strobilurine

Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl]-benzyl)-carbaminsäuremethylester, 2-(ortho-(2,5-Dimethylphenyl-oxymethylen)phenyl)-3-methoxy-acrylsäuremethylester;

### Carbonsäureamide

- Carbonsäureanilide: Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluor-biphenyl-2-yl)-amid, 3,4-Dichlor-isothiazol-5-carbonsäure-(2-cyano-phenyl)-amid;
- Carbonsäuremorpholide: Dimethomorph, Flumorph;
- Benzoesäureamide: Flumetover, Fluopicolide (Picobenzamid), Zoxamide;
- Sonstige Carbonsäureamide: Carpropamid, Diclocymet, Mandipropamid, N-(2-(4-[3-(4-Chlor-phenyl)-prop-2-inyloxy]-3-methoxy-phenyl)-ethyl)-2-methansulfonylamino-3-methyl-butyramid, N-(2-(4-[3-(4-Chlor-phenyl)-prop-2-inyloxy]-3-methoxy-phenyl)-ethyl)-2-ethansulfonylamino-3-methyl-butyramid;

### Azole

- Triazole: Bitertanol, Bromuconazole, Cyproconazole, Difenoconazole, Diniconazole, Enilconazole, Epoxiconazole, Fenbuconazole, Flusilazole, Fluquinconazole, Flutriafol, Hexaconazol, Imibenconazole, Ipconazole, Metconazol, Myclobutanil, Penconazole, Propiconazole, Prothioconazole, Simeconazole, Tebuconazole, Tetraconazole, Triadimenol, Triadimefon, Triticonazole;
- Imidazole: Cyazofamid, Imazalil, Pefurazoate, Prochloraz, Triflumizole;
- Benzimidazole: Benomyl, Carbendazim, Fuberidazole, Thiabendazole;
- Sonstige: Ethaboxam, Etridiazole, Hymexazole;

### Stickstoffhaltige Heterocyclylverbindungen

- Pyridine: Fluazinam, Pyrifenox, 3-[5-(4-Chlor-phenyl)-2,3-dimethyl-isoxazolidin-3-yl]-pyridin;
- Pyrimidine: Bupirimate, Cyprodinil, Ferimzone, Fenarimol, Mepanipyrim, Nuarimol, Pyrimethanil;
- Piperazine: Triforine;
- Pyrrole: Fludioxonil, Fenpiclonil;
- Morpholine: Aldimorph, Dodemorph, Fenpropimorph, Tridemorph;
- Dicarboximide: Iprodione, Procymidone, Vinclozolin;
   - sonstige: Acibenzolar-S-methyl, Anilazin, Captan, Captafol, Dazomet, Diclomezine, Fenoxanil, Folpet, Fenpropidin, Famoxadone, Fenamidone, Octhilinone, Probenazole, Proquinazid, Pyroquilon, Quinoxyfen, Tricyclazole, 5-Chlor-7-(4-methylpiperidin-1-yl)-6-(2,4,6-trifluor-phenyl)-[1,2,4]triazolo[1,5-a]pyrimidin, 2-Butoxy-6-iodo-3-propyl-chromen-4-on, 3-(3-Brom-6-fluor-2-methyl-indol-1-sulfonyl)-[1,2,4]triazol-1-sulfonsäuredimethylamid;

### Carbamate und Dithiocarbamate

- Dithiocarbamate: Ferbam, Mancozeb, Maneb, Metiram, Metam, Propineb, Thiram, Zineb, Ziram;
- Carbamate: Diethofencarb, Flubenthiavalicarb, Iprovalicarb, Propamocarb, 3-(4-Chlor-phenyl)-3-(2-isopropoxycarbonylamino-3-methyl-butyrylamino)-propionsäuremethylester, N-(1-(1-(4-cyanophenyl)ethansulfonyl)-but-2-yl) carbaminsäure-(4-fluorphenyl)ester;

### Sonstige Fungizide

- Guanidine: Dodine, Iminoctadine, Guazatine;
- Antibiotika: Kasugamycin, Polyoxine, Streptomycin, Validamycin A;
- Organometallverbindungen: Fentin-Salze;
- Schwefelhaltige Heterocyclylverbindungen: Isoprothiolane, Dithianon;
- Organophosphorverbindungen: Edifenphos, Fosetyl, Fosetylaluminium, Iprobenfos, Pyrazophos, Tolclofos-methyl, Phosphorige Säure und ihre Salze;
- Organochlorverbindungen: Thiophanate Methyl, Chlorothalonil, Dichlofluanid, Tolylfluanid, Flusulfamide, Phthalide, Hexachlorbenzene, Pencycuron, Quintozene;
- Nitrophenylderivate: Binapacryl, Dinocap, Dinobuton;
- Anorganische Wirkstoffe: Bordeaux Brühe, Kupferacetat, Kupferhydroxid, Kupferoxychlorid, basisches Kupfersulfat, Schwefel;
- Sonstige: Spiroxamine, Cyflufenamid, Cymoxanil, Metrafenone.

### Herstellungsbeispiele:

### Beispiel 1: 5,6-Diethyl-3-[6-(4-fluor-phenyl)-5-methyl-pyridin-2-yl]-[1,2,4]-triazin

### 1) Herstellung von 6-Brom-5-methylpyridin-2-carboxamidin-Hydrochlorid

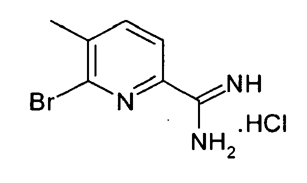

Zu 4,90 g (25 mmol) 6-Brom-5-methylpyridin-2-carbonitril [Zur Herstellung, siehe US 2003/0087940 A1 und Bioorg. Med. Chem. Lett. 1571-1574 (2003)] in 60 ml Methanol gab man 2,2 g einer 30%igen Natriummethylat-Lösung in Methanol und rührte 7 Stunden bei 23 °C nach. Anschließend gab man 1,5 g Ammoniumchlorid dazu und ließ weitere 8 Stunden bei 23 °C nachrühren. Nach Entfernen des Lösungsmittels versetzte man mit Methyl-tert.-butylether (MtBE) und saugte das Produkt ab. Ausbeute: 4,2 g als weißer Feststoff der ohne Aufreinigung weiter umgesetzt wurde.

### 2) Herstellung von 3-(6-Brom-5-methyl-pyridin-2-yl)-5,6-diethyl-[1,2,4]-triazin

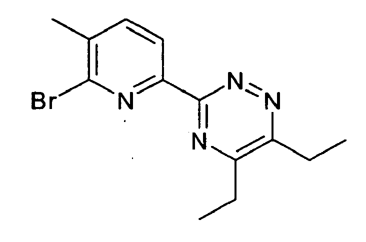

Zu der Lösung von 10 g (40 mmol) der in Schritt 1 hergestellten Verbindung in 100 ml Ethanol gab man 2,4 g (48 mmol) Hydraziniumhydroxid. Nach 30 min versetzte man mit 4,6 g (40 mmol) 3,4-Hexandion, rührte 5 Stunden unter Rückfluss und ließ für 12 Stunden bei 23 °C stehen. Zur Reaktionslösung gab man Wasser und n-Pentan und saugte das ausgefallene Produkt ab. Ausbeute: 9,9 g Produkt.
¹H-NMR (δ , CDCl₃,): 1,4 (m); 2,5 (s); 2,9 (m); 3,1 (m); 7,7 (m) und 8,4 (m).

### 3) Herstellung von 5,6-Diethyl-3-[6-(4-fluor-phenyl)-5-methyl-pyridin-2-yl]-[1,2,4]-triazin

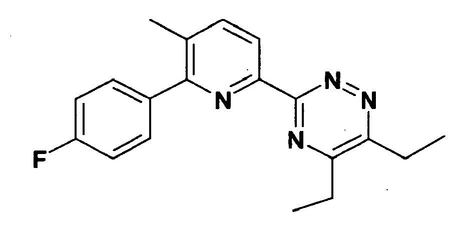

Zu der Lösung von 0,4 g der im Schritt 2 hergestellten Verbindung in 20 ml Ethylenglykoldimethylether gab man nacheinander 0,21 g 4-Fluorphenylboronsäure, und 0,41 g Natriumcarbonat in 20 ml Wasser. Nach Zugabe von ca. 30 mg [1,4-Bis(diphenylphosphino)butan]-palladium(II)-dichlorid rührte man 5 Stunden unter Rückfluss. Die Reaktionslösung wurde danach zwischen Wasser und MtBE verteilt. Die organische Phase wurde abgetrennt, das Lösungsmittel unter Vakuum entfernt und der Rückstand über Kieselgel mit Cyclohexan/MtBE (1:1) chromatographiert. Man erhielt so 0,24 g Produkt.
Fp. 108 °C

### Beispiel 2: 3-[6-(4-Fluorphenyl)-5-methyl-pyridin-2-yl]-5,6,7,8-tetrahydrobenzo-[1,2,4]-triazin

### 1) Herstellung von 3-(6-Brom-5-methyl-pyridin-2-yl)-5,6,7,8-tetrahydro-benzo-[1,2,4]-triazin

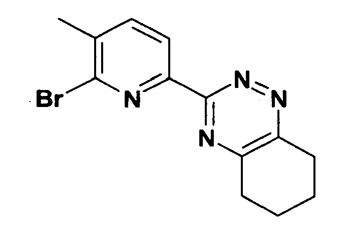

Zu einer Lösung von 10 g (40 mmol) 6-Brom-5-methylpyridin-2-carboxamidin-Hydrochlorid in 100 ml Ethanol (EtOH) gab man 2,4 g (48 mmol) Hydraziniumhydroxid. Nach 30 min versetzte man mit 4,5 g (40 mmol) Cyclohexan-1,2-dion und rührte 7 Stunden unter Rückfluss. Die Reaktionslösung wurde danach zwischen Wasser und MtBE verteilt, die organische Phase abgetrennt, das Lösungsmittel unter Vakuum entfernt und der Rückstand über Kieselgel mit Cyclohexan/MtBE (1 : 1) und MtBE/EtOH (1 : 1) chromatographiert. Ausbeute: 5,5 g Produkt.
¹H-NMR (δ , CDCl₃,): 1,9 (m); 2,5 (s); 2,8 (m); 3,1 (m); 3,2 (m); 7,7 (m) und 8,45 (m).

### 2) Herstellung von 3-[6-(4-Fluorphenyl)-5-methyl-pyridin-2-yl]-5,6,7,8-tetrahydrobenzo-[1,2,4]-triazin

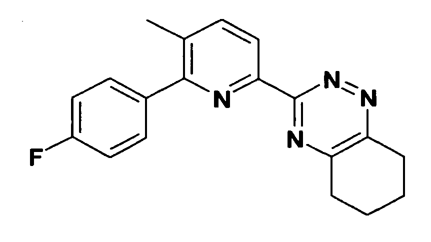

Zu der Lösung von 0,4 g der in Schritt 1 hergestellten Verbindung in 20 ml Ethylenglykoldimethylether gab man nacheinander 0,22 g 4-Fluorphenylboronsäure und 0,41 g Natriumcarbonat in 20 ml Wasser. Nach Zugabe von ca. 30 mg [1,4-Bis(diphenylphosphino)butan]-palladium(II)-dichlorid rührte man 5 Stunden unter Rückfluss. Die Reaktionslösung wurde danach zwischen Wasser und MtBE verteilt. Die organische Phase wurde abgetrennt, das Lösungsmittel unter Vakuum entfernt und der Rückstand über Kieselgel mit Cyclohexan/MtBE (1:1) chromatographiert. Man erhielt so 0,13 g Produkt.
Fp. 105 °C

In analoger Weise wurden die in Tabelle 1 beschriebenen Verbindungen der allgemeinen Formel I hergestellt:

**Tabelle 1:**

| Nr. | R⁵ | R⁴ | R³ | R¹ | R² | Phys. Daten (°C)/[M+H]⁺ |
|---|---|---|---|---|---|---|
| 1 | 4-Fluor-phenyl | CH₃ | H | CH₂CH₃ | CH₂CH₃ | 108 |
| 2 | 4-Fluor-phenyl | CH₃ | H | CH₂CH₂ CH₂CH₂ | | 105 |
| 3 | 4-Fluor-phenyl | H | H | CH₂CH₃ | CH₂CH₃ | 112-115 |
| 4 | Phenyl | CH₃ | H | CH₂CH₃ | CH₂CH₃ | 70-73 |
| 5 | 4-Fluor-phenyl | CH₃ | H | CH₃ | CH₃ | 151-154 |
| 6 | Phenyl | CH₃ | H | CH₃ | CH₃ | 193-196 |
| 7 | 3,4-Difluor-phenyl | CH₃ | H | CH₃ | CH₃ | 139-142 |
| 8 | 3,4,5-Trifluor-phenyl | CH₃ | H | CH₃ | CH₃ | 169 |
| 9 | 3,5-Difluor-phenyl | CH₃ | H | CH₃ | CH₃ | 158 |
| 10 | 3-Chlor-4-fluor-phenyl | CH₃ | H | CH₃ | CH₃ | 120 |
| 11 | 3-Methyl-4-fluor-phenyl | CH₃ | H | CH₃ | CH₃ | 154 |
| 12 | 4-Chlor-phenyl | CH₃ | H | CH₂CH₃ | CH₂CH₃ | 133-137 |
| 13 | 4-Methyl-phenyl | CH₃ | H | CH₂CH₃ | CH₂CH₃ | 128-130 |
| 14 | 4-Methoxy-phenyl | CH₃ | H | CH₂CH₃ | CH₂CH₃ | 118-120 |
| 15 | 4-Cyano-phenyl | CH₃ | H | CH₂CH₃ | CH₂CH₃ | 171-173 |
| 16 | 4-Formyl-phenyl | CH₃ | H | CH₂CH₃ | CH₂CH₃ | 122-124 |
| 17 | 4-tert.-Butyl-phenyl | CH₃ | H | CH₂CH₃ | CH₂CH₃ | 97-101 |
| 18 | 4-iso-Propyl-phenyl | CH₃ | H | CH₂CH₃ | CH₂CH₃ | 100-103 |
| 19 | 4-Trifluormethyl-phenyl | CH₃ | H | CH₂CH₃ | CH₂CH₃ | 131-137 |
| 20 | 2-Chlor-phenyl | CH₃ | H | CH₂CH₃ | CH₂CH₃ | 103-106 |
| 21 | 2-Fluor-phenyl | CH₃ | H | CH₂CH₃ | CH₂CH₃ | 129-132 |
| 22 | 2-Methyl-phenyl | CH₃ | H | CH₂CH₃ | CH₂CH₃ | 114-120 |
| 23 | 2,4-Difluor-phenyl | CH₃ | H | CH₂CH₃ | CH₂CH₃ | 142-144 |
| 24 | 3-Chlor-phenyl | CH₃ | H | CH₂CH₃ | CH₂CH₃ | 95-98 |
| 25 | 3-Methyl-phenyl | CH₃ | H | CH₂CH₃ | CH₂CH₃ | 61-64 |
| 26 | 3-Methoxy-phenyl | CH₃ | H | CH₂CH₃ | CH₂CH₃ | Öl |
| 27 | 4-Acetyl-phenyl | CH₃ | H | CH₂CH₃ | CH₂CH₃ | 124-126 |
| 28 | 4-Ethoxy-phenyl | CH₃ | H | CH₂CH₃ | CH₂CH₃ | 103-107 |
| 29 | 3,4-Dimethoxy-phenyl | CH₃ | H | CH₂CH₃ | CH₂CH₃ | 99-102 |
| 30 | 3,5-Dichlor-phenyl | CH₃ | H | CH₂CH₃ | CH₂CH₃ | 146-148 |
| 31 | Phenyl | CH₃ | H | CH₂CH₂CH₂CH₂ | | 161 |
| 32 | 4-Fluor-phenyl | H | H | CH₂CH₂CH₂CH₂ | | 140-143 |
| 33 | 3-Chlor-4-fluor-phenyl | CH₃ | H | CH₂CH₃ | CH₂CH₃ | 124-128 |
| 34 | 4-Phenoxyphenyl | CH₃ | H | CH₂CH₃ | CH₂CH₃ | 125-128 |
| 35 | 4-Thiomethyl-phenyl | CH₃ | H | CH₂CH₃ | CH₂CH₃ | 123-126 |
| 36 | 3,4-Dichlor-phenyl | CH₃ | H | CH₂CH₃ | CH₂CH₃ | 145-148 |
| 37 | 3-Chlor-4-methoxy-phenyl | CH₃ | H | CH₂CH₃ | CH₂CH₃ | 127-130 |
| 38 | 3-Fluor-4-ethoxy-phenyl | CH₃ | H | CH₂CH₃ | CH₂CH₃ | 96-99 |
| 39 | 3,5-Dimethyl-phenyl | CH₃ | H | CH₂CH₃ | CH₂CH₃ | M+H: 333 |
| 40 | 3-Chlor-4-iso-propoxy-phenyl | CH₃ | H | CH₂CH₃ | CH₂CH₃ | M+H: 397 |
| 41 | 3-Chlor-4-ethoxy-phenyl | CH₃ | H | CH₂CH₃ | CH₂CH₃ | 116-119 |
| 42 | 2-Methoxy-phenyl | CH₃ | H | CH₂CH₃ | CH₂CH₃ | 97-100 |
| 43 | 3-Fluor-phenyl | CH₃ | H | CH₂CH₃ | CH₂CH₃ | M+H: 323 |
| 44 | 3-Ethoxy-phenyl | CH₃ | H | CH₂CH₃ | CH₂CH₃ | 89-92 |
| 45 | 3-Fluor-4-methoxy | CH₃ | H | CH₂CH₃ | CH₂CH₃ | 117-120 |
| 46 | 3,4-Difluor-phenyl | CH₃ | H | CH₂CH₃ | CH₂CH₃ | 121-124 |
| 47 | 4-Ethyl-phenyl | CH₃ | H | CH₂CH₃ | CH₂CH₃ | 102-106 |
| 48 | 3,5-Difluor-phenyl | CH₃ | H | CH₂CH₃ | CH₂CH₃ | 111-113 |
| 49 | 3-Isopropoxy-phenyl | CH₃ | H | CH₂CH₃ | CH₂CH₃ | M+H:363,2 |
| 50 | 2,3-Difluor-phenyl | CH₃ | H | CH₂CH₃ | CH₂CH₃ | 114-117 |
| 51 | 2,5-Difluor-phenyl | CH₃ | H | CH₂CH₃ | CH₂CH₃ | 114-118 |
| 52 | 2,5-Dichlor-phenyl | CH₃ | H | CH₂CH₃ | CH₂CH₃ | 117-120 |
| 53 | 3-Methyl-4-fluor-phenyl | CH₃ | H | CH₂CH₃ | CH₂CH₃ | 92-95 |
| 54 | 2,4-Dimethoxy-phenyl | CH₃ | H | CH₂CH₃ | CH₂CH₃ | M+H:365,2 |
| 55 | 2,3-Dimethyl-phenyl | CH₃ | H | CH₂CH₃ | CH₂CH₃ | 125-130 |
| 56 | 2,5-Dimethyl-phenyl | CH₃ | H | CH₂CH₃ | CH₂CH₃ | M+H: 333,2 |
| 57 | 2-Ethoxy-phenyl | CH₃ | H | CH₂CH₃ | CH₂CH₃ | 89-96 |
| 58 | 4-Trifluormethoxy-phenyl | CH₃ H | | CH₂CH₃ | CH₂CH₃ | 113-117 |
| 59 | 3,4,5-Trifluor-phenyl | CH₃ | H | CH₂CH₃ | CH₂CH₃ | 157-160 |
| 60 | 4-n-Propoxy-phenyl | CH₃ | H | CH₂CH₃ | CH₂CH₃ | 113-115 |
| 61 | 3-Fluor-4-methyl-phenyl | CH₃ H | | CH₂CH₃ | CH₂CH₃ | 119-122 |
| 62 | 4-iso-Propoxy-phenyl | CH₃ | H | CH₂CH₃ | CH₂CH₃ | 91-95 |
| 63 | 4-n-Butoxy-phenyl | CH₃ | H | CH₂CH₃ | CH₂CH₃ | M+H:377,2 |
| 64 | 4-Chlor-phenyl | CH₃ | H | CH₂CH₂CH₂CH₂ | | 165-168 |
| 65 | 2-Chlor-phenyl | CH₃ | H | CH₂CH₂CH₂CH₂ | | 175-178 |
| 66 | 2-Methyl-phenyl | CH₃ | H | CH₂CH₂CH₂CH₂ | | 156-159 |
| 67 | 2,4-Difluor-phenyl | CH₃ | H | CH₂CH₂CH₂CH₂ | | 142-145 |
| 68 | 2,4-Dichlor-phenyl | CH₃ | H | CH₂CH₂CH₂CH₂ | | 173-176 |
| 69 | 4-Methoxy-phenyl | CH₃ | H | CH₂CH₂CH₂CH₂ | | 159-163 |
| 70 | 2-Methoxy-phenyl | CH₃ | H | CH₂CH₂CH₂CH₂ | | 144-147 |
| 71 | 2-Fluor-phenyl | CH₃ | H | CH₂CH₂CH₂CH₂ | | 148-152 |
| 72 | 2,3-Dimethyl-phenyl | CH₃ | H | CH₂CH₂CH₂CH₂ | | 142-145 |
| 73 | 2-Ethyl-phenyl | CH₃ | H | CH₂CH₂CH₂CH₂ | | 140-142 |
| 74 | 2-Trifluormethyl-phenyl | CH₃ H | | CH₂CH₂CH₂CH₂ | | 163-166 |
| 75 | 4-Trifluormethyl-phenyl | CH₃ H | | CH₂CH₂CH₂CH₂ | | 155-158 |
| 76 | 2-Methyl-4-fluor-phenyl | CH₃ H | | CH₂CH₃ | CH₂CH₃ | M+H:337,3 |
| 77 | 3-Methyl-4-methoxy | CH₃ | H | CH₂CH₃ | CH₂CH₃ | 101-108 |
| 78 | 3-Methyl-4-chlor-phenyl | CH₃ H | | CH₂CH₃ | CH₂CH₃ | 105-109 |
| 79 | Thiophen-2-yl | CH₃ | H | CH₂CH₃ | CH₂CH₃ | 141-144 |
| 80 | 4-Methyl-thiophen-3-yl | CH₃ | H | CH₂CH₃ | CH₂CH₃ | 98-102 |
| 81 | 6-Fluor-pyridin-3-yl | CH₃ | H | CH₂CH₃ | CH₂CH₃ | 126-129 |
| 82 | Pyridin-3-yl | CH₃ | H | CH₂CH₃ | CH₂CH₃ | 93-97 |
| 83 | 6-Methoxy-pyridin-3-yl | CH₃ | H | CH₂CH₃ | CH₂CH₃ | 113-117 |
| 84 | 2-Chlor-pyridin-3-yl | CH₃ | H | CH₂CH₃ | CH₂CH₃ | 93-99 |
| 85 | 6-Chlor-pyridin-3-yl | CH₃ H | | CH₂CH₃ | CH₂CH₃ | 143-147 |
| 86 | Pyridin-4-yl | CH₃ | H | CH₂CH₃ | CH₂CH₃ | 127-130 |

### Prüfung der fungiziden Wirksamkeit:

Die Wirkstoffe wurden getrennt oder gemeinsam als eine Stammlösung aufbereitet mit 25 mg Wirkstoff, welcher mit einem Gemisch aus Aceton und/oder Dimethylsulfoxid (DMSO) und dem Emulgator Wettol® EM 31 (Netzmittel mit Emulgier- und Dispergierwirkung auf der Basis ethoxylierter Alkylphenole) im Volumenverhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser-Gemisch zu der angegebenen Wirkstoffkonzentration verdünnt.

Anwendungsbeispiel 1- Wirksamkeit gegen die Dürffleckenkrankheit der Tomate, verursacht durch Alternaria solani

Blätter von getopften Tomatenpflanzen wurden mit einer wässrigen Suspension in der unten angegebenen Wirkstoffkonzentration bis zur Tropfnässe besprüht. Am nächsten Tag wurden die behandelten Pflanzen mit einer Sporensuspension von Alternaria solani die 0,17 x 10⁶ Sporen/ml in einer 2%igen wässrigen Biomalzlösung enthielt, inokuliert. Anschließend wurden die Versuchspflanzen in einer mit Wasserdampf gesättigten Kammer bei Temperaturen von 20 bis 22 °C aufgestellt. Nach 5 Tagen hatte sich die Krankheit auf den unbehandelten, infizierten Kontrollpflanzen so stark entwickelt, dass der Befall aller Pflanzen visuell in % ermittelt werden konnte.

In diesem Test zeigten die mit 250 ppm der Wirkstoffe aus den Beispielen 4, 6, 7, 8, 9, 10, 11 bzw. 33 behandelten Pflanzen maximal einen Befall von 5 %, während die unbehandelten Pflanzen zu 90 % befallen waren.

Anwendungsbeispiel 2 - Wirksamkeit gegen den Grauschimmel an Paprikablättern, verursacht durch Botrytis cinerea bei 1 Tag protektiver Anwendung

Paprikablätter der Sorte "Neusiedler Ideal Elite" wurden, nachdem sich 2 bis 3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von Botrytis cinerea, die 1,7 x 10⁶ Sporen/ml in einer 2%igen wässrigen Biomalzlösung enthielt, inokuliert. Anschließend wurden die Versuchspflanzen in eine Klimakammer bei Temperaturen zwischen 22 und 24 °C, Dunkelheit und hoher Luftfeuchtigkeit gestellt. Nach 5 Tagen wurde das Ausmaß des Pilzbefalls auf den Blättern visuell in % ermittelt.

In diesem Test zeigten die mit 250 ppm der Wirkstoffe aus den Beispielen 4, 6, 7, 8, 9, 10, 11, 31 bzw. 33 behandelten Pflanzen maximal einen Befall von 10 %, während die unbehandelten Pflanzen zu 90 % befallen waren.

Anwendungsbeispiel 3 - Wirksamkeit gegen die Netzfleckenkrankheit der Gerste verursacht durch Pyrenophora teres bei 1 Tag protektiver Anwendung

Blätter von in Töpfen gewachsenen Gerstenkeimlingen wurden mit wässriger Suspension in der unten angegebenen Wirkstoffkonzentration bis zur Tropfnässe besprüht. 24 Stunden nach dem Antrocknen des Spritzbelages wurden die Versuchspflanzen mit einer wässrigen Sporensuspension von Pyrenophora [syn. Drechslera] teres, dem Erreger der Netzfleckenkrankheit, inokuliert. Anschließend wurden die Versuchspflanzen im Gewächshaus bei Temperaturen zwischen 20 und 24 °C und 95 bis 100 % relativer Luftfeuchtigkeit aufgestellt. Nach 6 Tagen wurde das Ausmaß der Krankheitsentwicklung visuell in % Befall der gesamten Blattfläche ermittelt.

In diesem Test zeigten die mit 250 ppm des Wirkstoffs aus Beispiel 31 behandelten Pflanzen maximal einen Befall von 10 %, während die unbehandelten Pflanzen zu 90 % befallen waren.

## Patentansprüche

1. 3-(Pyridin-2-yl)-[1,2,4]-triazin-Verbindungen der allgemeinen Formel I 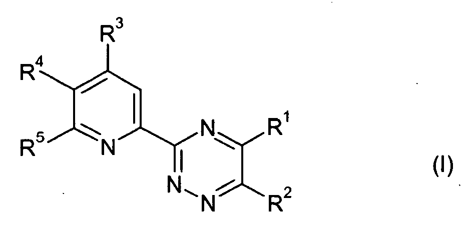 worin:
R¹, R² unabhängig voneinander für OH, Halogen, NO₂, NH₂, C₁-C₈-Alkyl, C₁-C₈-Alkoxy, C₁-C₈-Halogenalkyl, C₁-C₈-Halogenalkoxy, C₁-C₈-Alkylamino oder Di(C₁-C₈-alkyl)amino stehen, oder gemeinsam mit den C-Atomen, an die sie gebunden sind, einen ge- sättigten 5-, 6- oder 7-gliedrigen Carbocyclus oder Heterocyclus bilden können, der neben den Kohlenstoffringgliedern ein oder zwei, unter Sauer- stoff und Schwefel ausgewählte Heteroatome als Ringglieder aufweist, wo- bei der Carbocyclus und der Heterocyclus unsubstituiert sind oder 1, 2, 3 oder 4 C₁-C₄-Alkylgruppen als Substituenten aufweisen;
R³ für Wasserstoff, C₁-C₄-Alkyl, C₁-C₄-Alkoxy, C₁-C₄-Halogenalkyl, C₁-C₄-Halogenalkoxy, C₃-C₆-Cycloalkyl, C₃-C₆-Cycloalkylmethyl oder Halo- gen steht;
R⁴ für Wasserstoff, C₁-C₄-Alkyl, C₁-C₄-Alkoxy, C₁-C₄-Halogenalkyl, C₁-C₄-Halogenalkoxy oder Halogen steht;
R⁵ für C₁-C₈-Alkyl, C₁-C₈-Halogenalkyl, C₁-C₈-Alkoxy, C₁-C₈-Halogenalkoxy, C₃-C₈-Cycloalkyl, C₃-C₈-Cycloalkyloxy, 5- oder 6-gliedriges Heteroaryl, Phenyl, Phenoxy, Benzyl, Benzyloxy, 5- oder 6-gliedriges Heteroarylmethyl oder 5- oder 6-gliedriges Heteroaryloxy, wobei die zuvor genannten cycli- schen Reste unsubstituiert sind oder 1, 2, 3, 4 oder 5 Reste R^{a} aufweisen können, wobei R^{a} ausgewählt ist unter OH, SH, Halogen, NO₂, NH₂, CN, COOH, C₁-C₈-Alkyl, C₁-C₈-Alkoxy, C₁-C₈-Halogenalkyl, C₁-C₈-Halogenalkoxy, C₁-C₈-Alkylamino, Di(C₁-C₈-alkyl)amino, C₁-C₈-Alkylthio, C₁-C₈-Halogenalkylthio, C₁-C₈-Alkylsulfinyl, C₁-C₈-Halogenalkylsulfinyl, C₁-C₈-Alkylsulfonyl, C₁-C₈-Halogenalkylsulfonyl, C₃-C₈-Cycloalkyl, Phenyl, Phenoxy und Resten der Formel C(=Z)R^{aa}, worin Z für O, S, N(C₁-C₈-Alkyl), N(C₁-C₈-Alkoxy), N(C₃-C₈-Alkenyloxy) oder N(C₃-C₈-Alkinyloxy) steht und R^{aa} Wasserstoff, C₁-C₈-Alkyl, C₁-C₈-Alkoxy NH_{2,} C₁-C₈-Alkylamino oder Di(C₁-C₈-alkyl)amino bedeutet, oder zwei an benachbarte C-Atome gebun- dene Reste R^{a} gemeinsam mit den C-Atomen, an die sie gebunden sind, auch einen gesättigten 5-, 6- oder 7-gliedrigen Carbocyclus, einen Benzol- ring oder einen 5-, 6- oder 7-gliedrigen Heterocyclus bilden können, der neben den Kohlenstoffringgliedern ein oder zwei, unter Sauerstoff und Schwefel ausgewählte Heteroatome als Ringglieder aufweist, wobei der Carbocyclus und der Heterocyclus unsubstituiert sind oder 1, 2, 3 oder 4 C₁-C₄-Alkylgruppen als Substituenten aufweisen;
und die landwirtschaftlich brauchbaren Salze von Verbindungen der Formel I, ausgenommen:
2,6-Bis-(5,6-dimethyl-1,2,4-triazin-3-yl)pyridin;
2,6-Bis-(5,6-diethyl-1,2,4-triazin-3-yl)pyridin;
2,6-Bis-(5,6-dipropyl-1,2,4-triazin-3-yl)pyridin;
2,6-Bis-(5,6-diisopropyl-1,2,4-triazin-3-yl)pyridin;
2,6-Bis-(5,6-dibutyl-1,2,4-triazin-3-yl)pyridin;
2,6-Bis-(5,6-diisobutyl-1,2,4-triazin-3-yl)pyridin;
2,6-Bis-(5,6-dipentyl-1,2,4-triazin-3-yl)pyridin;
2,6-Bis-(5,6-dihexyl-1,2,4-triazin-3-yl)pyridin;
2,6-Bis-(5,6-diheptyl-1,2,4-triazin-3-yl)pyridin;
3-[6-(2,2'-bipyridyl)]-5,6-dimethyl-1,2,4-triazin;
3-[6-(2,2'-bipyridyl)]-5,6-diethyl-1,2,4-triazin;
3-[6-(2,2'-bipyridyl)]-5,6-dipropyl-1,2,4-triazin;
3-[6-(2,2'-bipyridyl)]-5,6-dibutyl-1,2,4-triazin;
5,6-Diethyl-3-[6-(2-pyridyl)-4-methoxypyridin-2-yl]-1,2,4-triazin;
3-(6-Methylpyridin-2-yl)-5,6-dimethyl-1,2,4-triazin;
3-(6-Methylpyridin-2-yl)-5,6-diethyl-1,2,4-triazin;
2,6-Bis-(5,6-dimethoxy-1,2,4-triazin-3-yl)pyridin; und
2,6-Bis-(5,6-diethoxy-1,2,4-triazin-3-yl)pyridin.

2. Verbindungen nach Anspruch 1, worin R¹ und R² unabhängig voneinander ausgewählt sind unter Fluor, Chlor, C₁-C₄-Alkyl, Methoxy, Ethoxy, CF₃, CHF₂, OCF₃ und OCHF₂.

3. Verbindungen nach Anspruch 1, worin R¹ und R² gemeinsam mit den C-Atomen des Triazinrings, an die sie gebunden sind, für einen der folgenden Ringe stehen: 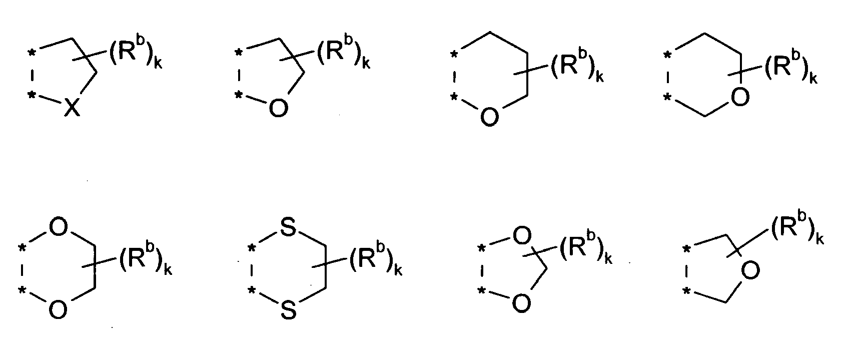 worin
* die Atome des Triazinrings bezeichnet;
k 0, 1, 2, 3 oder 4 bedeutet;
R^{b} für C₁-C₄-Alkyl steht; und
X für (CH₂)ₙ steht mit n = 1, 2 oder 3 und wobei, wenn k ≠ 0 ist, 1, 2, 3 oder 4 der Wasserstoffatome in (CH₂)ₙ durch R^{b} ersetzt sein können.

4. Verbindungen nach Anspruch 1, worin R¹ und R² für C₁-C₄-Alkyl stehen oder gemeinsam mit den C-Atomen des Triazinrings, an die sie gebunden sind, für einen Ring der Formel 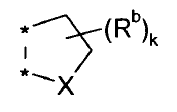 worin
* die Atome des Triazinrings bezeichnet;
k 0, 1, 2, 3 oder 4 bedeutet;
R^{b} für C₁-C₄-Alkyl steht; und
X für (CH₂)ₙ steht mit n = 2 oder 3 und wobei, wenn k * 0 ist, 1, 2, 3 oder 4 der Wasserstoffatome in (CH₂)ₙ durch R^{b} ersetzt sein können.

5. Verbindungen nach einem der vorhergehenden Ansprüche, worin R³ für Wasserstoff, Fluor, Chlor, C₁-C₄-Alkyl, Methoxy, Ethoxy, CF₃, CHF₂, OCF₃ oder OCHF₂ steht.

6. Verbindungen nach einem der vorhergehenden Ansprüche, worin R⁴ für Wasserstoff, Fluor, Chlor, Methyl, Ethyl, Methoxy, Ethoxy, CF₃, CHF₂, OCF₃ oder OCHF₂ steht.

7. Verbindungen nach einem der vorhergehenden Ansprüche, worin R^{a} ausgewählt ist unter Halogen, C₁-C₄-Alkyl, C₁-C₄-Alkoxy, C₁-C₄-Alkylcarbonyl, C₁-C₄-Alkoxycarbonyl, und Resten der Formel C(=N-O-C₁-C₈-Alkyl)R^{aa}, worin R^{aa} für Wasserstoff oder C₁-C₄-Alkyl steht.

8. Verbindungen nach einem der vorhergehenden Ansprüche, worin R⁵ für Phenyl, Phenoxy oder Benzyl steht, worin der Phenylring 1, 2, 3, 4 oder 5 Reste R^{a} aufweist.

9. Verbindungen nach Anspruch 8, worin der Phenylring in Phenyl, Phenoxy oder Benzyl die allgemeine Formel P 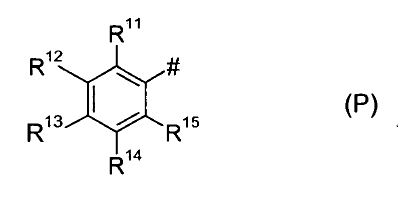 aufweist, worin # die Verknüpfung mit dem Rest des Moleküls bedeutet;
R¹¹ Wasserstoff, Fluor, Chlor, CH₃, OCH₃, OCHF₂, OCF₃ oder CF₃ bedeutet;
R¹², R¹⁴ unabhängig voneinander Wasserstoff, Chlor, Fluor, CH₃, OCH₃, OCHF₂, OCF₃ oder CF₃ bedeuten, wobei einer der Reste R¹² und R¹⁴ auch für NO₂, C(O)CH₃ oder COOCH₃ stehen kann;
R¹³ Wasserstoff, Fluor, Chlor, Cyano, OH, CHO, NO₂, NH₂, Methylamino, Di- methylamino, Diethylamino, C₁-C₄-Alkyl, C₃-C₈-Cycloalkyl, C₁-C₄-Alkoxy, C₁-C₄-Alkylthio, C₁-C₄-Haloalkyl, C₁-C₄-Haloalkoxy, CO(A²), worin A² für C₁-C₄-Alkyl oder C₁-C₄-Alkoxy steht, oder eine Gruppe C(R^{13a})=NOR^{13b}, worin R^{13a} für Wasserstoff oder Methyl steht und R^{13b} für C₁-C₄-Alkyl, Pro- pargyl oder Allyl steht, bedeutet oder R¹² und R¹³ gemeinsam eine Gruppe O-CH₂-O bilden; und
R¹⁵ Wasserstoff, Fluor, Chlor oder C₁-C₄-Alkyl bedeutet.

10. Verbindungen nach einem der Ansprüche 1 bis 7, worin R⁵ für C₁-C₆-Alkyl oder C₁-C₆-Haloalkyl steht.

11. Verbindungen nach einem der Ansprüche 1 bis 7, worin R⁵ ausgewählt ist unter 5-gliedrigem Heteroaryl, das 1, 2, 3 oder 4 Stickstoffatome oder 1 unter Sauerstoff und Schwefel ausgewähltes Heteroatom und gegebenenfalls 1, 2 oder 3 Stickstoffatome als Ringatome aufweist, und 6-gliedrigem Hetaryl, das 1, 2, 3 oder 4 Stickstoffatome als Ringglieder aufweist, wobei 5- und 6-gliedriges Hetaryl 1, 2, 3 oder 4 Substituenten R^{a} aufweisen können.

12. Verwendung von Verbindungen der Formel I gemäß einem der Ansprüche 1 bis 11 und ihrer Salze zur Bekämpfung von pflanzenpathogenen Pilzen,

13. Mittel für den Pflanzenschutz, enthaltend einen festen oder flüssigen Träger und eine Verbindung der Formel I gemäß einem der Ansprüche 1 bis 11 und/oder ein Salz davon.

14. Saatgut, enthaltend wenigstens eine Verbindung der Formel I gemäß einem der Ansprüche 1 bis 11 und/oder ein Salz davon.

15. Verfahren zur Bekämpfung von pflanzenpathogenen Pilzen, **dadurch gekennzeichnet, dass** man die Pilze, oder die vor Pilzbefall zu schützenden Materialien, Pflanzen, den Boden oder Saatgüter mit einer wirksamen Menge einer Verbindung der Formel I gemäß einem der Ansprüche 1 bis 11 oder einem Salz davon behandelt.

## Claims

1. A 3-(pyridin-2-yl)-[1,2,4]-triazine compound of the general formula I 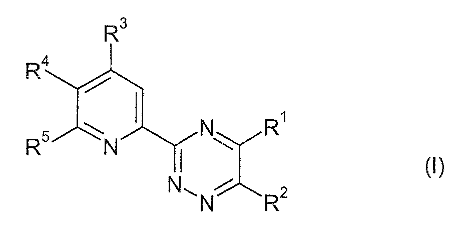 in which:
R¹, R² independently of one another are OH, halogen, NO₂, NH₂, C₁-C₈-alkyl, C₁- C₈-alkoxy, C₁-C₈-haloalkyl, C₁-C₈-haloalkoxy, C₁-C₈-alkylamino or di(C₁-C₈- alkyl)amino, or together with the carbon atoms to which they are attached may form a saturated 5-, 6- or 7-membered carbocycle or heterocycle which, in addition to the carbon ring members, has one or two heteroatoms selected from the group consisting of oxygen and sulfur as ring members, where the carbo- cycle and the heterocycle are unsubstituted or have 1, 2, 3 or 4 C₁-C₄-alkyl groups as substituents;
R³ is hydrogen, C₁-C₄-alkyl, C₁-C₄-alkoxy, C₁-C₄-haloalkyl, C₁-C₄-haloalkoxy, C₃-C₆-cycloalkyl, C₃-C₆-cycloalkylmethyl, or halogen;
R⁴ is hydrogen, C₁-C₄-alkyl, C₁-C₄-alkoxy, C₁-C₄-haloalkyl, C₁-C₄-haloalkoxy
or halogen;
R⁵ is C₁-C₈-alkyl, C₁-C₈-haloalkyl, C₁-C₈-alkoxy, C₁-C₈-haloalkoxy, C₃-C₈- cycloalkyl, C₃-C₈-cycloalkyloxy, 5- or 6-membered heteroaryl, phenyl, phe- noxy, benzyl, benzyloxy, 5- or 6-membered heteroarylmethyl or 5- or 6- membered heteroaryloxy, where the cyclic radicals mentioned above are unsubstituted or may have 1, 2, 3, 4 or 5 radicals R^{a}, where R^{a} is selected from the group consisting of OH, SH, halogen, NO₂, NH₂, CN, COOH, C₁-C₈-alkyl, C₁-C₈-alkoxy, C₁-C₈-haloalkyl, C₁-C₈-haloalkoxy, C₁-C₈-alkylamino, di(C₁-C₈-alkyl)amino, C₁-C₈-alkylthio, C₁-C₈-haloalkylthio, C₁-C₈-alkylsulfinyl, C₁-C₈-haloalkylsulfinyl, C₁-C₈-alkylsulfonyl, C₁-C₈- haloalkylsulfonyl, C₃-C₈-cycloalkyl, phenyl, phenoxy and radicals of the formula C(=Z)R^{aa} in which Z is O, S, N(C₁-C₈-alkyl), N(C₁-C₈-alkoxy), N(C₃- C₈-alkenyloxy) or N(C₃-C₈-alkynyloxy) and R^{aa} is hydrogen, C₁-C₈-alkyl, C₁- C₈-alkoxy, NH₂, C₁-C₈-alkylamino or di(C₁-C₈-alkyl)amino, or two radicals R^{a} attached to adjacent carbon atoms together with the carbon atoms to which they are attached may also form a saturated 5-, 6- or 7-membered carbo- cycle, a benzene ring or a 5-, 6- or 7-membered heterocycle which, in addi- tion to the carbon ring members, has one or two heteroatoms selected from the group consisting of oxygen and sulfur as ring members, where the car- bocycle and the heterocycle are unsubstituted or have 1, 2, 3 or 4 C₁-C₄- alkyl groups as substituents;
or an agriculturally useful salt of a compound of the formula I, except for:
2,6-bis-(5,6-dimethyl-1,2,4-triazine-3-yl)pyridine;
2,6-bis-(5,6-diethyl-1,2,4-triazine-3-yl)pyridine;
2,6-bis-(5,6-dipropyl-1,2,4-triazine-3-yl)pyridine;
2,6-bis-(5,6-diisopropyl-1,2,4-triazine-3-yl)pyridine;
2,6-bis-(5,6-dibutyl-1,2,4-triazine-3-yl)pyridine;
2,6-bis-(5,6-diisobutyl-1,2,4-triazine-3-yl)pyridine;
2,6-bis-(5,6-dipentyl-1,2,4-triazine-3-yl)pyridine;
2,6-bis-(5,6-dihexyl-1,2,4-triazine-3-yl)pyridine;
2,6-bis-(5,6-diheptyl-1,2,4-triazine-3-yl)pyridine;
3-[6-(2,2"-bipyridyl)]-5,6-dimethyl-1,2,4-triazine;
3-[6-(2,2'-bipyridyl)]-5,6-diethyl-1,2,4-triazine;
3-[6-(2,2'-bipyridyl)]-5,6-dipropyl-1,2,4-triazine;
3-[6-(2,2'-bipyridyl)]-5,6-dibutyl-1,2,4-triazine;
5,6-diethyl-3-[6-(2-pyridyl)-4-methoxypyridine-2-yl]-1,2,4-triazine;
3-[6-methylpyridine-2-yl)-5,6-dimethyl-1,2,4-triazine;
3-[6-methylpyridine-2-yl)-5,6-diethyl-1,2,4-triazine;
2,6-bis-(5,6-dimethoxy-1,2,4-triazine-3-yl)pyridine; and
2,6-bis-(5,6-diethoxy-1,2,4-triazine-3-yl)pyridine.

2. The compound according to claim 1 in which R¹ and R² independently of one another are selected from the group consisting of fluorine, chlorine, C₁-C₄-alkyl, methoxy, ethoxy, CF₃, CHF₂, OCF₃ and OCHF₂.

3. The compound according to claim 1 in which R¹ and R² together with the carbon atoms of the triazine ring to which they are attached are one of the following rings: 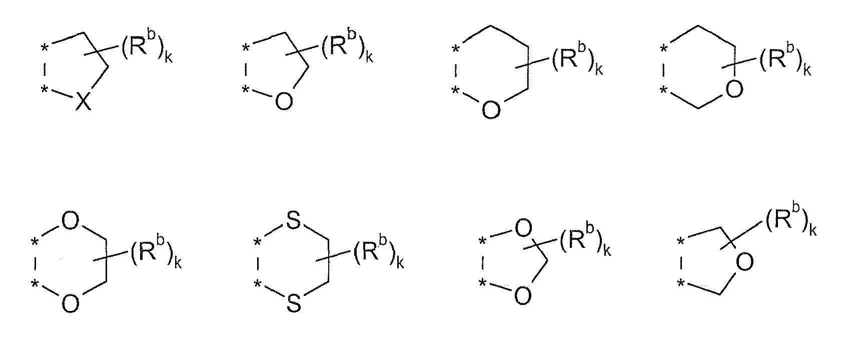 in which
* are the atoms of the triazine ring;
k is 0, 1, 2, 3 or 4;
R^{b} is C₁-C₄-alkyl; and
X is (CH₂)ₙ where n = 1, 2 or 3 and where 1, 2, 3 or 4 of the hydrogen atoms in (CH₂)ₙ may be replaced by R^{b} if k ≠ 0.

4. The compound according to claim 1 in which R¹ and R² are C₁-C₄-alkyl or together with the carbon atoms of the triazine ring to which they are attached are a ring of the formula 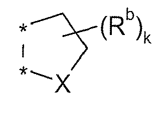 in which
* are the atoms of the triazine ring;
k is 0, 1, 2, 3 or 4;
R^{b} is C₁-C₄-alkyl; and
X is (CH₂)ₙ where n = 2 or 3 and where 1, 2, 3 or 4 of the hydrogen atoms in (CH₂)ₙ may be replaced by R^{b} if k ≠ 0.

5. The compound according to any of the preceding claims in which R³ is hydrogen, fluorine, chlorine, C₁-C₄-alkyl, methoxy, ethoxy, CF₃, CHF₂, OCF₃ or OCHF₂.

6. The compound according to any of the preceding claims in which R⁴ is hydrogen, fluorine, chlorine, methyl, ethyl, methoxy, ethoxy, CF₃, CHF₂, OCF₃ or OCHF₂.

7. The compound according to any of the preceding claims in which R^{a} is selected from the group consisting of halogen, C₁-C₄-alkyl, C₁-C₄-alkoxy, C₁-C₄-alkylcarbonyl, C₁-C₄-alkoxycarbonyl, and radicals of the formula C(=N-O-C₁-C₈-alkyl)R^{aa} in which R^{aa} is hydrogen or C₁-C₄-alkyl.

8. The compound according to any of the preceding claims in which R⁵ is phenyl, phenoxy or benzyl in which the phenyl ring has 1, 2, 3, 4 or 5 radicals R^{a}.

9. The compound according to claim 8 in which the phenyl ring in phenyl, phenoxy
or benzyl has the general formula P 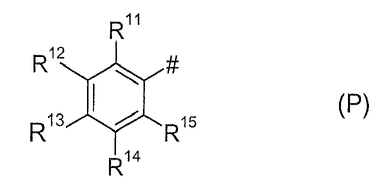 in which # is the point of attachment to the remainder of the molecule;
R¹¹ is hydrogen, fluorine, chlorine, CH₃, OCH₃, OCHF₂, OCF₃ or CF₃;
R¹², R¹⁴ independently of one another are hydrogen, chlorine, fluorine, CH₃, OCH₃, OCHF₂, OCF₃ or CF₃, where one of the radicals R¹² and R¹⁴ may al- so be NO₂, C(O)CH₃ or COOCH₃;
R¹³ is hydrogen, fluorine, chlorine, cyano, OH, CHO, NO₂, NH₂, methylamino, dimethylamino, diethylamino, C₁-C₄-alkyl, C₃-C₈-cycloalkyl, C₁-C₄-alkoxy, C₁-C₄-alkylthio, C₁-C₄-haloalkyl, C₁-C₄-haloalkoxy, CO(A²), in which A² is C₁-C₄-alkyl or C₁-C₄-alkoxy, or a group C(R^{13a})=NOR^{13b} in which R^{13a} is hy- drogen or methyl and R^{13b} is C₁-C₄-alkyl, propargyl or allyl, or R¹² and R¹³ together form a group O-CH₂-O; and
R¹⁵ is hydrogen, fluorine, chlorine or C₁-C₄-alkyl.

10. The compound according to any of claims 1 to 7 in which R⁵ is C₁-C₆-alkyl or C₁-C₆-haloalkyl.

11. The compound according to any of claims 1 to 7 in which R⁵ is selected from the group consisting of 5-membered heteroaryl which has 1, 2, 3 or 4 nitrogen atoms or 1 heteroatom selected from the group consisting of oxygen and sulfur and optionally 1, 2 or 3 nitrogen atoms as ring atoms and 6-membered hetaryl which has 1, 2, 3 or 4 nitrogen atoms as ring members, where 5- and 6-membered hetaryl may have 1, 2, 3 or 4 substituents R^{a}.

12. The use of a compound of the formula I according to any of claims 1 to 11 or of a salt thereof for controlling phytopathogenic fungi.

13. A crop protection composition comprising a solid or liquid carrier and a compound of the formula I according to any of claims 1 to 11 and/or a salt thereof.

14. Seed comprising at least one compound of the formula I according to any of claims 1 to 11 and/or a salt thereof.

15. A method for controlling phytopathogenic fungi wherein the fungi, or the materials, plants, the soil or seed to be protected against fungal attack are/is treated with an effective amount of a compound of the formula I according to any of claims 1 to 11 or a salt thereof.

## Revendications

1. Composés de type 3-(pyridin-2-yl-[1,2,4]triazine de formulé générale I 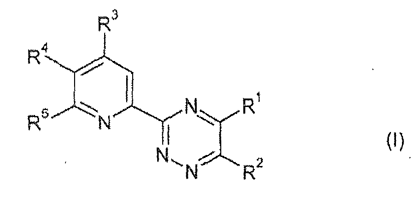 dans laquelle :
R¹, R² représentent, indépendamment l'un de l'autre, OH, un atome d'halogène, un groupe NO₂, NH₂, alkyle en C₁-C₈, alcoxy en C₁-C₈, halogénoalkyle en C₁-C₈, halogénoalcoxy en C₁-C₈, alkyl(C₁-C₈)- amino ou di[alkyl(C₁-C₈)]amino, ou forment ensemble, avec les atomes de carbone auxquels ils sont liés, un carbocycle ou hétérocycle à 5, 6 ou 7 chaînons, qui comporte, en plus des chaînons carbone formant le cycle, un ou deux hétéroatomes choisis parmi les atomes d'oxygène et de soufre, en tant que chaînons formant le cycle, le carbocycle et l'hétérocycle étant non substitués ou comportant 1, 2, 3 ou 4 groupes alkyle en C₁-C₄ en tant que substituants ;
R³ représente un atome d'hydrogène ou d'halogène ou un groupe alkyle en C₁-C₄, alcoxy en C₁-C₄, halogénoalkyle en C₁-C₄, halogénoalcoxy en C₁-C₄, cycloalkyle en C₃-C₆ ou cycloalkyl(C₃-C₆)- méthyle ;
R⁴ représente un atome d'hydrogène ou d'halogène ou un groupe alkyle en C₁-C₄, alcoxy en C₁-C₄, halogénoalkyle en C₁-C₄ ou halogénoalcoxy en C₁-C₄ ;
R⁵ représente un groupe alkyle en C₁-C₈, halogénoalkyle en C₁-C₈, alcoxy en C₁-C₈, halogénoalcoxy en C₁-C₈, cycloalkyle en C₃-C₈, cycloalkyloxy en C₃-C₈, hétéroaryle à 5 ou 6 chaînons, phényle, phénoxy, benzyle, benzyloxy, hétéroarylméthyle à 5 ou 6 chaînons ou hétéroaryloxy à 5 ou 6 chaînons, les radicaux cycliques précités étant non substitués ou pouvant comporter 1, 2, 3, 4 ou 5 radicaux R^{a}, R^{a} étant choisi parmi OH, SH, un atome d'halogène, NO₂, NH₂, CN, COOH, des groupes alkyle en C₁-C₈, alcoxy en C₁-C₈, halogénoalkyle en C₁-C₈, halogénoalcoxy en C₁-C₈, alkyl(C₁-C₈)amino, di[alkyl(C₁-C₈)]amino, alkyl(C₁-C₈)thio, halogénoalkyl(C₁-C₈)thio, alkyl(C₁-C₈)sulfinyle, halogénoalkyl(C₁-C₈)sulfinyle, alkyl(C₁-C₈)- sulfonyle, halogénoalkyl(C₁-C₈)sulfonyle, cycloalkyle en C₃-C₈, phényle, phénoxy et des radicaux de formule C(=Z)R^{aa}, où Z représente O, S, un groupe N[alkyle(C₁-C₈)], N[alcoxy(C₁-C₈)], N[alcényl(C₃-C₈)oxy] ou N[alcynyl(C₃-C₈)oxy] et R^{aa} représente un atome d'hydrogène, un groupe alkyle en C₁-C₈, alcoxy (C₁-C₈) NH₂, alkyl(C₁-C₈)amino ou di[alkyl(C₁-C₈)]amino, ou deux radicaux R^{a} liés à des atomes de carbone contigus peuvent également former ensemble, avec les atomes de carbone auxquels il sont liés, un carbocycle saturé à 5, 6 ou 7 chaînons, un cycle benzénique ou un hétérocycle à 5, 6 ou 7 chaînons, un cycle benzénique ou un hétérocycle à 5, 6 ou 7 chaînons, qui comporte, en plus des chaînons carbone formant le cycle, un ou deux hétéroatomes choisis parmi les atomes d'oxygène et de soufre, en tant que chaînons formant le cycle, le carbocycle et l'hétérocycle étant non substitués ou comportant 1, 2, 3 ou 4 groupes alkyle en C₁-C₄ en tant que substituants ;
et les sels utilisables en agriculture des composés de formule I, à l'exclusion de :
la 2,6-bis-(5,6-diméthyl-1,2,4-triazin-3-yl)pyridine ;
la 2,6-bis-(5,6-diéthyl-1,2,4-triazin-3-yl)pyridine ;
la 2,6-bis-(5,6-dipropyl-1,2,4-triazin-3-yl)pyridine ;
la 2,6-bis-(5,6-diisoprapyl-1,2,4-triazin-3-yl)-pyridine ;
la 2,6-bis-(5,6-dibutyl-1,2,4-triazin-3-yl)pyridine ;
la 2,6-bis-(5,6-diisobutyl-1,2,4-triazin-3-yl)-pyridine ;
la 2,6-bis-(5,6-dipentyl-1,2,4-triazin-3-yl)pyridine ;
la 2,6-bis-(5,6-dihexyl-1,2,4-triazin-3-yl)pyridine ;
la 2,6-bis-(5,6-diheptyl-1,2,4-triazin-3-yl)pyridine ;
la 3-[6-(2,2'-bipyridyl)]-5,6-diméthyl-1,2,4-triazine ;
la 3-[6-(2,2'-bipyridyl)]-5,6-diéthyl-1,2,4-triazine ;
la 3-[6-(2,2'-bipyridyl)]-5,6-dipropyl-1,2,4-triazine ;
la 3-[6-(2,2'-bipyridyl)]-5,6-dibutyl-1,2,4-triazine ;
la 5,6-diéthyl-3-[6-(2-pyridyl)-4-méthoxypyridin-2-y1]-1,2,4-triazine ;
la 3-(6-méthylpyridin-2-yl)-5,6-dimêthyl-1,2,4-triazine ;
la 3-(6-méthylpyridin-2-yl)-5,6-diéthyl-1,2,4-triazine ;
la 2,6-bis-(5,6-diméthoxy-1,2,4-triazin-3-yl)pyridine ; et
la 2,6-bis-(5,6-diéthoxy-1,2,4-triazin-3-yl)pyridine.

2. Composés selon la revendication 1, dans lesquels R¹ et R² sont choisis, indépendamment l'un de l'autre, parmi les atomes de fluor, de chlore, les groupes alkyle en C₁-C₄, méthoxy, éthoxy, CF₃, CHF₂, OCF₃ et OCHF₂.

3. Composés selon la revendication 1, dans lesquels R¹ et R² représentent ensemble, avec les atomes de carbone du cycle triazine auxquels ils sont liés, l'un des cycles suivants : 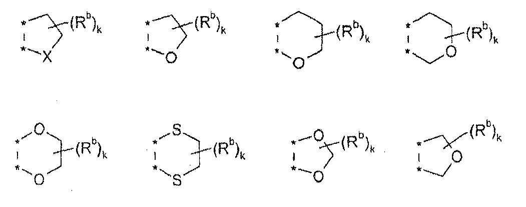 dans lesquels
* désigne les atomes du cycle triazine ;
k représente 0, 1, 2, 3 ou 4 ;
R^{b} représente un groupe alkyle en C₁-C₄ ; et
X représente (CH₂)ₙ, où n = 1, 2 ou 3 et, lorsque k est # 0, un, deux, trois ou quatre des atomes d'hydrogène dans (CH₂)ₙ pouvant être remplacés par R^{b}.

4. Composés selon la revendication 1, dans lesquels R¹ et R² représentent un groupe alkyle en C₁-C₄ ou forment ensemble, avec les atomes de carbone du cycle triazine auxquels ils sont liés, un cycle de formule : 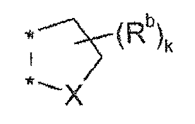 dans laquelle
* désigne les atomes du cycle triazine ;
k représente 0, 1, 2, 3 ou 4 ;
R^{b} représente un groupe alkyle en C₁-C₄ ; et
X représente (CH₂)ₙ, où n = 2 ou 3 et, lorsque k est ≠ 0, un, deux, trois ou quatre des atomes d'hydrogène dans (CH₂)ₙ pouvant être remplacés par R^{b}.

5. Composés selon l'une quelconque des revendications précédentes, dans lesquels R³ représente un atome d'hydrogène, de fluor ou de chlore, ou un groupe alkyle en C₁-C₄, méthoxy, éthoxy, CF₃, CHF₂, OCF₃ ou OCHF₂.

6. Composés selon l'une quelconque des revendications précédentes, dans lesquels R⁴ représente un atome d'hydrogène, de fluor ou de chlore, ou un groupe méthyle, éthyle, méthoxy, CF₃, CHF₂, OCF₃ ou OCHF₂.

7. Composés selon l'une quelconque des revendications précédentes, dans lesquels R^{a} est choisi parmi un atome d'halogène, un groupe alkyle en C₁-C₄, alcoxy en C₁-C₄, alkyl(C₁-C₄) carbonyle, alcoxy(C₁-C₄)carbonyle, et les radicaux de formule C[=N-O-alkyle(C₁-C₈)]R^{aa}, où R^{aa} représente un atome d'hydrogène ou un groupe alkyle en C₁-C₄.

8. Composés selon l'une quelconque des revendications précédentes, dans lesquels R⁵ représente le groupe phényle, phénoxy ou benzyle, le cycle phényle comportant 1, 2, 3, 4 ou 5 radicaux R^{a}.

9. Composés selon la revendication 8, dans lesquels le cycle phényle dans le groupe phényle, phénoxy ou benzyle présente la formule générale P 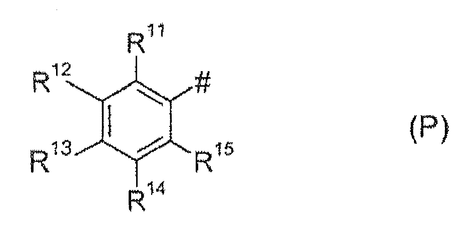 dans laquelle # signifie la liaison au reste de la molécule ;
R¹¹ représente un atome d'hydrogène, de fluor, de chlore, CH₃, OCH₃, OCHF₂, OCF₃ ou CF₃ ;
R¹², R¹⁴ représentent, indépendamment l'un de l'autre, un atome d'hydrogène, de chlore, de fluor, CH₃, OCH₃, OCHF₂, OCF₃ ou CF₃, l'un des radicaux R¹² et R¹⁴ pouvant également représenter NO₂, C(O)CH₃ ou COOCH₃ ;
R¹³ représente un atome d'hydrogène, de fluor, de chlore, un groupe cyano, OH, CHO, NO₂, NH₂, méthylamino, diméthylamino, diéthylamino, alkyle en C₁-C₄, cycloalkyle en C₃-C₈, alcoxy en C₁-C₄, alkyl(C₁-C₄) thio, halogénoalkyle en C₁-C₄, halogénoalcoxy en C₁-C₄, CO(A²), où A² représente un groupe alkyle en C₁-C₄ ou alcoxy en C₁-C₄, ou un groupe C(R^{13a})=NOR^{13b}, dans lequel R^{13a} représente un atome d'hydrogène ou le groupe méthyle et R^{13b} représente un groupe alkyle en C₁-C₄, propargyle ou allyle, ou R¹² et R¹³ forment ensemble un groupe O- CH₂-O ; et
R¹⁵ représente un atome d'hydrogène, de fluor, de chlore ou un groupe alkyle en C₁-C₄.

10. Composés selon l'une quelconque des revendications 1 à 7, dans lesquels R⁵ représente un groupe alkyle en C₁-C₆ ou halogénoalkyle en C₁-C₆.

11. Composés selon l'une quelconque des revendications 1 à 7, dans lesquels R⁵ est choisi parmi un groupe hétéroaryle à 5 chaînons, qui comporte 1, 2, 3 ou 4 atomes d'azote ou 1 hétéroatome choisi parmi les atomes d'oxygène et de soufre et éventuellement 1, 2 ou 3 atomes d'azote en tant que chaînons formant le cycle, et un groupe hétéroaryle à 6 chaînons, qui comporte 1, 2, 3 ou 4 atomes d'azote en tant que chaînons formant le cycle, les groupe hétéroaryle à 5 et 6 chaînons pouvant comporter 1, 2, 3 ou 4 substituants R^{a}.

12. Utilisation de composés de formule I selon l'une quelconque des revendications 1 à 11 et de leurs sels, pour la lutte contre des champignons pathogènes.

13. Composition pour la protection des plantes, contenant un support liquide ou solide et un composé de formule I selon l'une quelconque des revendications 1 à 11 et/ou un sel d'un tel composé.

14. Semence, contenant un composé de formule I selon l'une quelconque des revendications 1 à 11 et/ou un sel d'un tel composé.

15. Procédé pour la lutte contre des champignons phytopathogènes, **caractérisé en ce qu'**on traite les champignons ou les matériaux, les plantes, le sol ou les semences à protéger contre l'attaque des champignons, par une quantité efficace d'un composé de formule I selon l'une quelconque des revendications 1 à 11 ou d'un sel d'un tel composé.
